# EUROPEAN PATENT SPECIFICATION

(11) **EP 1 471 146 B1**
(45) Date of publication and mention of the grant of the patent: **07.01.2009**
(21) Application number: 04014954.4
(22) Date of filing: 24.09.1997
(51) Int. Cl.: C12N 15/34, C12N 15/861, C12N 5/10, C07K 14/075, A61K 48/00

(54) **A packaging cell line expressing for use in facilitating the development of high capacity adenoviral vectors**
Verpackungszellinien in der Verwendung zur Erleichterung der Entwicklung hocheffizienter adenoviraler Vektoren
Lignées cellulaires d'encapsidation permettant de faciliter le développement de vecteurs adénoviraux de grande capacité

(30) Priority: 25.09.1996 US 719806
(43) Date of publication of application: 27.10.2004
(62) Divisional of application: 97944892.5
(73) Proprietor: NOVARTIS AG, 4056 Basel (CH); THE SCRIPPS RESEARCH INSTITUTE, La Jolla, CA 92037 (US); Novartis Pharma GmbH, 1230 Wien (AT)
(72) Inventor: Nemerow, Glen R., Encinitas, CA 92024 (US); von Seggern, Daniel J., San Diego, CA 92122 (US)
(74) Representative: HOFFMANN EITLE

(56) References cited:
- WO-A-95/02697
- WO-A-95/26412
- WO-A-96/07734
- WO-A-96/26281
- BROUGH D E ET AL: "A GENE TRANSFER VECTOR-CELL LINE SYSTEM FOR COMPLETE FUNCTIONAL COMPLEMENTATION OF ADENOVIRUS EARLY REGIONS E1 AND E4" JOURNAL OF VIROLOGY, THE AMERICAN SOCIETY FOR MICROBIOLOGY, US, vol. 70, no. 9, 1 September 1996 (1996-09-01), pages 6497-6501, XP002923470 ISSN: 0022-538X
- SEGGERN VON D J ET AL: "A HELPER-INDEPENDENT ADENOVIRUS VECTOR WITH E1, E3, AND FIBER DELETED: STRUCTURE AND INFECTIVITY OF FIBERLESS PARTICLES" JOURNAL OF VIROLOGY, THE AMERICAN SOCIETY FOR MICROBIOLOGY, US, vol. 73, no. 2, 1999, pages 1601-1608, XP000906914 ISSN: 0022-538X

## Description

The present invention relates to gene therapy, especially to adenovirus-based gene therapy. In particular, novel packaging cell lines are disclosed, for use in facilitating the development of high-capacity vectors. High-capacity adenovirus vectors are also disclosed herein, as are related compositions, kits, and methods of preparation and use of the disclosed vectors, cell lines and kits.

Enhanced transfer of DNA conjugates into cells has been achieved with adenovirus, a human DNA virus which readily infects epithelial cells (Horwitz, "Adenoviridae and their replication", in Virology, Fields and Knipe, eds., Raven Press, NY (1990) pp. 1679-1740).

Although adenovirus-mediated gene therapy represents an improved method of DNA transfer into cells, a potential limitation of this approach is that adenovirus replication results in disruption of the host cell. In addition, adenovirus also possesses oncogenic properties including the ability of one of its proteins to bind to tumor suppressor gene products. The use of so-called replication defective strains of adenovirus (which typically possess E1A and/or E1B deletions that render the virus unable to replicate in host cells) is in principle more suitable for *in vivo* therapy; however, the potential of co-infection of epithelial cells with wild-type strains of virus resulting in transactivation of the recombinant virus may represent a significant safety concern for *in vivo* applications. Furthermore, it is not yet known which recombinant adenoviruses are capable of integrating their genome into host cell DNA allowing for long-term stable expression of any foreign genes they may be transporting.

Another undesirable aspect of using intact or replication-competent adenovirus as a gene transfer means is that it is an oncogenic virus whose gene products are known to interfere with the function of host cell tumor suppressor proteins as well as immune recognition molecules, such as the major histocompatibility complex (MHC). In addition, pre-existing circulating antibodies to adenovirus may significantly reduce the efficiency of *in vivo* gene delivery. Lastly, only a foreign gene of 6 kilobases (kb) or less can be incorporated into the intact adenovirus genome for gene transfer experiments, whereas DNA segments of greater than 15 kb can be transferred using the methods of this invention.

In order to make Ad vectors more replication-incompetent, some investigators have attempted to construct recombinant Ad-derived vectors which have nearly all of their genome deleted, except for portions known to be required for packaging of virus particles. For example, helper-dependent vectors lacking all viral ORFs but including essential cis elements (the inverted terminal repeats - ITRs -- and the contiguous packaging sequence) have been constructed, but the virions package less efficiently than the helper and package as multimers part of the time, which suggests that the virus may "want" to package a fuller DNA complement (see, e.g., Fisher, et al., Virology 217: 11-22 (1996)). Mitani et al. (PNAS USA 92: 3854-3858 (1995)) also describe a helper-dependent Ad vector that was apparently not completely replication-defective.

WO 95/02697 discloses defective adenovirus vectors and their use in gene therapy. The claimed cell lines are not disclosed.

WO 96/26281 discloses chimeric adenoviral fiber protein and methods of using the same, but does not refer to the cell lines disclosed in the present invention.

Amalfitano, et al. (PNAS USA 93: 3352-3356 (1996)) describe the construction of an Ad packaging cell lines that support the growth of E1- and polymerase-deleted Ad vectors, in an effort to block the replication of Ad vectors *in vivo*. Similarly, Armentano, et al. (Hum. Gene Ther. 6: 1343-53 (1995)) describes Ad vectors with most -- but not all -- of the E4 sequence deleted therefrom. However, since such a small amount of genetic material is deleted from the vectors, their ability to transport therapeutic sequences is rather limited.

In view of the aforementioned problems, the design and construction of the within-disclosed packaging cell lines and systems provides a novel and elegant solution, as described further herein. The use of the recombinant sequences and vectors of this invention to mediate the transfer of foreign genes into recipient cells both *in vitro* and *in vivo* overcomes the limitations of the above-described gene transfer systems. This invention utilizes recombinant constructs which duplicate the cell receptor binding and DNA delivery properties of intact adenovirus virions and thus represents an improved method for gene therapy as well as for antisense-based antiviral therapy.

In contrast to the disadvantages of using intact adenovirus, modified adenovirus vectors requiring a helper plasmid or virus, or so-called replication-deficient adenovirus, the use of recombinant adenovirus-derived vectors according to the present invention provides certain advantages for gene delivery. First, the Ad-derived vectors of the present invention possess all of the functional properties required for gene therapy including binding to epithelial cell receptors and penetration of endocytic vesicles. Therapeutic viral vectors of the present invention may also be engineered to target the receptors of and achieve penetration of non-epithelial cells; means of engineering viral vectors to accomplish these ends are described in detail hereinbelow.

Second, the vectors disclosed herein have deletions of substantial portions of the Ad genome, which not only limits the ability of the Ad-derived vectors to "spread" to other host cells or tissues, but allows significant amounts of "foreign" (or non-native) nucleic acids to be incorporated into the viral genome without interfering with the reproduction and packaging of the viral genome. Therefore, the vectors disclosed herein are ideal for use in a wide variety of therapeutic applications.

Third, while the vectors disclosed herein are safe for use as therapeutic agents in the treatment of a variety of human afflictions, they do not require the presence of any "helpers" for propagation and packaging, largely because of the novel cell lines in which they are reproduced. Such cell lines -- referred to herein as packaging cell lines -- comprise yet another aspect of the invention.

To reduce the frequency of contamination with wild-type adenovirus, it is desirable to improve either the viral vector or the cell line to reduce the probability of recombination. For example, an adenovirus from a group with less homology to the group C viruses may be used to engineer recombinant viruses with little propensity for recombination with the Ad5 sequence in 293 cells. Similarly, an epithelial cell line -- 293 or another -- may be prepared according to within-disclosed methods which stably expresses adenovirus proteins or polypeptides from Ad3 and/or proteins or polypeptides from another non-group-C or group C serotype; such a cell line would is useful for supporting adenovirus-derived viral vectors bearing deletions of regulatory and/or structural genes, irrespective of the serotype from which such a vector was derived.

It is also contemplated that the constructs and methods disclosed herein will support the design and engineering of chimeric viral vectors which express amino acid residue sequences derived from two or more Ad serotypes. Thus, unlike methods and constructs available prior to the advent of the present disclosure, this allows the greatest possible flexibility in the design and preparation of useful viral vectors and cell lines which support their construction and propagation -- all with a decreased risk of recombining with wild-type Ad to produce potentially-harmful recombinants.

In part, the present invention discloses a simpler, alternative means of reducing the recombination between viral and cellular sequences than those discussed in the art. One such means is to increase the size of the deletion in the recombinant virus and thereby reduce the extent of shared sequences between that virus and any Ad genes present in a packaging cell line e.g., the Ad5 genes in 293 cells, or the various Ad genes in the novel cell lines of the present invention.

Deletions of all or portions of structural genes of the adenovirus have been considered undesirable because of the anticipated deleterious effects such deletions would have on viral reproduction and packaging. Indeed, the use of "helper" viruses or plasmids has often been recommended when using Ad-derived vectors containing large deletions in structural protein sequences precisely for this reason.

Contrary to what has been suggested in the art, however, this specification discloses and claims the preparation, propagation and use of recombinant Ad-derived vectors having deletions of all or part of various gene sequences encoding Ad structural proteins, both as a means of reducing the risk of wild-type adenovirus contamination in virus preparations and as a means of allowing foreign DNA to be packaged in such vectors for a variety of diagnostic and therapeutic applications. The invention is defined in the appending claim.

Thus, a packaging cell line wherein DNA sequences encoding one or more adenovirus regulatory polypeptides and DNA sequences encoding one or more adenovirus structural polypeptides have been stably integrated into the cellular genome is disclosed.

Thus, a packaging cell line expressing one or more adenovirus structural proteins, polypeptides, or fragments thereof, wherein said structural protein is selected from the group consisting of:
a. penton base;
b. hexon;
c. fiber;
d. polypeptide IIIa;
e. polypeptide V;
f. polypeptide VI;
g. polypeptide VII;
h. polypeptide VIII; and
i. biologically active fragments thereof is disclosed.

In one variation, the sequences are constitutively expressed; in another, one or more sequences is under the control of a regulatable promoter. In a preferred embodiment expression is constitutive. In various preferred embodiments, the polypeptides expressed by the DNA sequences are biologically active.

In a further and preferred embodiment the packaging cell line of the present invention supports the production of a viral vector. In a preferred embodiment the viral vector is a therapeutic vector.

In one aspect, each DNA sequence is introduced into the genome of the within-disclosed cell lines via a separate complementing plasmid. In other embodiments, two or more DNA sequences were introduced into the genome via a single complementing plasmid. In one variation, the complementing plasmid comprises a DNA sequence encoding adenovirus fiber protein, polypeptide or fragment thereof. An example of a useful complementing plasmid according to the present invention is a plasmid having the characteristics of pCLF (for deposit details, see Example 3)

In another aspect, the complementing plasmid used to transform a cell line of the present invention further comprises a DNA sequence encoding an adenovirus regulatory protein, polypeptide or fragment thereof. In one variation, the regulatory protein is selected from the group consisting of E1A, E1B, E2A, E2B, E3, E4 and L4 (also referred to as "the 100K protein"); an exemplary complementing plasmid has the characteristics of is pE4/Hygro (for deposit details, see Example 3). In another aspect, the complementing plasmid used to transform a cell line of the present invention further comprises a DNA sequence encoding two or more of the above mentioned adenovirus regulatory proteins, polypeptides or fragments thereof.

In one variation, the two or more regulatory proteins, polypeptides or fragments thereof are selected from the group consisting of E1A, E1B, E2A, E2B, E3, E4 and L4 (also referred to as "the 100K protein"). In another variation, the structural protein is selected from the group consisting of penton base; hexon; fiber; polypeptide IIIa; polypeptide V; polypeptide VI; polypeptide VII; polypeptide VIII; and biologically active fragments thereof.

In one variation of the present invention, a packaging cell line expresses fiber protein. In one embodiment, the fiber protein has been modified to include a non-native amino acid residue sequence which targets a specific receptor, but which does not disrupt trimer formation or transport of fiber into the nucleus. In another variation, the non-native amino acid residue sequence alters the binding specificity of the fiber for a targeted cell type. In still another embodiment, the structural protein is fiber comprising amino acid residue sequences from more than one adenovirus serotype. As disclosed herein, the nucleotide sequences encoding fiber protein or polypeptide need not be modified solely at one or both termini; fiber protein - and indeed, any of the adenovirus structural proteins, as taught herein - may be modified "internally" as well as at the termini.

Disclosed is a packaging cell line wherein the viral vector produced in that cell line comprises a nucleic acid sequence having a deletion or mutation of a DNA sequence encoding an adenovirus structural protein, polypeptide, or fragment thereof. In one variation, the viral vector further comprises a nucleic acid sequence having a deletion or mutation of the DNA sequences encoding regulatory polypeptides E1A and E1B. In another variation, the viral vector further comprises a nucleic acid sequence having a deletion or mutation of a DNA sequence encoding one or more of the following regulatory proteins or polypeptides: E2A, E2B, E3, E4, L4, or fragments thereof.

Yet another variation discloses that a foreign DNA sequence encoding one or more foreign proteins, polypeptides or fragments thereof has been inserted in place of any of the deletions in the therapeutic viral vector. In one embodiment, the foreign DNA encodes a tumor-suppressor protein or a biologically active fragment thereof. In another embodiment, the foreign DNA encodes a suicide protein or a biologically active fragment thereof. As before, cell lines as described herein may be procaryotic or eucaryotic in origin, with mammalian cell lines often being preferred. Epithelial and non-epithelial cell lines are useful in the aforementioned variations; some particularly useful cell lines include 293, A549, W162, HeLa, Vero, 211, and 211A cell lines.

The invention further contemplates that the aforementioned cell lines support the production of viral vectors including foreign DNA sequences encoding one or more foreign proteins, polypeptides or fragments thereof has been inserted in place of any structural and/or regulatory proteins (or portions thereof) that have been deleted. Thus, in one embodiment, the foreign DNA encodes a tumor-suppressor protein; a suicide protein; a cystic fibrosis transmembrane conductance regulator (CFTR) protein; or a biologically active fragment of any of them.

Any of the within-disclosed cell lines may have a DNA sequence encoding all or part of a fiber protein -- including modified or chimeric proteins -- stably integrated into the genome. Thus, in one variation, the fiber protein has been modified to include a non-native amino acid residue sequence which targets a specific receptor, but which does not disrupt trimer formation or transport of fiber into the nucleus. In one variation, the non-native amino acid residue sequence is coupled to the carboxyl terminus of the fiber. In yet another, the non-native amino acid residue sequence further includes a linker sequence. Alternatively, the fiber protein further comprises a ligand coupled to the linker. A suitable ligand may be selected from the group consisting of ligands that specifically bind to a cell surface receptor and ligands that can be used to couple other proteins or nucleic acid molecules. In one variation, the ligand is selected from the group consisting of ligands that specifically bind to a cell surface receptor and ligands that can be used to couple other proteins or nucleic acid molecules.

In yet another embodiment, the non-native amino acid residue sequence is incorporated into the fiber amino acid residue sequence at a location other than one of the fiber termini. Alternatively, the non-native amino acid residue sequence alters the binding specificity of the fiber for a targeted cell type. In other embodiments, the linker sequence alters the binding specificity of the fiber for a targeted cell type. The expressed fiber may, in various embodiments, bind to a specific targeted cell type not usually targeted by adenovirus and/or may comprise amino acid residue sequences from more than one adenovirus serotype.

In various aspects of the present invention, a packaging cell line of the present invention is derived from a procaryotic cell line; in another, it is derived from a eucaryotic cell line. While various embodiments suggest the use of mammalian cells, and more particularly, epithelial cell lines, a variety of other, non-epithelial cell lines are used in various embodiments. Thus, while various embodiments disclose the use of a cell line selected from the group consisting of 293, A549, W162, HeLa, Vero, 211, and 211A cell lines, it is understood that various other cell lines are likewise contemplated for use as disclosed herein.

The specification further discloses a wide variety of nucleic acid sequences and viral vectors. Thus, in one embodiment, a nucleic acid sequence encoding any one of the aforementioned adenovirus fiber proteins, polypeptides or fragments thereofincluding, without limitation, those that include deletions or other mutations; those that are chimeric; and those that have linkers, foreign amino acid residues, or other molecules attached for various purposes as disclosed herein are disclosed. Nucleic acid sequences encoding various other adenovirus structural and/or regulatory proteins or polypeptides are also disclosed.

A wide variety of therapeutic viral vectors are also described, e.g.; a therapeutic viral vector which lacks a DNA sequence encoding fiber protein, or a portion thereof. In another variation, a therapeutic viral vector may further or alternatively comprise deletion of a DNA sequence encoding one or more regulatory proteins, polypeptides, or fragments thereof. Foreign DNA sequences are inserted in place of the DNA sequence encoding fiber protein in the viral vectors. Furthermore, the therapeutic viral vectors further comprise foreign DNA sequences inserted in place of the DNA sequences encoding one or more regulatory proteins, polypeptides, or fragments thereof, and/or one or more structural proteins, polypeptides, fragments thereof.

The description further discloses a number of viral vectors. In one variation, a viral vector comprises a deletion or mutation of a DNA sequence encoding an adenovirus structural protein, polypeptide, or fragment thereof. A vector may further comprise deletion or mutation of the DNA sequences encoding regulatory polypeptides E1A and E1B; and it may still further comprise deletion or mutation of the DNA sequence encoding one or more of the following regulatory proteins or polypeptides: E2A, E2B, E3, E4, L4, or fragments thereof. In another variation, in a viral vector, the structural protein comprises fiber. Any combination of the foregoing is also contemplated. The viral vectors are suitable for the preparation of pharmaceutical compositions comprising any of the therapeutic viral vectors disclosed herein -- including combinations thereof -- are also disclosed herein. A further use of the viral vectors is for targeting specific cells in a cell population comprising different cell types.

The specification further discloses complementing plasmids and methods of making same. In one embodiment, a complementing plasmid comprises a promoter nucleotide sequence operatively linked to a nucleotide sequence encoding an adenovirus structural polypeptide. In one variation, the complementing plasmid comprises pCLF. In another variation, a complementing plasmid further comprises a nucleotide sequence encoding a first adenovirus regulatory polypeptide, a nucleotide sequence encoding a second regulatory polypeptide, a nucleotide sequence encoding a third regulatory polypeptide; or any combination of the foregoing. In still another embodiment, the adenovirus structural polypeptide is selected from the group consisting of penton base; hexon; fiber; polypeptide IIIa; polypeptide V; polypeptide VI; polypeptide VII; polypeptide VIII; and biologically active fragments thereof.

The specification also discloses a complementing plasmid comprising a promoter nucleotide sequence operatively linked to a nucleotide sequence encoding an adenovirus structural protein, polypeptide or fragment thereof and a nucleotide sequence encoding an adenovirus regulatory protein, polypeptide or fragment thereof. In one variation, the early region polypeptide is E4; in another, the plasmid comprises pE4/Hygro. In still another variation, the early region polypeptides are E1 and E4. Complementing plasmids further comprising a nucleotide sequence encoding an adenovirus structural protein, polypeptide or fragment thereof are also contemplated, as are plasmids wherein the promoter nucleotide sequence is selected from the group consisting of MMTV, CMV and E4 promoter nucleotide sequences.

Viral vectors are also disclosed which comprise nucleotide sequences encoding a packaging signal and a foreign protein or polypeptide, wherein the nucleotide sequence encoding an adenovirus structural protein has been deleted. In one variation, the nucleotide sequence encoding the foreign protein or polypeptide is a DNA molecule up to about 3 kb in length; in another, the nucleotide sequence encoding the foreign protein or polypeptide is a DNA molecule up to about 9.5 kb in length; in still another, the nucleotide sequence encoding the foreign protein or polypeptide is a DNA molecule up to about 12.5 kb in length. Nucleotide sequences of intermediate lengths are also contemplated by the present invention, as are sequences in excess of 12.5 kb.

The specification also discloses viral vectors wherein the sequence encoding a foreign protein or polypeptide is a sequence encoding an anti-tumor agent, a tumor suppressor protein, a suicide protein, or a fragment or functional equivalent thereof. In one variation, nucleotide sequences encoding one or more regulatory proteins have also been deleted from the vector. In another, the regulatory proteins are selected from the group consisting of E1A, E1B, E2A, E2B, E3, E4, and L4 (100K protein).

In various embodiments, the adenovirus is a Group C adenovirus selected from serotypes 1, 2, 5, or 6; in other embodiments, adenovirus selected from other serotypes are useful as disclosed herein. The specification also discloses useful vaccines comprising a viral vector according to any of the foregoing specifications, and a pharmaceutically acceptable carrier or excipient.

Various useful compositions are also disclosed herein. One embodiment discloses a composition useful in the preparation of recombinant adenovirus viral vectors comprising a cell containing a delivery plasmid comprising an adenovirus genome lacking a nucleotide sequence encoding fiber. In one variation, the cell further comprises a complementing plasmid containing a nucleotide sequence encoding fiber, the plasmid being stably integrated into the cellular genome of the cell. In another variation, the delivery plasmid further comprises a nucleotide sequence encoding a foreign polypeptide. In one variation, the delivery plasmid is pDV44, p E1B gal, or p E1sp1B.

In another embodiment, the polypeptide is a therapeutic molecule. In yet another, the polypeptide is a therapeutic molecule. Another variation provides that the delivery plasmid further comprises a nucleotide sequence encoding a foreign polypeptide.

Compositions useful in the preparation of recombinant adenovirus viral vectors are also disclosed. In one embodiment, a composition comprises a cell containing a first delivery plasmid comprising an adenovirus genome lacking a nucleotide sequence encoding fiber and incapable of directing the packaging of new viral particles in the absence of a second delivery plasmid; and a second delivery plasmid comprising an adenoviral genome capable of directing the packaging of new viral particles in the presence of the first delivery plasmid.

In another variation, the first and second delivery plasmids interact within the cell to produce a therapeutic viral vector. In yet another, the cell further comprises a complementing plasmid containing a nucleotide sequence encoding fiber, the plasmid being stably integrated into the cellular genome of the cell. In still another, the first or second delivery plasmid further comprises a nucleotide sequence encoding a foreign polypeptide. In various embodiments, the polypeptide is a therapeutic molecule.

Another embodiment discloses a composition as before, wherein the first delivery plasmid lacks adenovirus packaging signal sequences. In another aspect, the second delivery plasmid contains a LacZ reporter construct. Another variation discloses that the second delivery plasmid further lacks a nucleotide sequence encoding an adenovirus regulatory protein. In one variation, the regulatory protein is E1. In one embodiment of the above-noted compositions, the complementing plasmid has the characteristics of pCLF.

In another embodiment, a composition is disclosed wherein the first delivery plasmid lacks a nucleotide sequence encoding an adenovirus structural protein and the second delivery plasmid lacks a nucleotide sequence encoding adenovirus E1 protein. In another, the first delivery plasmid lacks a nucleotide sequence encoding adenovirus E4 protein and the second delivery plasmid lacks a nucleotide sequence encoding adenovirus E1 protein. In still another, the cell contains at least one complementing plasmid encoding an adenoviral regulatory protein and a structural protein.

In alternative embodiments, the regulatory protein is E4 and the structural protein is fiber; or the regulatory protein is E1 and the structural protein is fiber. In still another embodiment, the adenoviral regulatory protein and the structural protein are encoded by separate complementing plasmids.

Another variation discloses a composition wherein the cell is selected from the group consisting of 293, A549, W162, HeLa, Vero, 211, and 211A. In another embodiment, the delivery plasmid is DV1, or p E1B gal, or p E1sp1B.

Various methods of making and using the vectors, plasmids, cell lines and other compositions and constructs of the present invention are also disclosed herein. The following methods are considered exemplary and not limiting.

Thus, in one variation, the specification discloses a method of constructing therapeutic viral vectors, comprising introducing a delivery plasmid into an Ad fiber-expressing complementing cell line, wherein the DNA sequence encoding Ad fiber protein has been deleted from the delivery plasmid. In one variation, the delivery plasmid further includes a DNA sequence encoding a foreign protein, polypeptide, or fragment thereof. In other embodiments, the delivery plasmid is DV1, p E1B gal, p E1sp1B, or similar constructs.

The specification also discloses methods of transforming a pathologic hyperproliferative mammalian cell comprising contacting the cell with any of the vectors described herein. In another embodiment, methods of infecting a mammalian target cell with a viral vector containing a preselected foreign nucleotide sequence are disclosed. One such variation comprises the following steps: (a) infecting the target cell with a viral vector of the present invention , the viral vector carrying a preselected foreign nucleotide sequence; and (b) expressing the foreign nucleotide sequence in the targeted cell.

The specification also encompasses mammalian target cells infected with a preselected foreign nucleotide sequence produced by the methods disclosed herein. In one variation, the target cells are selected from the group consisting of replicating, slow-replicating and non-replicating human cells.

Methods of treating an acquired or hereditary disease are also disclosed. One method comprises (a) administering a pharmaceutically acceptable dose of a viral vector to a target cell, wherein the vector comprises a preselected therapeutic nucleotide sequence; and (b) expressing the therapeutic sequence in the target cell for a time period sufficient to ameliorate the acquired or hereditary disease in the cell. Method of gene therapy comprising administering to a subject an effective amount of a therapeutic viral vector produced by a packaging cell line of the present invention are also disclosed.

Also contemplated are various methods of inhibiting the proliferation of a tumor in a subject comprising administering an effective amount of a therapeutic viral vector of the present invention under suitable conditions to the subject. In one variation, the gene encodes an anti-tumor agent. In another variation, the agent is a tumor-suppressor gene. In still another embodiment, the agent is a suicide gene or a functional equivalent thereof. In another variation, the vector is administered via intra-tumoral injection,

The specification also discloses systems or kits for use in any of the aforementioned methods. The systems or kits may contain any appropriate combination of the within-described vectors, plasmids, cell lines, and additional therapeutic agents as disclosed. Preferably, each such kit or system includes a quantity of the appropriate therapeutic substance or sequence sufficient for at least one administration, and instructions for administration and use. Thus, one system further comprises an effective amount of a therapeutic agent which enhances the therapeutic effect of the therapeutic viral vector-containing composition. Another variation discloses that the composition and the therapeutic agent are each included in a separate receptacle or container.

It will also be appreciated that any combination of the preceding elements may also be efficacious as described herein.

DETAILED DESCRIPTION To reduce the frequency of contamination with wild-type adenovirus, it is considered desirable to improve either the viral vector or the cell line to reduce the probability of recombination. For example, an adenovirus from a group with less homology to the group C viruses may be used to engineer recombinant viruses with little propensity for recombination with the Ad5 sequence in 293 cells. Similarly, an epithelial cell line -- e.g. the cell line known as 293 - may be used or further modified according to within-disclosed methods which stably expresses adenovirus proteins or polypeptides from Ad3 and/or proteins or polypeptides from another non-group-C or group C serotype; such a cell line would be useful to support adenovirus-derived viral vectors bearing deletions of regulatory and/or structural genes, irrespective of the serotype from which such a vector was derived.

It is also contemplated that the constructs and methods of the present invention will support the design and engineering of chimeric viral vectors which express amino acid residue sequences derived from two or more Ad serotypes. Thus, unlike methods and constructs available prior to the advent of the present disclosure, this specification allows the greatest possible flexibility in the design and preparation of useful viral vectors and cell lines which support their construction and propagation -- all with a decreased risk of recombining with wild-type Ad to produce potentially-harmful recombinants.

In part, the present invention discloses a simpler, alternative means of reducing the recombination between viral and cellular sequences than those discussed in the art. One such means is to increase the size of the deletion in the recombinant virus and thereby reduce the extent of shared sequences between that virus and any Ad genes present in a packaging cell line -- e.g., the Ad5 genes in 293 cells, or the various Ad genes in the novel cell lines of the present invention.

By the term "substantially homologous" is meant having at least 80%, preferably at least 90%, most preferably at least 95% homology therewith.

The amino acid residues described herein are preferably in the "L" isomeric form. However, residues in the "D" isomeric form can be substituted for any L-amino acid residue, as long as the desired functional property is retained by the polypeptide. NH₂ refers to the free amino group present at the amino terminus of a polypeptide.

DNA Homolog: A nucleic acid having a preselected conserved nucleotide sequence and a sequence encoding a preferred polypeptide according to the present invention.

Foreign Gene: This term is used to identify a DNA molecule not present in the exact orientation and position as the counterpart DNA molecule found in wild-type adenovirus. It may also refer to a DNA molecule from another Ad serotype or from an entirely different species -- e.g. a human DNA sequence.

Penton: The terms "penton" or "penton complex" are preferentially used herein to designate a complex of penton base and fiber. The term "penton" may also be used to indicate penton base, as well as penton complex. The meaning of the term "penton" alone should be clear from the context within which it is used.

Polypeptide and Peptide: These terms are used interchangeably herein to designate a series of no more than about 50 amino acid residues connected one to the other by peptide bonds between the alpha-amino and carboxy groups of adjacent residues.

Receptor: Receptor is a term used herein to indicate a biologically active molecule that specifically binds to (or with) other molecules. The term "receptor protein" may be used to more specifically indicate the proteinaceous nature of a specific receptor.

Transgene or Therapeutic Nucleotide Sequence: As described and claimed herein, such a sequence includes DNA and RNA sequences encoding an RNA or polypeptide. Such sequences may be "native" or naturally-derived sequences; they may also be "non-native" or "foreign" sequences which are naturally- or recombinantly-derived. The term "transgene," which may be used interchangeably herein with the term "therapeutic nucleotide sequence," is often used to describe a heterologous or foreign (exogenous) gene that is carried by a viral vector and transduced into a host cell.

Therefore, therapeutic nucleotide sequences include antisense sequences or nucleotide sequences which may be transcribed into antisense sequences. Therapeutic nucleotide sequences (or transgenes) further comprise sequences which function to produce a desired effect in the cell or cell nucleus into which said therapeutic sequences are delivered. For example, a therapeutic nucleotide sequence may encode a functional protein intended for delivery into a cell which is unable to produce that functional protein.

Expression or Delivery Vector: Any plasmid or virus into which a foreign DNA may be inserted for expression in a suitable host cell -- i.e., the protein or polypeptide encoded by the DNA is synthesized in the host cell's system. Vectors capable of directing the expression of DNA segments (genes) encoding one or more proteins are referred to herein as "expression vectors". Also included are vectors which allow cloning of cDNA (complementary DNA) from mRNAs produced using reverse transcriptase.

Adenoviral Vector or Ad-Derived Vector: Any adenovirus-derived plasmid or virus into which a foreign DNA may be inserted or expressed. This term may also be used interchangeably with "viral vector" This "type" of vector may be utilized to carry nucleotide sequences encoding therapeutic proteins or polypeptides to specific cells or cell types in a subject in need of treatment, as described further hereinbelow.

Complementing Plasmid: This term is generally used herein to describe plasmid vectors used to deliver particular nucleotide sequences into a packaging cell line, with the intent of having said sequences stably integrate into the cellular genome.

Delivery Plasmid: This term is generally used herein to describe a plasmid vector that carries or delivers nucleotide sequences in or into a cell line (e.g., a packaging cell line) for the purpose of propagating therapeutic viral vectors of the present invention.

The adenovirus (Ad) particle is relatively complex and may be resolved into various substructures. The outer shell is strikingly icosahedral in shape and, at first glance, appears to have a triangulation number of 25. The structures at the fivefold positions ("pentons") are different from the rest ("hexons"), however, and the hexons are chemically trimers rather than hexamers. Thus, the structure really does not correspond to a simple sub-triangulated icosahedral design. (See, e.g., Fields, et al., Virology, Vol. I, Raven Press, NY, pp. 54-56 (1990).)

Fiber plays a crucial role in adenovirus infection by attaching the virus to a specific receptor on the cell surface. The fiber consists of three domains: an N-terminal tail that interacts with penton base; a shaft composed of 22 repeats of a 15-amino-acid segment that forms -sheet and -bends; and a knob at the C-terminus that contains the type-specific antigen and is responsible for binding to the cell surface receptor. The fiber protein is also responsible for transport of viral nucleic acids into the nucleus. The gene encoding the fiber protein from Ad2 has been expressed in human cells and has been shown to be correctly assembled into trimers, glycosylated and transported to the nucleus. (See, e.g., Hong and Engler, Virology 185: 758-761 (1991).) Thus, alteration of gene delivery mediated by recombinant adenovirus vectors to specific cell types has great utility for a variety of gene therapy applications and is thus one of the objects of the present invention.

Hexon and penton capsomeres are the major components on the surface of the virion. Their constituent polypeptides, nos. II, III and IV, contain tyrosine residues that are exposed on the surface of the virion and can be labeled -- e.g., by iodination of intact particles.

The fiber is an elongated protein which exists as a trimer of three identical polypeptides (polypeptide IV) of 582 amino acids in length. The N-terminus of the fiber mediates binding to the penton base to form what is generally called the penton capsomere. The C-terminus of the fiber is involved in initial binding of the virus to cellular receptors.

The 35,000+ base pair (bp) genome of adenovirus type 2 has been sequenced and the predicted amino acid sequences of the major coat proteins (hexon, fiber and penton base) have been described. (See, e.g., Neumann et al., Gene 69: 153-157 (1988); Herisse et al., Nuc. Acids Res. 9: 4023-4041 (1981); Roberts et al., J. Biol. Chem. 259: 13968-13975 (1984); Kinloch et al., J. Biol. Chem. 259: 6431-6436 (1984); and Chroboczek et al., Virol. 161: 549-554 (1987).)

The sequence of Ad5 DNA was completed more recently; its sequence includes a total of 35,935 bp. Portions of many other adenovirus genomes have also been sequenced. It is presently understood that the upper packaging limit for adenovirus virions is about 105% of the wild-type genome length. (See, e.g., Bett, et al., J. Virol. 67(10): 5911-21 (1993).) Thus, for Ad2 and Ad5, this would be an upper packaging limit of about 38kb of DNA.

Adenovirus DNA also includes inverted terminal repeat sequences (ITRs) ranging in size from about 100 to 150 bp, depending on the serotype. The inverted repeats enable single strands of viral DNA to circularize by base-pairing of their terminal sequences, and the resulting base-paired "panhandle" structures are thought to be important for replication of the viral DNA.

For efficient packaging, the ITRS and the packaging signal (a few hundred bp in length) appear to comprise the "minimum requirement." Helper-dependent vectors lacking all viral ORFs but including these essential cis elements (the ITRs and contiguous packaging sequence) have been constructed, but the virions package less efficiently that the helper and package as multimers part of the time, which suggests that the virus may "want" to package a fuller DNA complement (see, e.g., Fisher, et al., Virology 217: 11-22 (1996).

While some prefer to use replication-defective Ad viral vectors for fear that replication-competent vectors raise safety issues, the viral vectors disclosed herein may retain their ability to express the genome packaged within -- i.e., they may retain their "infectivity" -- they do not act as infectious agents to the extent that they cause disease in the subjects to whom they are administered for therapeutic purposes.

It is to be appreciated that the viral vectors have several distinct advantages over adenoviral and Ad-derived vectors described in the art. For example, recombination of such vectors is rare; there are no known associations of human malignancies with adenoviral infections despite common human infection with adenoviruses; the genome may be manipulated to accommodate foreign genes of a fairly substantial size; and host proliferation is not required for expression of adenoviral proteins.

An extension of this invention is that the Ad-derived viral vectors disclosed herein may be used to target and deliver genes into specific cells by incorporating the attachment sequence for other receptors (such as CD4) onto the fiber protein by recombinant DNA techniques, thus producing a chimeric molecule. This should result in the ability to target and deliver genes into a wide range of cell types with the advantage of evading recognition by the host's immune system. The within-disclosed delivery systems thus provide for increased flexibility in gene design to enable stable integration into proliferating and nonproliferating cell types.

For example, published International App. No. WO95/26412 and Krasnykh, et al. (J. Virol. 70: 6839-46 (1996)), describe modifications that may be made to the adenovirus fiber protein. Such modifications are useful in altering the targeting mechanism and specificity of adenovirus and could readily be utilized in conjunction with the constructs of the present invention to target the novel viral vectors disclosed herein to different receptors and different cells. Moreover, modifications to fiber protein which alter its tropism may permit greater control over the localization of viral vectors in therapeutic applications.

Similarly, incorporation of various structural proteins into cell lines of the present invention, whether or not those proteins are modified, is also contemplated by the present invention. Thus, for example, modified penton base polypeptides such as those described in Wickham, et al. (J Virol. 70: 6831-8 (1996)) may have therapeutic utility when used according to the within-disclosed methods.

### C. Packaging Cell Lines

The first generation of recombinant adenoviral vectors currently available tend to have a deletion in the first viral early gene region which is generally referred to as E1, which comprises the E1a and E1b regions. (These regions typically span genetic map units 1.30 to 9.24.) Figure 3 in chapter 67 of Fields Virology, 3d Ed. (Fields et al. (eds.), Lippincott-Raven Publ., Philadelphia, (1996), p. 2116) illustrates a transcription and translation map of adenovirus type 2 (Ad2) that is a helpful example.

According to various published reports, deletion of the viral E1 region renders the recombinant adenovirus defective for replication and incapable of producing infectious viral particles in the subsequently-infected target cells. Thus, the ability to generate E1-deleted adenovirus is often based on the availability of the human embryonic kidney packaging cell line called 293. This cell line contains the E1 region of adenovirus, which provides E1 gene region products to "support" the growth of E1-deleted virus in the cell line (see, e.g., Graham et al., J. Gen. Virol. 36: 59-71 (1977)).

Nevertheless, the inherent problems with current first-generation recombinant adenoviruses have raised increasing concerns about their use in patients. For example, several recent studies have shown that E1-deleted adenoviruses are not completely replication-incompetent (see Rich, Hum. Gene. Ther. 4: 461-476 (1993); Engelhardt, et al., Nature Genet. 4: 27-34 (1993)).

Three general limitations are associated with the adenoviral vector technology. First, infection both *in vivo* and *in vitro* with the adenoviral vector at high multiplicity of infection ("MOI") has resulted in cytotoxicity to the target cells, due to the accumulation of penton protein, which is itself toxic to mammalian cells (Kay, Cell Biochem. 17E: 207 (1993)). Second, host immune responses against adenoviral late gene products, including penton protein, cause the inflammatory response and destruction of the infected tissue which received the vectors (Yang, et al., PNAS USA 92: 4407-4411 (1994)). Lastly, host immune responses and cytotoxic effects together prevent the long-term expression of transgenes and cause decreased levels of gene expression following subsequent administration of adenoviral vectors (Mittal, et al., Virus Res. 28: 67-90 (1993)).

The packaging cell lines disclosed herein support viral vectors with deletions of major portions of the viral genome, without the need for helper viruses.

### D. Therapeutic Viral Vectors and Related Systems

### 1. Nucleic Acid Segments

A therapeutic viral vector or composition comprises a nucleotide sequence encoding a protein or polypeptide molecule -- or a biologically active fragment thereof -- which may be used for therapeutic applications, as described herein. A therapeutic viral vector or composition may further comprise an enhancer element or a promoter located 5' to and controlling the expression of such a therapeutic nucleotide sequence or gene.

In general, promoters are DNA segments that contain a DNA sequence that controls the expression of a gene located 3' or downstream of the promoter. The promoter is the DNA sequence to which RNA polymerase specifically binds and initiates RNA synthesis (transcription) of that gene, typically located 3' of the promoter. If more than one nucleic acid sequence encoding a particular polypeptide or protein is included in a therapeutic viral vector or nucleotide sequence, more than one promoter or enhancer element may be included, particularly if that would enhance efficiency of expression. For purposes of the present invention, regulatable (inducible) as well as constitutive promoters may be used, either on separate vectors or on the same vector.

A subject therapeutic nucleotide composition or vector consists of a nucleic acid molecule that comprises at least 2 different operatively linked DNA segments. The DNA can be manipulated and amplified by PCR as described herein and by using standard techniques, such as those described in Molecular Cloning: A Laboratory Manual, 2nd Ed., Sambrook et al., eds., Cold Spring Harbor, New York (1989). Typically, to produce a therapeutic viral vector of the present invention, the sequence encoding the selected therapeutic composition and the promoter or enhancer are operatively linked to a DNA molecule capable of autonomous replication in a cell either *in vivo* or *in vitro.* By operatively linking the enhancer element or promoter and the nucleotide sequence encoding the therapeutic nucleotide composition to the vector, the attached segments are replicated along with the vector sequences.

Thus, a recombinant DNA molecule (rDNA) is a hybrid DNA molecule comprising at least 2 nucleotide sequences not normally found together in nature. In various preferred embodiments, one of the sequences is a sequence encoding an Ad-derived polypeptide, protein, or fragment thereof. Stated another way, a therapeutic nucleotide sequence is one that encodes an expressible protein, polypeptide or fragment thereof, and it may further include an active constitutive or regulatable (e.g. inducible) promoter sequence.

A therapeutic viral vector or composition is optimally from about 20 base pairs to about 40,000 base pairs in length. Preferably the nucleic acid molecule is from about 50 bp to about 38,000 bp in length. In various embodiments, the nucleic acid molecule is of sufficient length to encode one or more adenovirus proteins or functional polypeptide portions thereof. Since individual Ad polypeptides vary in length from about 19 amino acid residues to about 967 amino acid residues, corresponding nucleotide sequences will range from about 50 bp up to about 3000 bp, depending on the size and of individual polypeptide-encoding sequences that are "replaced" in the viral vectors by therapeutic nucleotide sequences of the present invention.

Various Ad proteins are comprised of more than one polypeptide sequence. Thus, deletion of the corresponding genes from an Ad vector as taught herein will thus allow the vector to accommodate even larger "foreign" DNA segments. Thus, if the sequences encoding one or more adenovirus polypeptides or proteins are supplanted by a recombinant nucleotide sequence of the present invention, the length of the recombinant sequence can conceivably extend nearly to the packaging limit of the relevant adenovirus-derived vector.

In view of the fact that preferred embodiments disclosed herein are helper-independent Ad-derived vectors, the entire wild-type Ad genome cannot be completely supplanted by recombinant nucleic acid molecules without transforming such a vector into a vector requiring "help" of some kind. However, the Ad-derived vectors disclosed herein do not depend on a helper virus; instead, the vectors are propagated in cell lines stably expressing proteins or polypeptides that have been removed from said vectors to allow the addition of "foreign" DNA into the vectors. In various disclosed embodiments, specific early region and structural polypeptides are deleted from the vectors thereby enabling the vectors to accommodate recombinant nucleic acid sequences (or cassettes) of various lengths. For example, Ad-derived vectors may easily include 12 kb or more of foreign (or "therapeutic") DNA sequences.

The therapeutic (or foreign) nucleotide sequence can be a gene or gene fragment that encodes a protein or polypeptide -- or a biologically active fragment thereof - that provides a desired therapeutic effect such as replacement of alpha 1-antitrypsin or cystic fibrosis transmembrane conductance regulator protein (CFTR) and the like. (See, e.g., Crystal, et al., Nature Genetics 8: 42-51 (1994); Zabner, et al., Cell 75: 207-216 (1993); Knowles, et al., NEJM 333(13): 823-831 (1995); and Rosenfeld, et al., Cell 68: 143-155 (1992).

An Ad-derived vector may also comprise a nucleotide sequence encoding a protein, polypeptide or fragment thereof that is effective in regulating the cell cycle -- such as p53, Rb, or mitosin -- or which is effective in inducing cell death, such as thymidine kinase. (See, e.g., published International App. No. WO 95/11984) It is further contemplated that a therapeutic protein or polypeptide expressed by a therapeutic viral vector may be used in conjunction with another therapeutic agent when appropriate -- e.g., a thymidine kinase metabolite may be used in conjunction with the gene encoding thymidine kinase and its gene product -- in order to be even more effective.

Alternatively, a therapeutic viral vector can include a DNA or RNA oligonucleotide sequence that exhibits enzymatic therapeutic activity without needing to be translated into a polypeptide product before exerting a therapeutic effect. Examples of the latter include antisense oligonucleotides that will inhibit the transcription of deleterious genes or ribozymes that act as site-specific ribonucleases for cleaving selected mutated gene sequences. In another variation, a therapeutic nucleotide sequence may comprise a DNA construct capable of generating therapeutic nucleotide molecules, including ribozymes and antisense DNA, in high copy numbers in target cells, as described in published PCT application No. WO 92/06693. Other preferred therapeutic nucleotide sequences are capable of delivering HIV antisense nucleotides to latently-infected T cells via CD4. Similarly, delivery of Epstein-Barr Virus (EBV) EBNa-1 antisense nucleotides to B cells via CR2 is capable of effecting therapeutic results.

As noted elsewhere herein, an Ad-derived vector may also include a promoter sequence. Both constitutive and regulatable (often called "inducible") promoters are useful in constructs and methods of the present invention. For example, some useful regulatable promoters are those of the CREB-regulated gene family and include - and -inhibin, - gonadotropin, cytochrome c, glucagon, and the like. (See, e.g., published International App. No. WO96/14061)

A regulatable or inducible promoter may be described as a promoter wherein the rate of RNA polymerase binding and initiation is modulated by external stimuli. Such stimuli include various compounds or compositions, light, heat, stress, chemical energy sources, and the like. Inducible, suppressible and repressible promoters are considered regulatable promoters.

Regulatable promoters may also include tissue-specific promoters. Tissue-specific promoters direct the expression of the gene to which they are operably linked to a specific cell type. Tissue-specific promoters cause the gene located 3' of it to be expressed predominantly, if not exclusively, in the specific cells where the promoter expressed its endogenous gene. Typically, it appears that if a tissue-specific promoter expresses the gene located 3' of it at all, then it is expressed appropriately in the correct cell types (see, e.g., Palmiter et al., Ann. Rev. Genet. 20: 465-499 (1986)).

When a tissue-specific promoter controls the expression of a gene, that gene will be expressed in a small number of tissues or cell types rather than in substantially all tissues and cell types. Examples of tissue-specific promoters include the immunoglobulin promoter described by Brinster et al., Nature 306: 332-336 (1983) and Storb et al., Nature 310: 238-231 (1984); the elastase-I promoter described by Swift et al., Cell 38: 639-646 (1984); the globin promoter described by Townes et al., Mol. Cell. Biol. 5: 1977-1983 (1985), and Magram et al., Mol. Cell. Biol. 9: 4581-4584 (1989), the insulin promoter described by Bucchini et al., PNAS USA, 83: 2511-2515 (1986) and Edwards et al., Cell 58: 161 (1989); the immunoglobulin promoter described by Ruscon et al., Nature 314: 330-334 (1985) and Grosscheld et al., Cell 38: 647-658 (1984); the alpha actin promoter described by Shani, Mol. Cell. Biol. 6: 2624-2631 (1986); the alpha crystalline promoter described by Overbeek et al., PNAS USA 82: 7815-7819 (1985); the prolactin promoter described by Crenshaw et al., Genes and Development 3: 959-972 (1989); the proopiomelanocortin promoter described by Tremblay et al., PNAS USA 85: 8890-8894 (1988); the beta-thyroid stimulating hormone (BTSH) promoter described by Tatsumi et al., Nippon Rinsho 47: 2213-2220 (1989); the mouse mammary tumor virus (MMTV) promoter described by Muller et al., Cell 54: 105 (1988); the albumin promoter described by Palmiter et al., Ann. Rev. Genet. 20: 465-499 (1986); the keratin promoter described by Vassar et al., PNAS USA 86: 8565-8569 (1989); the osteonectin promoter described by McVey et al., J. Biol. Chem. 263: 11,111-11,116 (1988); the prostate-specific promoter described by Allison et al., Mol. Cell. Biol. 9: 2254-2257 (1989); the opsin promoter described by Nathans et al., PNAS USA 81: 4851-4855 (1984); the olfactory marker protein promoter described by Danciger et al., PNAS USA 86: 8565-8569 (1989); the neuron-specific enolase (NSE) promoter described by Forss-Pelter et al., J. Neurosci. Res. 16: 141-151 (1986); the L-7 promoter described by Sutcliffe, Trends in Genetics 3: 73-76 (1987) and the protamine 1 promoter described Peschon et al., Ann. New York Acad. Sci. 564: 186-197 (1989) and Braun et al., Genes and Development 3: 793-802 (1989).

### 2. Compositions

In various alternative embodiments, therapeutic sequences and compositions useful for practicing the therapeutic methods described herein are contemplated. Therapeutic compositions may contain a physiologically tolerable carrier together with one or more therapeutic nucleotide sequences, dissolved or dispersed therein as an active ingredient. In a preferred embodiment, the composition is not immunogenic or otherwise able to cause undesirable side effects when administered to a subject for therapeutic purposes.

As used herein, the terms "pharmaceutically acceptable", "physiologically tolerable" and grammatical variations thereof, as they refer to compositions, carriers, diluents and reagents, are used interchangeably and represent that the materials are capable of administration to or upon a subject -- e.g., a mammal -- without the production of undesirable physiological effects such as nausea, dizziness, gastric upset and the like.

For example, the specification discloses therapeutic compositions useful in the specific targeting of epithelial or non-epithelial cells as well as in delivering a therapeutic nucleotide sequence to those cells. Therapeutic compositions designed to preferentially target to epithelial cells may comprise an adenovirus-derived vector including a therapeutic nucleotide sequence. As described herein, a number of adenovirus-derived moieties are useful in the presently-disclosed therapeutic compositions and methods.

While some of the Examples appearing below specifically recite fiber proteins, polypeptides, and fragments thereof, it is expressly provided herein that other structural and non-structural Ad proteins and polypeptides (e.g., regulatory protein s and polypeptides) may be used as components of the various disclosed vectors and cell lines. Moreover, chimeric molecules comprised of proteins, polypeptides, and/or fragments thereof which are derived from different Ad serotypes may be used in any of the within-disclosed methods, constructs and compositions. Similarly, recombinant DNA sequences may be prepared using nucleic acid sequences derived from different Ad serotypes, in order to design useful constructs with broad applicability, as disclosed herein.

It should also be appreciated that, while the members of Group C adenovirus -- i.e., Ad serotypes 1,2, 5, and 6 -- are specifically recited in various examples herein, the present specification is in no way limited to those serotypes alone. In view of the fact that the adenovirus serotypes are all closely-related in structure and functionality, therapeutic viral vectors, packaging cell lines, and plasmids may be constructed from components of any and all Ad serotypes -- and the within-disclosed methods of making and using various constructs and the inventive cell lines apply to all of said serotypes.

The preparation of a pharmacological composition that contains active ingredients dissolved or dispersed therein is well understood in the art. Typically such compositions are prepared as injectables -- either as liquid solutions or suspensions -- however, solid forms suitable for solution or suspension in liquid prior to use can also be prepared. A preparation can also be emulsified, or formulated into suppositories, ointments, creams, dermal patches, or the like, depending on the desired route of administration.

The active ingredient can be mixed with excipients which are pharmaceutically acceptable and compatible with the active ingredient and in amounts suitable for use in the therapeutic methods described herein. Suitable excipients are, for example, water, saline, dextrose, glycerol, ethanol or the like and combinations thereof, including vegetable oils, propylene glycol, polyethylene glycol and benzyl alcohol (for injection or liquid preparations); and petrolatum (e.g., VASELINE), vegetable oil, animal fat and polyethylene glycol (for externally applicable preparations). In addition, if desired, the composition can contain wetting or emulsifying agents, isotonic agents, dissolution promoting agents, stabilizers, colorants, antiseptic agents, soothing agents and the like additives (as usual auxiliary additives to pharmaceutical preparations), pH buffering agents and the like which enhance the effectiveness of the active ingredient.

The therapeutic compositions can include pharmaceutically acceptable salts of the components therein. Pharmaceutically acceptable salts include the acid addition salts (formed with the free amino groups of the polypeptide) that are formed with inorganic acids such as, for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like.

Physiologically tolerable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, polyethylene glycol and other solutes.

Liquid compositions can also contain liquid phases in addition to and to the exclusion of water. Exemplary of such additional liquid phases are glycerin, vegetable oils such as cottonseed oil, and water-oil emulsions.

A therapeutic composition typically contains an amount of a therapeutic nucleotide sequence of the present invention sufficient to deliver a therapeutically effective amount to the target tissue, typically an amount of at least 0.1 weight percent to about 90 weight percent of therapeutic nucleotide sequence per weight of total therapeutic composition. A weight percent is a ratio by weight of therapeutic nucleotide sequence to total composition. Thus, for example, 0.1 weight percent is 0.1 grams of DNA segment per 100 grams of total composition.

The therapeutic nucleotide compositions comprising synthetic oligonucleotide sequences can be prepared using any suitable method, such as, the phosphotriester or phosphodiester methods. See Narang et al., Meth. Enzymol., 68:90, (1979); U.S. Patent No. 4,356,270; and Brown et al., Meth. Enzymol., 68:109, (1979).

For therapeutic oligonucleotides sequence compositions in which a family of variants is preferred, the synthesis of the family members can be conducted simultaneously in a single reaction vessel, or can be synthesized independently and later admixed in preselected molar ratios. For simultaneous synthesis, the nucleotide residues that are conserved at preselected positions of the sequence of the family member can be introduced in a chemical synthesis protocol simultaneously to the variants by the addition of a single preselected nucleotide precursor to the solid phase oligonucleotide reaction admixture when that position number of the oligonucleotide is being chemically added to the growing oligonucleotide polymer. The addition of nucleotide residues to those positions in the sequence that vary can be introduced simultaneously by the addition of amounts, preferably equimolar amounts, of multiple preselected nucleotide precursors to the solid phase oligonucleotide reaction admixture during chemical synthesis. For example, where all four possible natural nucleotides (A,T,G and C) are to be added at a preselected position, their precursors are added to the oligonucleotide synthesis reaction at that step to simultaneously form four variants.

This manner of simultaneous synthesis of a family of related oligonucleotides has been previously described for the preparation of "Degenerate Oligonucleotides" by Ausubel et al. (Current Protocols in Molecular Biology, Suppl. 8. p.2.11.7, John Wiley & Sons, Inc., New York (1991)), and can readily be applied to the preparation of the therapeutic oligonucleotide compositions described herein.

Nucleotide bases other than the common four nucleotides (A,T,G or C), or the RNA equivalent nucleotide uracil (U), can also be used in the present invention. For example, it is well known that inosine (I) is capable of hybridizing with A, T and G, but not C. Examples of other useful nucleotide analogs are known in the art; many may be found listed in 37 C.F.R. §1.822.

Thus, where all four common nucleotides are to occupy a single position of a family of oligonucleotides, that is, where the preselected therapeutic nucleotide composition is designed to contain oligonucleotides that can hybridize to four sequences that vary at one position, several different oligonucleotide structures are contemplated. The composition can contain four members, where a preselected position contains A,T,G or C. Alternatively, the composition can contain two members, where a preselected position contains I or C, and has the capacity the hybridize at that position to all four possible common nucleotides. Finally, other nucleotides may be included at the preselected position that have the capacity to hybridize in a non-destabilizing manner with more than one of the common nucleotides in a manner similar to inosine.

### 3. Expression Vector Systems

The introduction of exogenous DNA into eucaryotic cells has become one of the most powerful tools of the molecular biologist. The term "exogenous" encompasses any therapeutic composition of this invention which is administered by the therapeutic methods of this invention. Thus, "exogenous" may also be referred to herein as "foreign," "non-native," and the like. The methods disclosed herein preferably require efficient delivery of the DNA into the nucleus of the recipient cell and subsequent identification of cells that are expressing the foreign DNA.

A widely-used plasmid is pBR322, a vector whose nucleotide sequence and endonuclease cleavage sites are well known. Various other useful plasmid vectors are described in the Examples that follow.

A vector used herein comprises a nucleic acid (preferably DNA) molecule capable of autonomous replication in a cell and to which a DNA segment, e.g., a gene or polynucleotide, can be operatively linked so as to bring about replication of the attached segment. Herein, one of the nucleotide segments to be operatively linked to vector sequences encodes at least a portion of a therapeutic nucleic acid molecule -- in effect, a nucleic acid sequence that encodes one or more therapeutic proteins or polypeptides, or fragments thereof.

In various embodiments, the entire peptide-coding sequence of the therapeutic gene is inserted into the vector and expressed; however, it is also feasible to construct a vector which also includes some non-coding sequences as well. Preferably, however, non-coding sequences are excluded. Alternatively, a nucleotide sequence for a soluble form of a polypeptide may be utilized. Another preferred therapeutic viral vector includes a nucleotide sequence encoding at least a portion of a therapeutic nucleotide sequence operatively linked to the vector for expression. As used herein with regard to DNA sequences or segments, the phrase "operatively linked" generally means the sequences or segments have been covalently joined into one piece of DNA, whether in single or double stranded form.

The choice of viral vector into which a therapeutic nucleotide sequence is operatively linked depends directly, as is well known in the art, on the functional properties desired, e.g., vector replication and protein expression, and the host cell to be transformed -- these being limitations inherent in the art of constructing recombinant DNA molecules. Although certain adenovirus serotypes are recited herein in the form of specific examples, it should be understood that the present invention contemplates the use of *any* adenovirus serotype, including hybrids and derivatives thereof. As one will observe, it is not unusual to utilize nucleotide and/or amino acid residue sequences of two or more serotypes in constructs, compositions and methods disclosed herein.

As one of skill in the art will note, in various embodiments described herein, different "types" of vectors are disclosed. For example, one "type" of vector is used to deliver particular nucleotide sequences into a packaging cell line, with the intent of having said sequences stably integrate into the cellular genome; these "types" of vectors are generally identified herein as complementing plasmids. A further "type" of vector described herein carries or delivers nucleotide sequences in or into a cell line (e.g., a packaging cell line) for the purpose of propagating therapeutic viral vectors; hence, these vectors are generally referred to herein as delivery plasmids. A third "type" of vector described herein is utilized to carry nucleotide sequences encoding therapeutic proteins or polypeptides to specific cells or cell types in a subject in need of treatment; these vectors are generally identified herein as therapeutic viral vectors or Ad-derived vectors.

In one embodiment, the directional ligation means is provided by nucleotides present in the upstream nucleotide sequence, downstream nucleotide sequence, or both. In another embodiment, the sequence of nucleotides adapted for directional ligation comprises a sequence of nucleotides that defines multiple directional cloning means. Where the sequence of nucleotides adapted for directional ligation defines numerous restriction sites, it is referred to as a multiple cloning site.

A translatable nucleotide sequence is a linear series of nucleotides that provide an uninterrupted series of at least 8 codons that encode a polypeptide in one reading frame. Preferably, the nucleotide sequence is a DNA sequence. The vector itself may be of any suitable type, such as a viral vector (RNA or DNA), naked straight-chain or circular DNA, or a vesicle or envelope containing the nucleic acid material and any polypeptides that are to be inserted into the cell.

A preferred viral vector in which therapeutic nucleotide compositions are present is derived from adenovirus (Ad). It is also desirable that the vector contain a promoter sequence. As taught herein, viral vectors may be designed and constructed in such a way that they specifically target a preselected recipient cell type, depending on the nature of therapy one seeks to administer. Methods of making and using therapeutic viral vectors that target specific cells are further described in the Examples that follow.

Vectors and compositions may also be designed and prepared to preferentially target cells that might not otherwise be targeted by wild-type adenovirus virions. For example, in order to target non-epithelial cells, one following the teachings of the present specification may be able to prepare a therapeutic vector including a nucleotide sequence encoding a foreign protein, polypeptide or other ligand directed to a non-epithelial cell or to a different receptor than that generally targeted by a particular adenovirus. Examples of useful ligands directed to specific receptors (identified in parentheses) include the V3 loop of HIV gp120 (CD4); transferrin (transferrin receptor); LDL (LDL receptors); and deglycosylated proteins (asialoglycoprotein receptor). Various useful ligands which may be added to adenovirus fiber -- and methods for preparing and attaching same -- are set forth in published International App. No. WO95/26412.

Useful ligands which may be encoded by a foreign nucleotide sequence contained within a viral vector, or which may be linked to proteins or polypeptides expressed thereby after said vectors are administered to a subject, also include antibodies and attachment sequences, as well as receptors themselves. For example, antibodies to cell receptor molecules such as integrins and the like, MHC Class I and Class II, asialoglycoprotein receptor, transferrin receptors, LDL receptors, CD4, and CR2 are but a few which are useful. It is also understood that the ligands typically bound by receptors, as well as analogs to those ligands, may be used as cellular targeting agents, as disclosed herein.

### E. Therapeutic Methods

The vectors disclosed herein are particularly suited for gene therapy. Thus, various therapeutic methods are contemplated.

For example, it has now been discovered that Ad-derived viral vectors are capable of delivering a therapeutic nucleotide sequence to a specific cell or tissue, thereby expanding and enhancing treatment options available in numerous conditions in which more conventional therapies are of limited efficacy. Accordingly, methods of gene therapy utilizing these vectors are contemplated. Vectors are typically purified and then an effective amount is administered *in vivo* or *ex vivo* into the subject.

For example, the compositions may be used prophylactically or therapeutically *in vivo* to disrupt HIV infection and mechanisms of action by inhibiting gene expression or activation, via delivery of antisense HIV sequences or ribozymes to T cells or monocytes. Using methods disclosed herein, one may target therapeutic viral vectors as disclosed herein to specific cells and tissues, including hematopoietic cells, as infection of such cells appears to be mediated by distinct integrins to which viral vectors discussed herein may readily be targeted. (See, e.g., Huang, et al., J. Virol. 70: 4502-8 (1996).)

Other useful therapeutic nucleotide sequences include antisense nucleotide sequences complementary to EBV EBNa-1 gene. Use of such therapeutic sequences may remediate or prevent latent infection ofB cells with EBV. As discussed herein and in the Examples below, targeting and delivery may be accomplished via the use of various ligands, receptors, and other appropriate targeting agents.

Thus, in one embodiment, a therapeutic method comprises contacting the cells of a subject infected with EBV or HIV with a therapeutically effective amount of a pharmaceutically acceptable composition comprising a therapeutic nucleotide sequence of this invention. In a related embodiment, the contacting involves introducing the therapeutic nucleotide sequence composition into cells having an EBV or HIV-mediated infection.

Methods of gene therapy are well known in the art (see, e.g., Larrick and Burck, Gene Therapy: Application of Molecular Biology, Elsevier Science Publ. Co., Inc., New York, NY (1991); Kriegler, Gene Transfer and Expression: A Laboratory Manual, W. H. Freeman and Company, New York (1990)). The term "subject," should be understood to include any animal -- particularly mammalian -- patient, such as any murine, rat, bovine, porcine, canine, feline, equine, ursine, or human patient.

When the foreign gene carried in the vector encodes a tumor suppressor gene or another anti-tumor protein, the vector is useful to treat or reduce hyperproliferative cells in a subject, to inhibit tumor proliferation in a subject or to ameliorate a particular, related pathology. Pathologic hyperproliferative cells are characteristic of various disease states, such as thyroid hyperplasia, psoriasis, eczema, benign prostatic hypertrophy, Li-Fraumeni syndrome including breast cancer, sarcomas and other neoplasms, bladder cancer, colon cancer, lung cancer, various leukemias, and lymphomas.

Non-pathologic hyperproliferative cells are found, for example, in cells associated with wound repair. Pathologic hyperproliferative cells, however, characteristically exhibit loss of contact inhibition and a decline in their ability to selectively adhere which implies a change in the surface properties of the cell and a further breakdown in intercellular communication. These changes include stimulation to divide and the ability to secrete proteolytic enzymes.

The present specification describes methods of depleting suitable samples of pathologic mammalian hyperproliferative cells contaminating hematopoietic precursors during bond marrow reconstitution via the introduction of a wild-type tumor suppressor gene into the cell preparation using a vector described herein. As used herein, a suitable sample is defined as a heterogeneous cell preparation obtained from a patient, e.g., a mixed population of cells containing both phenotypically normal and pathogenic cells.

Administration includes -- but is not limited to -- the introduction of therapeutic agents into a cell or subject via various means, including direct injection, intravenously, intraperitoneally, via intra-tumor injection, via aerosols, or topically. Therapeutic agents as disclosed herein may also be combined for administration of an effective amount of the agents with a pharmaceutically-acceptable carrier, as described herein.

As used herein, "effective amount" generally means the amount of vector (or proteins produced/released thereby) which achieves a positive outcome in the subject to whom the vector is administered. The total volume administered will necessarily vary depending on the mode of administration, as those of skill in the relevant art will appreciate, and dosages may vary as well.

The dose of a biologic vector is somewhat complex and may be described in terms of the concentration (in plaque-forming units per milliliter (pfu/ml)), the total dose (in pfus), and the estimated number of vectors administered per cell (the estimated multiplicity of infection or MOI). Thus, if a vector is administered via infusion - say, across nasal epithelium -- at a constant total volume, the respective concentration, etc. may be described as follows:

| Concentration (pfu/ml) | Volume (ml) | Dose (pfu) | Estimated MOI |
|---|---|---|---|
| 10⁷ | 2 | 2x10⁷ | 1 |
| 10⁸ | 2 | 2x10⁸ | 10 |
| 10⁹ | 2 | 2x10⁹ | 100 |
| 10¹⁰ | 2 | 2x10¹⁰ | 1000 |

In general, when adenoviral vectors are administered via infusion across the nasal epithelium, administered amounts producing an estimated MOI of about 10 or greater are much more effective than lower dosages. (See, e.g., Knowles, et al., New Eng. J. Med. 333: 823-831 (1995).) Similarly, when direct injection is the preferred treatment modality -- e.g., direct injection of a viral vector into a tumor -- doses of 1 x 10⁹ pfu or greater are generally preferred. (See, e.g., published International App. No. WO95/11984.)

Thus, depending on the mode of administration, an effective amount administered in a single dose preferably contains from about 10⁶ to about 10¹⁵ infectious units. A typical course of treatment would be one such dose per day over a period of five days. As those of skill in the art will appreciate, an effective amount may vary depending on (1) the pathology or other condition to be treated, (2) the status and sensitivity of the patient, and (3) various other factors well known to those of skill in the art, such as the patient's tolerance to other courses of treatment that may have been applied previously. Thus, those of skill in the art may easily and precisely determine effective amounts of agents/vectors which may be administered to a particular patient, based on their understanding of and evaluation of such factors.

The present specification discloses methods of ameliorating pathologies characterized by hyperproliferative cells or genetic defects in a subject, by administering to the subject an effective amount of a vector as described herein. Such vectors preferably contain a foreign gene encoding a gene product (e.g. polypeptide or protein) having the ability to ameliorate the pathology, under suitable conditions. As used herein, the term "genetic defect" means any disease, condition or abnormality which results from inherited factors, e.g. Huntington's Disease, Tay-Sachs Disease, or Sickle Cell Disease.

The specification further describes methods for reducing the proliferation of tumor cells in a subject by introducing into the tumor mass an effective amount of an adenoviral expression vector containing an anti-tumor gene other than a tumor suppressor gene. The anti-tumor gene can encode, for example, thymidine kinase (TK). An effective amount of a therapeutic agent is then administered to the subject; the therapeutic agent, in the presence of the anti-tumor gene, is toxic to the cell.

Using thymidine kinase as exemplary, the therapeutic agent is a thymidine kinase metabolite such as ganciclovir (GCV), 6-methoxypurine arabinonucleoside (araM), or a functional equivalent thereof. Both the thymidine kinase gene and the thymidine kinase metabolite must be used concurrently in order to exert a toxic effect on the host cell. In the presence of the TK gene, GCV is phosphorylated and becomes a potent inhibitor of DNA synthesis, whereas araM is converted to the cytotoxic anabolite araATP. Thus, the precise method of action or synergism is not relevant to therapeutic efficacy, what is relevant is the fact that the concurrent use of appropriate genes and therapeutic agents may effectively ameliorate a specific disease condition.

Another useful example contemplates use of a vector which expresses the enzyme cytosine deaminase. Such a vector could be used in conjunction with administration of the drug 5-fluorouracil (Austin and Huber, Mol. Pharm. 43: 380-387 (1993)) or the recently-described *E. coli* Deo gene in combination with 6-methyl-purine-2'-deoxyribonucleoside (Sorscher et al., Gene Therapy 1: 233-238 (1994)).

As with the use of the tumor suppressor genes described previously, the use of other anti-tumor genes, either alone or in combination with the appropriate therapeutic agent, provides a treatment for the uncontrolled cell growth or proliferation characteristic of tumors and malignancies. Thus, the specification describes therapies to halt the uncontrolled cellular growth in a patient, thereby alleviating the symptoms or the disease or cachexia present in the patient. The effect of this treatment includes, but is not limited to, prolonged survival time of the patient, reduction in tumor mass or burden, apoptosis of tumor cells, or the reduction in the number of circulating tumor cells. Means of quantifying the beneficial effects of this therapy are well known to those of skill in the art.

The specification a recombinant adenovirus expression vector characterized by the partial or total deletion of one or more adenoviral structural protein genes, such as the gene encoding fiber, which allows the vector to accommodate a therapeutic, foreign nucleic acid sequence encoding a functional foreign polypeptide, protein, or biologically active fragment thereof. For example, such a functional polypeptide moiety may be a suicide gene or a functional equivalent thereof, of which the anti-cancer gene TK is but one example. TK genes, when expressed, produce a gene product which is lethal to the cell, particularly in the presence of GCV. One source of the TK gene is the herpes simplex virus (HSV), albeit other sources are known as well and may be used as taught herein. The TK gene may readily be obtained from HSV by methods well known to those of skill in the art. For example, the plasmid pMLBKTK in *E. coli* HB 101 (from ATCC #39369) is a source of the HSV-1 TK gene, which may be used as disclosed herein. (See, e.g, published International application No. WO 95/11984).

A therapeutic gene sequence may be introduced into a tumor mass by combining the adenoviral expression vector with a suitable pharmaceutically acceptable carrier. Introduction can be accomplished, for example, via direct injection of the recombinant Ad vector into the tumor mass. In the specific case of a cancer such as hepatocellular carcinoma (HCC), direct injection into the hepatic artery can be used for delivery, because most HCCs derive their circulation from this artery. Similar techniques of administration may be applied to other specific types of tumors and malignancies, as is known to those of skill in the art.

A method of tumor-specific delivery of a tumor-suppressor gene is accomplished by contacting target tissue in a subject with an effective amount of a recombinant Ad-derived vector of this invention. In the case of anti-tumor therapy, the gene is intended to encode an anti-tumor agent, such as a functional tumor suppressor gene product or suicide gene product. The term "contacting" is intended to encompass any delivery method for the efficient transfer of the vector, such as via intra-tumoral injection.

In another example, adenovirus vectors can be used to transfer genes to central nervous system (CNS) tumors *in vivo.* Using stereotactic delivery, Ad-derived vectors can transfer genes into the CNS intended for tumor therapy. For example, Badie, et al. (Neurosurgery 35(5): 910-916 (1994), incorporated by reference herein) reported that 50% and 90% transduction at vector titers of approximately 10⁷ and 10⁸ plaque-forming units/ml (pfu/ml) were observed in *in vitro* experiments. In their *in vivo* studies using appropriate animal brain tumor models, titers above 10⁷ were observed to have a cytopathic effect; more than 50% reduction in tumor cell growth was noted at 10⁸ pfu/ml; no toxic effects were noted when titers as high as 10¹⁰ pfu/ml were injected into the brain tissue of subject animals (Id.). Thus, the use of titers greater than 10⁷ pfu/ml appear appropriate when

The specification also describes methods for determining the efficacy of the within-disclosed therapeutic compositions and methods. One such method for confirming efficacy utilizes the human/SCID (severe combined immunodeficient) mouse model of EBV-induced LPD (lymphoproliferative disease) to ascertain whether EBV-antisense therapeutic nucleotide sequences block tumor formation. (See, e.g., Pisa, et al., Blood 79: 173-179 (1992); Rowe, et al., Curr. Top. Microbiol. Immunol. 166: 325 (1990); and Cannon, et al., J. Clin. Invest. 85: 1333-1337 (1990)by reference herein.)

Finally, the use of Ad vectors to prepare medicaments for the treatment, therapy and/or diagnosis of various diseases is also possible. Moreover, other anti-tumor genes may be used in combination with the corresponding therapeutic agent to reduce the proliferation of tumor cells. Such other gene-and-therapeutic-agent combinations are known to those of skill in the art and may be applied as taught herein.

### F. Construction of Therapeutic Viral Vectors for Gene Delivery

For *in vitro* gene transfer, administration is often accomplished by first isolating a selected cell population from a patient such as lung epithelial cells, lymphocytes and the like followed by *in vitro* gene transfer of the therapeutic compositions and the replacement of the cells into the patient. *In vivo* therapy is also possible, e.g., via the administration of therapeutic compositions by various delivery means. For example, aerosol administration and administration via subcutaneous, intravenous, intraperitoneal, intramuscular, ocular means and the like are possible.

Other gene-delivery methods are also useful in conjunction with methods, compositions and constructs described herein; see, e.g., published International Application No. WO 95/11984.

Similarly, various non-human animals having inserted therein the vectors or transformed cells of this invention. These "transgenic" animals are made using methods well known to those of skill in the art. For example, see U.S. Patent No. 5,175,384.

The specification discloses various methods of targeting specific cells -- e.g., cells in a subject in need of diagnosis and/or treatment. As discussed herein, the disclosure indicates that viral vectors and compositions may be directed to specific receptors or cells, for the ultimate purpose of delivering those vectors and compositions to specific cells or cell types. The viral vectors and constructs described herein are particularly useful in this regard.

In general, adenovirus attachment and uptake into cells are separate but cooperative events that result from the interaction of distinct viral coat proteins with a receptor for attachment and _{V} integrin receptors for internalization. Adenovirus attachment to the cell surface via the fiber coat proteins has been discovered to be dissociable and distinct from the subsequent step of internalization, and the present invention is able to take advantage of and function in conjunction with these differing receptors.

### G. Other Applications

The cell lines, viral vectors and methods disclosed herein may also be used for purposes other than the direct administration of therapeutic nucleotide sequences. In one such application, the production of large quantities of biologically active proteins or polypeptides in cells transfected with the within-disclosed viral vectors is contemplated herein. For example, human lymphoblastoid cells may be transfected with an integrative viral vector carrying a human hematopoietic growth factor such as the gene for erythropoietin (EPO); cells so transfected are thus able to produce biologically active EPO. (See, e.g., Lopez et al., Gene 148: 285-91 (1994).)

### EXAMPLES

The following examples are intended to illustrate the present invention. As such, the following description provides details of the manner in which particular embodiments of the present invention may be made and used. This description, while exemplary of the present invention, is not to be construed as specifically limiting the invention. Variations and equivalents, now known or later developed, which would be within the understanding and technical competence of one skilled in this art are to be considered as falling within the scope of this invention.

### Example 1

### Preparation of Adenovirus Packaging Cell Lines

Cell lines that are commonly used for growing adenovirus are useful as host cells for the preparation of adenovirus packaging cell lines. Preferred cells include 293 cells, an adenovirus-transformed human embryonic kidney cell line obtained from the ATCC, having Accession Number CRL 1573; HeLa, a human epithelial carcinoma cell line (ATCC Accession Number CCL 2); A549, a human lung carcinoma cell line (ATCC Accession Number CCL 1889); and the like epithelial-derived cell lines. As a result of the adenovirus transformation, the 293 cells contain the E1 early region regulatory gene. All cells were maintained in complete DMEM + 10% fetal calf serum unless otherwise noted.

The cell lines of this invention allow for the production and propagation of novel adenovirus-based gene delivery vectors having deletions in preselected gene regions by cellular complementation of adenoviral genes. To provide the desired complementation of such deleted adenoviral genomes in order to generate a novel viral vector , plasmid vectors that contain preselected functional units were designed as described herein. Such units include but are not limited to E1 early region, and the viral fiber gene. The preparation of plasmids providing such complementation, thereby being "complementary plasmids or constructs", that are stably inserted into host cell chromosomes are described below.

### A. Preparation of an E4-Expressing Plasmid for Complementation of E4-Gene-Deleted Adenoviruses

The viral E4 regulatory region contains a single transcription unit which is alternately spliced to produce several different mRNAs. The E4-expressing plasmid prepared as described herein and used to transfect the 293 cell line contains the entire E4 transcriptional unit. A DNA fragment extending from 175 nucleotides upstream of the E4 transcriptional start site including the natural E4 promoter to 153 nucleotides downstream of the E4 polyadenylation signal including the natural E4 terminator signal, corresponding to nucleotides 32667-35780 of the adenovirus type 5 (hereinafter referred to as Ad5) genome as described in Chroboczek et al. (Virol., 186:280-285 (1992), GenBank Accession Number M73260), was amplified from Ad5 genomic DNA, obtained from the ATCC, via the polymerase chain reaction (PCR). Sequences of the primers used were 5'CGG'TACACAGAATTCAGGAGACACAACTCC3' (forward or 5' primer referred to as E4L) (SEQ ID NO 1) and 5'GCCTGGATCCGGGAAGTTACGTAACGTGGGAAAAC3' (SEQ ID NO 2) (backward or 3' primer referred to as E4R). To facilitate cloning of the PCR fragment, these oligonucleotides were designed to create novel sites for the restriction enzymes EcoRI and BamHI, respectively, as indicated with underlined nucleotides. DNA was amplified via PCR using 30 cycles of 92 C for 1 minute, 50 C for 1 minute, and 72 C for 3 minutes resulting in amplified full-length E4 gene products.

The amplified DNA E4 products were then digested with EcoRI and BamHI for cloning into the compatible sites of pBluescript/SK+ by standard techniques to create the plasmid pHS/E4. A 2603 base pair (bp) cassette including the herpes simplex virus thymidine kinase promoter, the hygromycin resistance gene, and the thymidine kinase polyadenylation signal was excised from the plasmid pMEP4 (Invitrogen, San Diego, CA) by digestion with FspI followed by addition of BamHI linkers (5'CGCGGATCCGCG3') (SEQ ID NO 3) for subsequent digestion with BamHI to isolate the hygromycin-containing fragment.

The isolated BamHI-modified fragment was then cloned into the BamHI site of pBS/E4 containing the E4 region to create the plasmid pE4/Hygro containing 8710 bp. The pE4/Hygro plasmid has been deposited with the ATCC as described in Example 3. The complete nucleotide sequence of pE4/Hygro is listed in SEQ ID NO 4. Position number 1 of the linearized vector corresponds to approximately the middle portion of the pBS/SK+ backbone between the 3' BamHI site in the hygromycin insert and the 3' EcoRI site in the E4 insert. The 5' and 3' ends of the E4 gene are located at respective nucleotide positions 3820 and 707 of SEQ ID NO 4 while the 5' and 3' ends of the hygromycin insert are located at respective nucleotide positions 3830 and 6470. In the clone that was selected for use, the E4 and hygromycin resistance genes were divergently transcribed.

### B. Preparation of a Fiber-Expressing Plasmid for Complementation of Fiber-Gene-Deleted Adenoviruses

To prepare a fiber-encoding construct, primers were designed to amplify the fiber coding region from Ad5 genomic DNA with the addition of unique BamHI and NotI sites at the 5' and 3' ends of the fragment, respectively. The Ad5 nucleotide sequence is available with the GenBank Accession Number M18369. The 5' and 3' primers had the respective nucleotide sequences of 5'ATGGGATCCAAGATGAAGCGCGCAAGACCG3' (SEQ ID NO 5) and 5'CATAACGCGGCCGCTTCTTTATTCTTGGGC3' (SEQ ID NO 6), where the inserted BamHI and NotI sites are indicated by underlining. The 5' primer also contained a nucleotide substitution 3 nucleotides 5' of the second ATG codon (C to A) that is the initiation site. The nucleotide substitution was included so as to improve the consensus for initiation of fiber protein translation.

The amplified DNA fragment was inserted into the BamHI and NotI sites of pcDNA 3 (Invitrogen) to create the plasmid designated pCDNA3/Fiber having 7148 bp. The parent plasmid contained the CMV promoter, the bovine growth hormone (BHG) terminator and the gene for conferring neomycin resistance. The viral sequence included in this construct corresponds to nucleotides 31040-32791 of the Ad5 genome.

The complete nucleotide sequence of pCDNA3/Fiber is listed in SEQ ID NO 7 where the nucleotide position 1 corresponds to approximately the middle of the pcDNA 3 vector sequence. The 5' and 3' ends of the fiber gene are located at respective nucleotide positions 916 with ATG and 2661 with TAA.

To enhance expression of fiber protein by the constitutive CMV promoter provided by the pcDNA vector, a BglII fragment containing the tripartite leader (TPL) of adenovirus type 2 was excised from pRD112a (Sheay et al., BioTechniques, 15:856-862 (1993) and inserted into the BamHI site of pCDNA3/Fiber to create the plasmid pCLF having 7469 bp. The adenovirus tripartite leader sequence, present at the 5' end of all major late adenoviral mRNAs as described by Logan et al., Proc. Natl. Acad. Sci., USA, 81:3655-3659 (1984) and Berkner, BioTechniques, 6:616-629 (1988), is encoded by three spatially separated exons corresponding to nucleotide positions 6071-6079 (the 3' end of the first leader segment), 7101-7172 (the entire second leader segment), and 9634-9721 (the third leader segment) in the adenovirus type 2 genome. The tripartite sequence, however, also shows correspondence with the Ad5 leader sequence having three spatially separated exons corresponding to nucleotide positions 6081-6089 (the 3' end of the first leader segment), 7111-7182 (the entire second leader segment), and 9644-9845 (the third leader segment and sequence downstream of that segment). The corresponding cDNA sequence of the tripartite leader sequence present in pCLF is listed in SEQ ID NO 8 bordered by BamHI/BglII 5' and 3' sites at respective nucleotide positions 907-912 to 1228-1233.

The pCLF plasmid has been deposited with the ATCC as described in Example 3.. The complete nucleotide sequence of pCLF is listed in SEQ ID NO 8 where the nucleotide position 1 corresponds to approximately the middle of the pcDNA 3 parent vector sequence. The 5' and 3 ends of the Ad5 fiber gene are located at respective nucleotide positions 1237-1239 with ATG and 2980-2982 with TAA. The rest of the vector construct has been previously described above.

### C. Generation of an Adenovirus Packaging Cell Line Carrying Plasmids Encoding Functional E4 and Fiber Proteins

The 293 cell line was selected for preparing the first adenovirus packaging line as it already contains the E1 gene as prepared by Graham et al., J. Gen. Virol., 36:59-74 (1977) and as further characterized by Spector, Virol., 130:533-538 (1983). Before electroporation, 293 cells were grown in RPMI medium + 10% fetal calf serum. Four x 10⁶ cells were electroporated with 20 µg each of pE4/Hygro DNA and pCLF DNA using a BioRad GenePulser and settings of 300 V, 25 µF. DNA for electroporation was prepared using the Qiagen system according to the manufacturer's instructions (Bio-Rad, Richmond, CA).

Following electroporation, cells were split into fresh complete DMEM + 10% fetal calf serum containing 200 µg/ml Hygromycin B (Sigma, St. Louis, MO).

From expanded colonies, genomic DNA was isolated using the "MICROTURBOGEN" system (Invitrogen) according to manufacturer's instructions. The presence of integrated E4 DNA was assessed by PCR using the primer pair E4R and ORF6L (5'TGCTTAAGCGGCCGCGAAGGAGAAGTCC3') (SEQ ID NO 9), the latter of which is a 5' forward primer near adenovirus 5 open reading frame 6.

One clone, designated 211, was selected exhibiting altered growth properties relative to that seen in parent cell line 293. The 211 clone contained the expected product, indicating the presence of inserted DNA corresponding to most, if not all, of the E4 fragment contained in the pE4/Hygro plasmid. The 211 cell line has been deposited with the ATCC as described in Example 3 This line was further evaluated by amplification using the primer pair E4L/E4R described above, and a product corresponding to the full-length E4 insert was detected. Genomic Southern blotting was performed on DNA restricted with EcoRI and BamHI. The E4 fragment was then detected at approximately one copy/genome compared to standards with the EcoRI/BamHI E4 fragment as cloned into pBS/E4 for use as a labeled probe with the Genius system according to manufacturer's instructions (Boehringer Mannheim, Indianapolis, IN). In DNA from the 211 cell line, the expected labeled internal fragment pE4/Hygro hybridized with the isolated E4 sequences. In addition, the probe hybridized to a larger fragment which may be the result of a second insertion event.

Although the 211 cell line was not selected by neomycin resistance, thus indicating the absence of fiber gene, to confirm the lack of fiber gene, the 211 cell line was analyzed for expression of fiber protein by indirect immunofluorescence with an anti-fiber polyclonal antibody and a FTTC-labeled anti-rabbit IgG (KPL) as secondary. No immunoreactivity was detected. Therefore, to generate 211 clones containing recombinant fiber genes, the 211 clone was expanded by growing in RPMI medium and subjected to additional electroporation with the fiber-encoding pCLF plasmid as described above.

Following electroporation, cells were plated in DMEM + 10% fetal calf serum and colonies were selected with 200 µg/ml G418 (Gibco, Gaithersburg, MD). Positive cell lines remained hygromycin resistant. These candidate sublines of 211 were then screened for fiber protein expression by indirect immunofluorescence as described above. The three sublines screened, 211A, 211B and 211R, along with a number of other sublines, all exhibited nuclear staining qualitatively comparable to the positive control of 293 cells infected with AdRSV gal (1 pfu/cell) and stained 24 hours post-infection.

Lines positive for nuclear staining in this assay were then subjected to Western blot analysis under denaturing conditions using the same antibody. Several lines in which the antibody detected a protein of the expected molecular weight (62 kd for the Ad5 fiber protein) were selected for further study including 211A, 211B and 211R. The 211A cell line has been deposited with ATCC as described in Example 3.

Western blot analysis using soluble nuclear extracts from these three cell lines and a seminative electrophoresis system demonstrated that the fiber protein expressed is in the functional trimeric form characteristic of the native fiber protein. The predicted molecular weight of a trimerized fiber is 186 kd. The lane marked 293 lacks fiber while the sublines contain detectable fiber. Under denaturing conditions, the trimeric form was destroyed resulting in detectable fiber monomers. Those clones containing endogenous E1, newly expressed recombinant E4 and fiber proteins were selected for use in complementing adenovirus gene delivery vectors having the corresponding adenoviral genes deleted as described in Example 2.

### D. Preparation of an E1-Expressing Plasmid for Complementation of E1-Gene-Deleted Adenoviruses

In order to prepare adenoviral packaging cell lines other than those based on the E1-gene containing 293 cell line as described in Example 1C above, plasmid vectors containing E1 alone or in various combinations with E4 and fiber genes are constructed as described below.

The region of the adenovirus genome containing the E1a and E1b gene is amplified from viral genomic DNA by PCR as previously described. The primers used are E1L, the 5' or forward primer, and E1R, the 3' or backward primer, having the respective nucleotide sequences 5'CCGAGCTAGCGACTGAAAATGAG3' (SEQ ID NO 10) and 5'CCTCTCGAGAGACAGCAAGACAC3' (SEQ ID NO 11). The E1L and E1R primers include the respective restriction sites NheI and XhoI as indicated by the underlines. The sites are used to clone the amplified E1 gene fragment into the NheI/XhoI sites in pMAM commercially available from Clontech (Palo Alto, CA) to form the plasmid pDEX/E1 having 11152 bp.

The complete nucleotide sequence of pDEX/E1 is listed in SEQ ID NO 12 where the nucleotide position 1 corresponds to approximately 1454 nucleotides from the 3' end of the pMAM backbone vector sequence. The pDEX/E1 plasmid includes nucleotides 552 to 4090 of the adenovirus genome positioned downstream (beginning at nucleotide position 1460 and ending at 4998 in the pDEX/E1 plasmid) of the glucocorticoid-inducible mouse mammary tumor virus (MMTV) promoter of pMAM. The pMAM vector contains the E. coli *gpt* gene that allows stable transfectants to be isolated using hypoxanthine/aminopterin/thymidine (HAT) selection. The pMAM backbone occupies nucleotide positions 1-1454 and 5005-11152 of SEQ ID NO 12.

### E. Generation of an Adenovirus Packaging Cell Line Carrying Plasmids Encoding Functional E1, and Fiber Proteins

To create separate adenovirus packaging cell lines equivalent to that of the 211 sublines, 211A, 211B and 211R, as described in Example 1C, alternative cell lines lacking adenoviral genomes are selected for transfection with the plasmid constructs as described below. Acceptable host cells include A549, Hela, Vero and the like cell lines as described in Example 1. The selected cell line is transfected with the separate plasmids, pDEX/E1 and pCLF, respectively for expressing E1, and fiber complementary proteins. Following transfection procedures as previously described, clones containing stable insertions of the two plasmids are isolated by selection with neomycin and HAT. Integration of full-length copy of the E1 gene is assessed by PCR amplification from genomic DNA using the primer set E1L/E1R, as described above. Functional insertion of the fiber gene is assayed by staining with the anti-fiber antibody as previously described.

The resultant stably integrated cell line is then used as a packaging cell system to complement adenoviral gene delivery vectors having the corresponding adenoviral gene deletions as described in Example 2.

### F. Preparation of a Plasmid Containing Two or More Adenoviral Genes for Complementing Gene-Deleted Adenoviruses

The methods described in the preceding Examples rely on the use of two plasmids, pE4/Hygro and pCLF, or, pCLF and pDEX/E1 for generating adenoviral cell packaging systems. In alternative embodiments contemplated for use with the methods of this invention, complementing plasmids containing two or more adenoviral genes for expressing of encoded proteins in various combinations are also prepared as described below. The resultant plasmids are then used in various cell systems with delivery plasmids having the corresponding adenoviral gene deletions. The selection of packaging cell, content of the delivery plasmids and content of the complementing plasmids for use in generating recombinant adenovirus viral vectors thus depends on whether other adenoviral genes are deleted along with the adenoviral fiber gene, and, if so, which ones.

### 1. Preparation of a Complementing Plasmid Containing Fiber and E1 Adenoviral Genes

A DNA fragment containing sequences for the CMV promoter, adenovirus tripartite leader, fiber gene and bovine growth hormone terminator is amplified from pCLF prepared in Example 1B using the forward primer 5'GACGGATCGGGAGATCTCC3' (SEQ ID NO 13), that anneals to the nucleotides 1-19 of the pCDNA3 vector backbone in pCLF, and the backward primer 5'CCGCCTCAGAAGCCATAGAGCC3' (SEQ ID NO 14) that anneals to nucleotides 1278-1257 of the pCDNA3 vector backbone. The fragment is amplified as previously described and then cloned into the pDEX/E1 plasmid, prepared in Example 1D. For cloning in the DNA fragment, the pDEX/E1 vector is first digested with NdeI, that cuts at a unique site in the pMAM vector backbone in pDEX/E1, then the ends are repaired by treatment with bacteriophage T4 polymerase and dNTPs.

The resulting plasmid containing E1 and fiber genes, designated pE1/Fiber, provides both dexamethasone-inducible E1 function as described for DEX/E1 and expression of Ad5 fiber protein as described above. A schematic plasmid map of pE1/Fiber, having 14455 bp, is shown in Figure 8.

The complete nucleotide sequence of pE1/Fiber is listed in SEQ ID NO 15 where the nucleotide position 1 corresponds to approximately to 1459 nucleotides from the 3' end of the parent vector pMAM sequence. The 5' and 3 ends of the Ad5 E1 gene are located at respective nucleotide positions 1460 and 4998 followed by pMAM backbone and then separated from the Ad5 fiber from pCLF by the filled-in blunt ended NdeI site. The 5' and 3' ends of the pCLF fiber gene fragment are located at respective nucleotide positions 10922-14223 containing elements as previously described for pCLF.

The resultant pE1/Fiber plasmid is then used to complement one or more delivery plasmids expressing E1 and fiber.

The pE1/Fiber construct is then used to transfect a selected host cell as described in Example 1E to generate stable chromosomal insertions preformed as previously described followed by selection on HAT medium. The stable cells are then used as packaging cells as described in Example 2.

### 2) Preparation of a Complementing Plasmid Containing E4 and Fiber Adenoviral Genes

pCLF prepared as described in Example 1B is partially digested with BglII to cut only at the site in the pCDNA3 backbone. The pE4/Hygro plasmid prepared in Example 1A is digested with BamHI to produce a fragment containing E4. The E4 fragment is then inserted into the BamHI site of pCLF to form plasmid pE4/Fiber. The resultant plasmid provides expression of the fiber gene as described for pCLF and E4 function as described for pE4/Hygro.

The complete nucleotide sequence of pE4/Fiber is listed in SEQ ID NO 16 where the nucleotide position 1 corresponds to approximately 14 bp from the 3' end of the parent vector pCDNA3 backbone sequence. The 5' and 3 ends of the Ad5 E4 gene are located at respective nucleotide positions 21 and 3149 followed by fused BglII/BamHI sites and pCDNA3 backbone including the CMV promoter again followed by BglII/BamHI sites. The adenovirus leader sequence begins at nucleotide position 4051 and extends to 4366 followed by fused BamHI/BgUI sites and the 5' and 3' ends of the fiber gene located at respective nucleotide positions 4372 and 6124.

Stable chromosonal insertions of pE4/Fiber in host cells are obtained as described above.

### Example 2

### Preparation of Adenoviral Gene Delivery Vectors Using Adenoviral Packaging Cell Lines

Adenoviral delivery vectors are prepared to separately lack the combinations of E1/fiber and E4/fiber. Such vectors are more replication-defective than those previously in use due to the absence of multiple viral genes. A preferred adenoviral delivery vector that is replication competent but only via a non-fiber means is one that only lacks the fiber gene but contains the remaining functional adenoviral regulatory and structural genes. Furthermore, the adenovirus delivery vectors have a higher capacity for insertion of foreign DNA.

### A. Preparation of Adenoviral Gene Delivery Vectors Having Specific Gene Deletions and Methods of Use

To construct the E1/ /fiber deleted viral vector containing the LacZ reporter gene construct, two new plasmids were constructed. The plasmid pΔ E1Bβ gal was constructed as follows. By digestion of pSVβ gal (ProMega Corp., Madison, WI) with VspI, a DNA fragment containing the SV40 regulatory sequences and the *E. coli* -β-galactosidase gene was isolated. The resulting fragment having overhanging ends was then filled in with Klenow fragment of DNA polymerase 1 in the presence of dNTPs followed by digestion with BamHI. The resulting fragment was cloned into the EcoRV and BamHI sites in the polylinker of pΔ ElsplB (Microbix Biosystems, Hamilton, Ontario) to form pΔ E1B gal that therefore contained the left end of the adenovirus genome with the Ela region replaced by the LacZ cassette (nucleotides 6690 to 4151) of pSVβ gal. Plasmid DNA was prepared by the alkaline lysis method as described by Birnboim and Doly, Nuc. Acids Res., 7:1513-1523 (1978) from transformed cells used to expand the plasmid. DNA was then purified by CsCl-ethidium bromide density gradient centrifugation.

The second plasmid (pDV44), prepared as described herein, is derived from pBHG10, a vector prepared a described by Bett et al., Proc. Natl. Acad. Sci., USA, 91:8802-8806 (1994) and commercially available from Microbix, which contains an Ad5 genome with the packaging signals at the left end deleted and the E3 region (nucleotides 28133:30818) replaced by a linker with a unique site for the restriction enzyme PacI. An 11.3 kb BamHI fragment, which contains the right end of the adenovirus genome, is isolated from pBHG10 and cloned into the BamHI site of pBS/SK(+) to create plasmid p11.3 having approximately 14,658 bp. A schematic of the plasmid map is shown in Figure 13. The p11.3 plasmid was then digested with PacI and SalI to remove the fiber, E4, and inverted terminal repeat (ITR) sequences.

This fragment is replaced with a fragment containing the ITR segments and the E4 gene which is generated by PCR amplification from pBHG10 using the following oligonucleotide sequences(SEQ ID NO 17) (SEQ ID NO 18). These primers incorporate sites for PacI and BamHI, respectively. Cloning this fragment into the PacI and blunt ended SalI sites of the p11.3 backbone resulted in a substitution of the fused ITRs, E4 region and fiber gene present in pBHG10, by the ITRs and E4 region alone.

In general, the method for virus production by recombination of plasmids followed by complementation in cell culture involves the isolation of recombinant viruses by cotransfection of any one of the adenovirus packaging cell systems prepared in Example 1, namely 211A, 211B, 211R, A549, Vero cells, and the like, with plasmids carrying sequences corresponding to viral gene delivery vectors.

A selected cell line is plated in dishes and cotransfected with pDV44 and pE1B gal using the calcium phosphate method as described by Bett et al., Proc. Natl. Acad. Sci., USA, 91:8802-8806 (1994). Recombination between the overlapping adenovirus sequences in the two plasmids leads to the creation of a full-length viral chromosome where pDV44 and pΔ E1Bβ gal recombine to form a recombinant adenovirus vector having multiple deletions. The deletion of E1 and of the fiber gene from the viral chromosome is compensated for by the sequences integrated into the packaging cell genome, and infectious virus particles are produced. The plaques thus generated are isolated and stocks of the recombinant virus are produced by standard methods.

In a preferred embodiment of this invention, a delivery plasmid is prepared that does not require the above-described recombination events to prepare a therapeutic viral vector having a fiber gene deletion. A single delivery plasmid containing all the adenoviral genome necessary for packaging but lacking the fiber gene is prepared from plasmid pFG140 containing full-length Ad85 that is commercially available from Microbix. The resultant delivery plasmid referred to as pFG140-f is then used with pCLF stably integrated cells as described above to prepare a therapeutic viral vector lacking fiber. In a preferred aspect of this invention, the fiber gene is replace with a therapeutic gene of interest for preparing a therapeutic delivery adenoviral vector.

Vectors for the delivery of any desired therapeutic gene are prepared by cloning the gene of interest into the multiple cloning sites in the polylinker of commercially available p E1sp1B (Microbix Biosystems), in an analogous manner as performed for preparing p E1B gal as described above. The same cotransfection and recombination procedure is then followed as described herein to obtain viral gene delivery vectors.

The recombinant viruses thus produced are used as gene delivery tools both in cultured cells and in vivo. For studies of the effectiveness and relative immunogenicity of multiply-deleted vectors, virus particles are produced by growth in the packaging lines described in Example 1 and are purified by CsCl gradient centrifugation. Following titering, virus particles are administered to mice via systemic or local injection or by aerosol delivery to lung. The LacZ reporter gene allows the number and type of cells which are successfully transduced to be evaluated. The duration of transgene expression is evaluated in order to determine the long-term effectiveness of treatment with multiply-deleted recombinant adenoviruses relative to the standard technologies which have been used in clinical trials to date. The immune response to the improved vectors described here is determined by assessing parameters such as inflammation, production of cytotoxic T lymphocytes directed against the vector, and the nature and magnitude of the antibody response directed against viral proteins.

Versions of the vectors which contain therapeutic genes such as CFTR for treatment of cystic fibrosis or tumor suppressor genes for cancer treatment are evaluated in the animal system for safety and efficiency of gene transfer and expression. Following this evaluation, they are used as experimental therapeutic agents in human clinical trials.

### B. Retargeting of Adenoviral Gene Delivery Vectors by Producing Viral Particles Containing Different or Altered Fiber Proteins

As the specificity of adenovirus binding to target cells is largely determined by the fiber protein, viral particles that incorporate modified fiber proteins or fiber proteins from different adenoviral serotypes (pseudotyped vectors) have different specificities. Thus, the expression of the native Ad5 fiber protein in adenovirus packaging cells as described above is also applicable to production of different fiber proteins.

In one aspect of invention, chimeric fiber proteins are produced according to the methods of Stevenson et al., J. Virol., 69:2850-2857 (1995). The authors showed that the determinants for fiber receptor binding activity are located in the head domain of the fiber and that isolated head domain is capable of trimerization and binding to cellular receptors. The head domains of adenovirus type 3 (Ad3) and Ad5 were exchanged in order to produce chimeric fiber proteins. Similar constructs for encoding chimeric fiber proteins for use in the methods of this invention are contemplated. Thus, instead of the using the intact Ad5 fiber-encoding construct prepared in Example 1 as a complementing viral vector in adenoviral packaging cells, the constructs described herein are used to transfect cells along with E4 and/or E1-encoding constructs.

Briefly, full-length Ad5 and Ad3 were amplified from purified adenovirus genomic DNA as a template. The Ad5 and Ad3 nucleotides sequences are available with the respective GenBank Accession Numbers M18369 and M12411. Oligonucleotide primers are designed to amplify the entire coding sequence of the full-length fiber genes, starting from the start codon, ATG, and ending with the termination codon TAA. For cloning purposes, the 5' and 3' primers contain the respective restriction sites BamHI and NotI for cloning into pcDNA plasmid as described in Example 1A. PCR is performed as described above.

The resultant products are then used to construct chimeric fiber constructs by PCR gene overlap extension, as described by Horton et al., BioTechniques, 8:525-535 (1990). The Ad5 fiber tail and shaft regions (5TS; the nucleotide region encoding amino acid residue positions 1 to 403) are connected to the Ad3 fiber head region (3H; the nucleotide region encoding amino acid residue positions 136 to 319) to form the 5TS3H fiber chimera. Conversely, the Ad3 fiber tail and shaft regions (3TS; the nucleotide region encoding amino acid residues positions 1 to 135) are connected to the Ad5 fiber head region (5H; the nucleotide region encoding the amino acid residue positions 404 to 581) to form the 3TS5H fiber chimera. The fusions are made at the conserved TLWT (SEQ ID NO 21) sequence at the fiber shaft-head junction.

The resultant chimeric fiber PCR products are then digested with BamHI and NotI for separate directional ligation into a similarly digested pcDNA vector. The Ad2 leader sequence is then subcloned into the BamHI as described in Example I A for preparing an expression vector for subsequent transfection into 211 cells as described above or into the alternative packaging cell systems as previously described. The resultant chimeric fiber construct-containing adenoviral packaging cell lines are then used to complement adenoviral delivery vectors as previously described. Other fiber chimeric constructs are obtained using a similar approach with the various adenovirus serotypes known.

In an alternative embodiment, the methods of this invention contemplate the use of the modified proteins including novel epitopes as described by Michael et al., Gene Therapy, 2:660-668 (1995) and in International Publication WO 95/26412, the disclosures of which are incorporated by reference herein. Both publications describe the construction of a cell-type specific therapeutic viral vector having a new binding specificity incorporated into the virus concurrent with the destruction of the endogenous viral binding specificity. In particular, the authors described the production of an adenoviral vector encoding a gastrin releasing peptide (GRP) at the 3' end of the coding sequence of the Ad5 fiber gene. The resulting fiber-GRP fusion protein was expressed and shown to assemble functional fiber trimers that were correctly transported to the nucleus of HeLa cells following synthesis.

Based on the teachings in the paper and International Publication, similar constructs are contemplated for use in the complementing adenoviral packaging cell systems of this invention for generating new adenoviral gene delivery vectors that are replication-deficient and less immunogenic. Heterologous ligands contemplated for use herein to redirect fiber specificity range from as few as 10 amino acids in size to large globular structures, some of which necessitate the addition of a spacer region so as to reduce or preclude steric hindrance of the heterologous ligand with the fiber or prevent trimerization of the fiber protein. The ligands are inserted at the end or within the linker region. Preferred ligands include those that target specific cell receptors or those that are used for coupling to other moieties such as biotin and avidin. The types of cell signaling as a result of binding by a ligand is dependent upon the specificity of that ligand; i.e, receptor internalization or lack thereof.

A preferred spacer includes a short 12 amino acid peptide linker composed of a series of serines and alanine flanked by a proline residue at each end. One of ordinary skill in the art is familiar with the preparation of linkers to accomplish sufficient protein presentation and for altering the binding specificity of the fiber protein without compromising the cellular events that follow viral internalization. Moreover, within the context of this invention, preparation of modified fibers having ligands positioned internally within the fiber protein, at the amino terminus and at the carboxy terminus as described below are contemplated for use with the methods described herein.

The preparation of a fiber having a heterologous binding ligand is prepared essentially as described in the above-cited paper. Briefly, for the ligand of choice, site-directed mutagenesis is used to insert the coding sequence for a linker into the NotI site at the 3' end of the Ad5 fiber construct in pCLF as prepared in Example 1. The 3' or antisense oligonucleotide encodes a preferred linker sequence of ProSerAlaSerAlaSerAlaSerAlaProGlySer (SEQ ID NO 22) followed by a unique restriction site and two stop codons, respectively, to allow the insertion of a coding sequence for a selected heterologous ligand and to ensure proper translation termination. The 3' end of the antisense oligonucleotide includes sequences that overlap with vector sequence into which the oligonucleotide is inserted via site-directed mutagenesis. Following mutagenesis of the pCLF sequence adding the linker and stop codon sequences, a nucleotide sequence encoding a preselected ligand is obtained, linkers corresponding to the unique restriction site are attached and then the sequence is cloned into linearized corresponding restriction site.

Into the resultant pCLF vector containing a Ad5 fiber gene sequence with 3' nucleotides encoding a linker and a ligand, the Ad2 leader sequence is inserted as previously described. The resultant fiber-ligand construct is then used to transfect 211 or the alternative cell packaging systems previously described to produce complementing viral vector packaging systems for use with the methods of this invention.

In a further embodiment, fiber proteins encoded by fiber genes isolated from different adenoviral serotypes are used intact for transfection into 211 or an alternative cell packaging system as previously described.

A gene encoding the fiber protein of interest is first cloned to create a plasmid analogous to pCLF, and stable cell lines producing the fiber protein are generated as described above for Ad5 fiber. The adenovirus vector described which lacks the fiber gene is then propagated in the cell line producing the fiber protein relevant for the purpose at hand. As the only fiber gene present is the one in the packaging cells, the adenoviruses produced contain only the fiber protein of interest and therefore have the binding specificity conferred by the complementing protein. Such viral particles are used in studies such as those described above to determine their properties in experimental animal systems.

### C. Targeted Gene Delivery Using Viral Vector Particles Lacking Fiber Protein

An alternative mode of entry for adenoviral infection of hematopoietic cells has been described by Huang, et al., J. Virol., 69:2257-2263 (1995) which does not involve the fiber protein-host cell receptor interaction. As infection of most other cell types does require the presence of fiber protein, vector particles which lack fiber may preferentially infect hematopoietic cells, such as monocytes or macrophages.

To produce a fiber-free adenovirus vector particle, a vector lacking the fiber gene as described above in Example 2A but containing a gene of interest for delivery is amplified by growth in cells which do not produce a fiber protein, such as the 211 cells prepared in Example 1, thereby producing large numbers of particles lacking fiber protein. The recovered fiber-free viral particles are then used to deliver the inserted gene of interest following the methods of this invention via targeting mechanisms provided by other regions of the adenoviral vector, i.e., via the native penton base.

### Example 3

### Deposit of Materials

The following cell lines and plasmids have been deposited on September 25, 1996, with the American Type Culture Collection, 1301 Parklawn Drive, Rockville, MD, USA (ATCC):

| Material | ATCC Accession No. |
|---|---|
| Plasmid pE4/Hygro | 97739 |
| Plasmid pCLF | 97737 |
| 211 Cell Line | CRL-12193 |
| 211A Cell Line | CRL-12194 |

The foregoing written specification is considered to be sufficient to enable one skilled in the art to practice the invention. The present invention is not to be limited in scope by the cell lines deposited, since the deposited embodiment is intended as a single illustration of one aspect of the invention and any cell lines that are functionally equivalent are within the scope of this invention.

### Example 4

The native fiber protein is a homotrimer. { Henry L.J. et al 1994 Characterization of the knob domain of the adenivirus Type 5 fiber protein expressed in Escherichia coli J. Virol 68:5239-5246 }, and trimerization is essential for assembly of the penton/fiber complex {Novelli A et al 1991 Assembly of adenovirus type 2 fiber synthesized in cell-free translation system. J. Biol. Chem 266:9299-9303 }. To assess the multimeric structure of the recombinant fiber protein produced by the cell lines, cells were labeled with 50 µCi/ml [³⁵S] Translabel (ICN) for two hours at 37 ° C, lysed in RIPA buffer, and fiber protein was immunoprecipitated as described { Harlow E et al 1988 Antibodies. Cold Spring Harbour Laboratory, cold Spring Harbour}. Immune complexes were collected on Protein A-Sepharose beads (Pierce), extensively washed with RIPA buffer, and incubated at room temperature in 0.1 M triethylamine, pH 11.5 to release bound fiber protein. A portion of the precipitated fiber was electrophoresed on a 8% SDS-PAGE gel under denaturing (1% SDS in loading buffer, samples boiled for 5 minutes) or semi-native (0.1% SDS in loading buffer, samples not heated) conditions.

Lines 211A, 211B, and 211R, but not the control 293 cells, expressed an immunologically reactive protein which migrated at the predicted molecular weight for trimer (186 kD) under seminative conditions and for monomer (62 kD) under denaturing conditions. The behavior of the precipitated fiber was indistinguishable from that of purified baculovirus-produced recombinant Ad2 fiber { Wickham T et al 1993 Cell 73:309-319} (the 58 kD Ad2 and 62 kD Ad5 fibers have very similar mobilities under these conditions).

To determine whether the fiber-expressing lines could support the growth of a fiber-defective adenovirus, we performed one-step growth experiments using the temperature-sensitive fiber mutant Ad H5*ts*142 (the gift of Harold Ginsberg). At the restrictive temperature (39.5 °C), this mutant produces an underglycoslyated fiber protein which is not incorporated into mature virions { Chee-Sheung C. C et al 1982 J. Virol 42: 932-950 }. This results in the accumulation of non-infectious viral particles. We asked whether the recombinant fiber protein expressed by our cell lines could complement the H5*ts*142 defect and rescue viral growth.

Cell lines 293, 211A,211B and 211R (2 x 10⁶ cells/sample) were infected with H5*ts* 142 at 10 pfu/cell. 48 hours later, cells were detached with 25 mM EDTA and virus was harvested by four rapid freeze-thaw cycles. Debris was removed by a 10 minute spin at 1500 x g, and viral titers determined by fluorescent focus assay { Thiel J.F et al 1967 Proc. Soc. Exp. Biol. Med. 125:892-895 } on SW480 cells with a polyclonal anti-penton base Ab { Wickham T et al 1993 Cell 73:309-319}. The fiber mutant virus replicated to high titers in 293 cells at 32.5° C (the permissive temperature), but to a much lower extent at the restrictive temperature of 39.5° C. The fiber-producing packaging lines 211A, 211B, or 211R supported virus production at 39.5° C to levels within two- to three-fold of those seen at the permissive temperature in 293 cells, indicating that these cells provided partial complementation of the fiber defect.

Interestingly, virus yields from the fiber-producing cell lines were also somewhat higher than those from 293 cells at 32.5° C (the 'permissive' temperature). This suggests that fiber produced by the ts142 virus may be partially defective even at the permissive temperature. Alternatively, a nonspecific increase in adenoviral titer could result when viruses are grown in our packaging cells, by a mechanism not involving fiber complementation. However, we have found that viruses with wild type fiber genes (such as Ad.RSVbgal) replicate to identical levels either in our packaging lines or in 293 cells (data not shown). Taken together, these results demonstrate that the observed increase in H5*ts*142 growth is due to specific complementation of the fiber mutation.

Even in the fiber-expressing cell lines, the fiber mutant grows to higher titers at 32 °C than at 39.5 °C. This incomplete complementation may be due to the packaging lines' expression of fiber at a level somewhat below that seen in a wild-type infection. A recent study reported an E4-deleted vector which coincidentally reduced fiber protein expression, resulting in a large reduction in the titer of virus produced { Brough et al 1996 J. Virol. 70: 6497-6501} . Another possibility is that the defective *ts*142 fiber protein produced at the restrictive temperature might form complexes with some of the wild type protein produced by the cells and prevent its assembly into particles.

Although the fiber proteins of different Ad serotypes differ in the length of their shaft domains and in their receptor-binding knob domains, the N-terminal regions responsible for interaction with the viral penton base are highly conserved { Arnberg N. et al 1997 Virology 227:239-244 }. This suggests that fibers from many viral serotypes, with their different cell-binding specificities, may be amenable for use in producing gene delivery vectors.

We asked whether the recombinant Ad5 fiber produced by our packaging cells could be incorporated into particles of another adenovirus serotype. Adenovirus type 3 was grown either in fiber-producing cell lines or in 293 cells. Viral particles were purified by two sequential centrifugations (3 h at 111,000 x g) on preformed 15-40% CsCl gradients to remove soluble cellular proteins and then dialyzed extensively against 10 mM Tris-HCl, pH 8.1, 150 mM NaCl, 10% glycerol. Ad5 fiber protein was detected by immunoblotting using the polyclonal anti-fiber serum, followed by detection with a horseradish peroxidase-conjugated goat anti-rabbit antibody (Kirkegaard and Perry Laboratories) and the ECL chemiluminescence substrate (Amersham). The purified Ad3 particles contained Ad5 fiber protein after a single passage through a fiber-expressing cell line but not after passage through 293 cells. Previous work has demonstrated that Ad2 fiber is capable of interacting *in vitro* with Ad3 penton base {Fender et al 1997 Nature Biotech. 15:52-56 }, and our result demonstrates that the type 5 fiber protein produced by the cells is capable of assembling into complete Ad3 particles.

A vector based on Ad5 but containing the gene for the Ad7 fiber protein has been described { Gall J. et al 1996 J. Virol. 70:2116-2123}, as well as Ads containing chimeric fiber genes (Krasnykh V. N et al J. Virol. 70:6839-6846). Addition of a short peptide linker to the fiber in order to confer binding to a different cellular protein has also been reported. By using packaging technology such as that presented here, Ad vectors equipped with different fiber proteins may be produced simply by growth in cells expressing the fiber of interest, without the time-consuming step of generating a new vector genome for each application.

Replacing or modifying the fiber gene in the vector chromosome would also require that the new fiber protein bind a receptor on the surface of the cells it which it is to be grown. The packaging cell approach will allow the generation of Ad particles containing a fiber which can no longer bind to its host cells, by a single round of growth in cells expressing the desired fiber gene. This will greatly expand the repertoire of fiber proteins which can be incorporated into particles, as well as simplifying the process of retargeting gene delivery vectors.

Finally, a novel fiber-independent pathway of infection has recently been described in hematopoietic cells, in which penton base provides the initial virus-cell interaction by binding to integrin αₘβ₂ {Huang S. et al 1996 J, Virol 70: 4502-4508}. This suggests that viral particles lacking fiber protein may be useful in targeting gene delivery to specific cell types via this pathway.

## Claims

1. An adenovirus packaging cell line that expresses an adenovirus fiber protein, comprising a stably integrated nucleic acid sequence that includes a nucleotide sequence encoding an adenovirus fiber protein, operatively-linked to a first heterologous promoter, wherein said first heterologous promoter comprises a sequence of nucleotides encoding an introduced adenovirus (Ad)2 or Ad5 tripartite leader (TPL), and a second stably integrated heterologous promoter operatively-linked to a nucleic acid encoding an adenoviral E4 gene, wherein said packaging cell line supports virus production at 39.5 C to levels two to three fold greater than virus production by 293 cells at the permissive temperature of 32.5 C.

2. The packaging cell line according to claim 1 wherein said nucleotide sequence encoding said adenovirus protein E4 gene is encoded by nucleotides 32667 to 35780 of the Ad5 genome.

3. The packaging cell line according to claim 1 wherein the nucleotide sequence encoding said adenovirus fiber protein comprises nucleotides 31040 to 32791 of the Ad5 genome.

4. The packaging cell line according to claim 2 or 3, further comprising a viral vector, wherein said viral vector produced in that cell line comprises a nucleic acid sequence having a deletion or mutation of a DNA sequence encoding one or more of the following regulatory proteins or polypeptides: E2A, E2B or E3, wherein the deletion or mutation disrupts expression of early region polypeptides, and wherein said packaging cell line complements the function of the deleted or mutated regulatory proteins or polypeptides.

5. The packaging cell line according to claim 4, wherein a foreign DNA sequence encoding one or more foreign proteins, polypeptides or fragments thereof has been inserted in place of any of said deletions.

6. The packaging cell line according to claim 5, wherein said foreign DNA encodes a tumor-suppressor protein or a biologically active fragment thereof.

7. The packaging cell line according to claim 5, wherein said foreign DNA encodes a suicide protein or a biologically active fragment thereof.

8. The packaging cell line according to any one of claims 2 to 7, wherein said cell line is an epithelial cell line.

9. The packaging cell line according to any one of claims 2 to 7, wherein said cell line is selected from the group consisting of 293, A549, W162, HeLa, Vero, 211, and 211A, 211B and 211R cell lines.

10. The packaging cell line according to any one of claims 1 to 9, wherein said cell line has been modified to include the nucleotide sequence of the pCLF plasmid, presented as SEQ ID NO: 8.

11. The packaging cell line according to any one of claims 1 to 10, wherein the second heterologous promoter is an HSV tk promoter or a CMV promoter.

12. The packaging cell line according to any one of claims 1 to 11, wherein said fiber comprises amino acid residue sequences from more than one adenovirus serotype.

## Patentansprüche

1. Adenovirus-Verpackungszellinie, die ein Adenovirus-Fiberprotein exprimiert, umfassend eine stabil integrierte Nukleinsäuresequenz, die ein Nukleotidsequenz einschließt, welche ein Adenovirus-Fiberprotein codiert, operativ verbunden mit einem ersten heterologen Promotor, wobei dieser erste heterologe Promotor eine Sequenz aus Nukleotiden umfaßt, die einen eingeschleusten Adenovirus (Ad)2- oder Ad5-Tripartit-Leader (TPL) codiert, und einen zweiten stabil integrierten heterologen Promotor, operativ verbunden mit einer Nukleinsäure, die ein adenovirales E4-Gen codiert, wobei diese Verpackungszellinie die Virusproduktion bei 39,5°C auf Niveaus unterstützt, die zwei- oder dreifach höher sind als die Virusproduktion durch 293 Zellen bei der permissiven Temperatur von ' 32,5°C.

2. Verpackungszellinie gemäß Anspruch 1, wobei die Nukleotidsequenz, die das Adenovirus-Protein E4-Gen codiert, durch die Nukleotide 326667 bis 35780 des Ad5-Genoms codiert wird.

3. Verpackungszellinie gemäß Anspruch 1, wobei die Nukleotidsequenz, die das Adenovirus-Fiberprotein codiert, die Nukleotide 31040 bis 32791 des Ad5-Genoms umfaßt.

4. Verpackungszellinie gemäß Anspruch 2 oder 3, weiter umfassend einen viralen Vektor, wobei der virale Vektor, der in dieser Zellinie produziert wird, eine Nukleinsäuresequenz umfaßt, die eine Deletion oder Mutation einer DNA-Sequenz hat, die ein oder mehrere der folgenden regulatorischen Proteine oder Polypeptide codiert: E2A, E2B oder E3, wobei die Deletion oder Mutation die Expression der Polypeptide des frühen Bereichs stört, wobei diese Verpackungszellinie die Funktion der deletierten oder mutierten regulatorischen Proteine oder Polypeptide komplementiert.

5. Verpackungszellinie gemäß Anspruch 4, wobei eine fremde DNA-Sequenz, die ein oder mehrere fremde Proteine, Polypeptide oder Fragmente davon codiert, anstelle irgendeiner dieser Deletionen inseriert worden ist.

6. Verpackungszellinie gemäß Anspruch 5, wobei diese fremde DNA ein Tumorsuppressorprotein oder ein biologisch aktives Fragment davon codiert.

7. Verpackungszellinie gemäß Anspruch 5, wobei diese fremde DNA ein Selbstmordprotein oder ein biologisch aktives Fragment davon codiert.

8. Verpackungszellinie gemäß irgendeinem der Ansprüche 2 bis 7, wobei diese Zellinie eine epitheliale Zellinie ist.

9. Verpackungszellinie gemäß irgendeinem der Ansprüche 2 bis 7, wobei diese Zellinie ausgewählt ist aus der Gruppe bestehend aus den Zellinien 293, A549, W162, HeLa, Vero, 211 und 211A, 211B und 211R.

10. Verpackungszellinie gemäß irgendeinem der Ansprüche 1 bis 9, wobei diese Zellinie modifiziert worden ist, um die Nukleotidsequenz des pCLF-Plasmids einzuschließen, welche in SEQ ID Nr. 8 dargestellt ist.

11. Verpackungszellinie gemäß irgendeinem der Ansprüche 1 bis 10, wobei dieser zweite heterologe Promotor ein HSV tk-Promotor oder ein CMV-Promotor ist.

12. Verpackungszellinie gemäß irgendeinem der Ansprüche 1 bis 11, wobei diese Fiber Aminosäurerestesequenzen von mehr als einem Adenovirus-Serotyp umfaßt.

## Revendications

1. Lignée cellulaire d'encapsidation d'un adénovirus qui exprime une protéine fibreuse d'adénovirus, comprenant une séquence d'acide nucléique intégrée de façon stable qui inclut une séquence nucléotidique codant pour une protéine fibreuse d'adénovirus, liée de façon opérationnelle à un premier promoteur hétérologue, dans laquelle ledit premier promoteur hétérologue comprend une séquence de nucléotides codant pour une séquence de tête tripartite (TPL) (Ad)2 ou Ad5 d'adénovirus introduite, et un second promoteur hétérologue intégré de façon stable lié de façon opérationnelle à un acide nucléique codant pour un gène E4 d'adénovirus, dans laquelle ladite lignée cellulaire d'encapsidation supporte la production virale à 39,5°C à des niveaux deux à trois fois plus élevés que la production virale par les cellules 293 à la température permissive de 32,5°C.

2. Lignée cellulaire d'encapsidation selon la revendication 1, dans laquelle ladite séquence nucléotidique codant pour ledit gène EA de protéine d'adénovirus est codée par les nucléotides 32667 à 35780 du génome Ad5.

3. Lignée cellulaire d'encapsidation selon la revendication 1, dans laquelle la séquence nucléotidique codant pour la protéine fibreuse d'adénovirus comprend les nucléotides 31040 à 32791 du génome Ad5.

4. Lignée cellulaire d'encapsidation selon la revendication 2 ou 3, comprenant en outre un vecteur viral, dans laquelle ledit vecteur viral produit dans cette lignée cellulaire comprend une séquence d'acide nucléique ayant une délétion ou une mutation d'une séquence d'ADN codant pour une ou plusieurs des protéines ou polypeptides de régulation suivants : E2A, E2B ou E3, dans laquelle la délétion ou la mutation interrompt l'expression des polypeptides de la région précoce, et dans laquelle ladite lignée cellulaire d'encapsidation complémente la fonction des protéines ou polypeptides de régulation délétés ou mutés.

5. Lignée cellulaire d'encapsidation selon la revendication 4, dans laquelle une séquence d'ADN étranger codant pour un ou plusieurs protéines, polypeptides ou fragments de ceux-ci ont été insérés à la place d'une quelconque desdites délétions.

6. Lignée cellulaire d'encapsidation selon la revendication 5, dans laquelle ledit ADN étranger code pour une protéine de suppression des tumeurs ou un fragment biologiquement actif de celle-ci.

7. Lignée cellulaire d'encapsidation selon la revendication 5, dans laquelle ledit ADN étranger code pour une protéine suicide ou un fragment biologiquement actif de celle-ci.

8. Lignée cellulaire d'encapsidation selon l'une quelconque des revendications 2 à 7, dans laquelle ladite lignée cellulaire est une lignée cellulaire épithéliale.

9. Lignée cellulaire d'encapsidation selon l'une quelconque des revendications 2 à 7, dans laquelle ladite lignée cellulaire est choisie dans le groupe constitué des lignées cellulaires 293, A549, W162, HeLa, Vero, 211 et 221A, 211B et 211R.

10. Lignée cellulaire d'encapsidation selon l'une quelconque des revendications 1 à 9, dans laquelle ladite lignée cellulaire a été modifiée pour inclure la séquence nucléotidique du plasmide pCLF, présenté comme SEQ ID N°8.

11. Lignée cellulaire d'encapsidation selon l'une quelconque des revendications 1 à 10, dans laquelle le second promoteur hétérologue est un promoteur tk de HSV ou un promoteur de CMV.

12. Lignée cellulaire d'encapsidation selon l'une quelconque des revendications 1 à 11, dans laquelle ladite fibre comprend les séquences des résidus d'acides aminés de plus d'un sérotype d'adénovirus.


### SEQUENCE LISTING

(1) GENERAL INFORMATION:
   (i) APPLICANT: Nemerow, Glen R.
      Von Seggern, Daniel J.
   (ii) TITLE OF INVENTION: PACKAGING CELL LINES, ADENOVIRUS VECTORS, AND METHODS OF USING SAME
   (iii) NUMBER OF SEQUENCES: 20
   (iv) CORRESPONDENCE ADDRESS:
      (A) ADDRESSEE: THE SCRIPPS RESEARCH INSTITUTE
      (B) STREET: 10550 North Torrey Pines Road
      (C) CITY: La Jolla
      (D) STATE: California
      (E) COUNTRY: United States
      (F) ZIP: 92037
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US
      (B) FILING DATE: 25-SEP-1996
      (C) CLASSIFICATION:
   (viii) ATTORNEY/AGENT INFORMATION:
      (A) NAME: Logan, April C.
      (B) REGISTRATION NUMBER: 33,950
      (C) REFERENCE/DOCKET NUMBER: TSRI 554.0
   (ix) TELECOMMUNICATION INFORMATION:
      (A) TELEPHONE: (619) 554-2937
      (B) TELEFAX: (619) 554-6312
(2) INFORMATION FOR SEQ ID NO:1:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:1:
   CGGTACACAG AATTCAGGAG ACACAACTCC
   30
(2) INFORMATION FOR SEQ ID NO:2:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:2:
   GCCTGGATCC GGGAAGTTAC GTAACGTGGG AAAAC
   35
(2) INFORMATION FOR SEQ ID NO:3:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:3:
   CGCGGATCCG CG
   12
(2) INFORMATION FOR SEQ ID NO:4 :
   (i) SEQUENCE CHARACTERISTICS:
      (A) LENGTH: 8710 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:4:
   CACCTAAATT GTAAGCGTTA ATATTTTGTT AAAATTCGCG TTAAATTTTT GTTAAATCAG 60
   CTCATTTTTT AACCAATAGG CCGAAATCGG CAAAATCCCT TATAAATCAA AAGAATAGAC 120
   CGAGATAGGG TTGAGTGTTG TTCCAGTTTG GAACAAGAGT CCACTATTAA AGAACGTGGA 180
   CTCCAACGTC AAAGGGCGAA AAACCGTCTA TCAGGGCGAT GGCCCACTAC GTGAACCATC 240
   ACCCTAATCA AGTTTTTTGG GGTCGAGGTG CCGTAAAGCA CTAAATCGGA ACCCTAAAGG 300
   GAGCCCCCGA TTTAGAGCTT GACGGGGAAA GCCGGCGAAC GTGGCGAGAA AGGAAGGGAA 360
   GAAAGCGAAA GGAGCGGGCG CTAGGGCGCT GGCAAGTGTA GCGGTCACGC TGCGCGTAAC 420
   CACCACACCC GCCGCGCTTA ATGCGCCGCT ACAGGGCGCG TCCCATTCGC CATTCAGGCT 480
   GCGCAACTGT TGGGAAGGGC GATCGGTGCG GGCCTCTTCG CTATTACGCC AGCTGGCGAA 540
   AGGGGGATGT GCTGCAAGGC GATTAAGTTG GGTAACGCCA GGGTTTTCCC AGTCACGACG
   600
   TTGTAAAACG ACGGCCAGTG AATTGTAATA CGACTCACTA TAGGGCGAAT TGGGTACCGG
   660
   GCCCCCCCTC GAGGTCGACG GTATCGATAA GCTTGATATC GAATTCAGGA GACACAACTC
   720
   CAAGTGCATA CTCTATGTCA TTTTCATGGG ACTGGTCTGG CCACAACTAC ATTAATGAAA
   780
   TATTTGCCAC ATCCTCTTAC ACTTTTTCAT ACATTGCCCA AGAATAAAGA ATCGTTTGTG
   840
   TTATGTTTCA ACGTGTTTAT TTTTCAATTG CAGAAAATTT CAAGTCATTT TTCATTCAGT
   900
   AGTATAGCCC CACCACCACA TAGCTTATAC AGATCACCGT ACCTTAATCA AACTCACAGA
   960
   ACCCTAGTAT TCAACCTGCC ACCTCCCTCC CAACACACAG AGTACACAGT CCTTTCTCCC
   1020
   CGGCTGGCCT TAAAAAGCAT CATATCATGG GTAACAGACA TATTCTTAGG TGTTATATTC
   1080
   CACACGGTTT CCTGTCGAGC CAAACGCTCA TCAGTGATAT TAATAAACTC CCCGGGCAGC
   1140
   TCACTTAAGT TCATGTCGCT GTCCAGCTGC TGAGCCACAG GCTGCTGTCC AACTTGCGGT
   1200
   TGCTTAACGG GCGGCGAAGG AGAAGTCCAC GCCTACATGG GGGTAGAGTC ATAATCGTGC
   1260
   ATCAGGATAG GGCGGTGGTG CTGCAGCAGC GCGCGAATAA ACTGCTGCCG CCGCCGCTCC
   1320
   GTCCTGCAGG AATACAACAT GGCAGTGGTC TCCTCAGCGA TGATTCGCAC CGCCCGCAGC
   1380
   ATAAGGCGCC TTGTCCTCCG GGCACAGCAG CGCACCCTGA TCTCACTTAA ATCAGCACAG
   1440
   TAACTGCAGC ACAGCACCAC AATATTGTTC AAAATCCCAC AGTGCAAGGC GCTGTATCCA
   1500
   AAGCTCATGG CGGGGACCAC AGAACCCACG TGGCCATCAT ACCACAAGCG CAGGTAGATT
   1560
   AAGTGGCGAC CCCTCATAAA CACGCTGGAC ATAAACATTA CCTCTTTTGG CATGTTGTAA
   1620
   TTCACCACCT CCCGGTACCA TATAAACCTC TGATTAAACA TGGCGCCATC CACCACCATC
   1680
   CTAAACCAGC TGGCCAAAAC CTGCCCGCCG GCTATACACT GCAGGGAACC GGGACTGGAA
   1740
   CAATGACAGT GGAGAGCCCA GGACTCGTAA CCATGGATCA TCATGCTCGT CATGATATCA
   1800
   ATGTTGGCAC AACACAGGCA CACGTGCATA CACTTCCTCA GGATTACAAG CTCCTCCCGC
   1860
   GTTAGAACCA TATCCCAGGG AACAACCCAT TCCTGAATCA GCGTAAATCC CACACTGCAG
   1920
   GGAAGACCTC GCACGTAACT CACGTTGTGC ATTGTCAAAG TGTTACATTC GGGCAGCAGC
   1980
   GGATGATCCT CCAGTATGGT AGCGCGGGTT TCTGTCTCAA AAGGAGGTAG ACGATCCCTA
   2040
   CTGTACGGAG TGCGCCGAGA CAACCGAGAT CGTGTTGGTC GTAGTGTCAT GCCAAATGGA
   2100
   ACGCCGGACG TAGTCATATT TCCTGAAGCA AAACCAGGTG CGGGCGTGAC AAACAGATCT
   2160
   GCGTCTCCGG TCTCGCCGCT TAGATCGCTC TGTGTAGTAG TTGTAGTATA TCCACTCTCT
   2220
   CAAAGCATCC AGGCGCCCCC TGGCTTCGGG TTCTATGTAA ACTCCTTCAT GCGCCGCTGC
   2280
   CCTGATAACA TCCACCACCG CAGAATAAGC CACACCCAGC CAACCTACAC ATTCGTTCTG
   2340
   CGAGTCACAC ACGGGAGGAG CGGGAAGAGC TGGAAGAACC ATGTTTTTTT TTTTATTCCA
   2400
   AAAGATTATC CAAAACCTCA AAATGAAGAT CTATTAAGTG AACGCGCTCC CCTCCGGTGG
   2460
   CGTGGTCAAA CTCTACAGCC AAAGAACAGA TAATGGCATT TGTAAGATGT TGCACAATGG
   2520
   CTTCCAAAAG GCAAACGGCC CTCACGTCCA AGTGGACGTA AAGGCTAAAC CCTTCAGGGT
   2580
   GAATCTCCTC TATAAACATT CCAGCACCTT CAACCATGCC CAAATAATTC TCATCTCGCC
   2640
   ACCTTCTCAA TATATCTCTA AGCAAATCCC GAATATTAAG TCCGGCCATT GTAAAAATCT
   2700
   GCTCCAGAGC GCCCTCCACC TTCAGCCTCA AGCAGCGAAT CATGATTGCA AAAATTCAGG 2760
   TTCCTCACAG ACCTGTATAA GATTCAAAAG CGGAACATTA ACAAAAATAC CGCGATCCCG 2820
   TAGGTCCCTT CGCAGGGCCA GCTGAACATA ATCGTGCAGG TCTGCACGGA CCAGCGCGGC 2880
   CACTTCCCCG CCAGGAACCT TGACAAAAGA ACCCACACTG ATTATGACAC GCATACTCGG 2940
   AGCTATGCTA ACCAGCGTAG CCCCGATGTA AGCTTTGTTG CATGGGCGGC GATATAAAAT 3000
   GCAAGGTGCT GCTCAAAAAA TCAGGCAAAG CCTCGCGCAA AAAAGAAAGC ACATCGTAGT 3060
   CATGCTCATG CAGATAAAGG CAGGTAAGCT CCGGAACCAC CACAGAAAAA GACACCATTT 3120
   TTCTCTCAAA CATGTCTGCG GGTTTCTGCA TAAACACAAA ATAAAATAAC AAAAAAACAT 3180
   TTAAACATTA GAAGCCTGTC TTACAACAGG AAAAACAACC CTTATAAGCA TAAGACGGAC 3240
   TACGGCCATG CCGGCGTGAC CGTAAAAAAA CTGGTCACCG TGATTAAAAA GCACCACCGA 3300
   CAGCTCCTCG GTCATGTCCG GAGTCATAAT GTAAGACTCG GTAAACACAT CAGGTTGATT 3360
   CATCGGTCAG TGCTAAAAAG CGACCGAAAT AGCCCGGGGG AATACATACC CGCAGGCGTA 3420
   GAGACAACAT TACAGCCCCC ATAGGAGGTA TAACAAAATT AATAGGAGAG AAAAACACAT 3480
   AAACACCTGA AAAACCCTCC TGCCTAGGCA AAATAGCACC CTCCCGCTCC AGAACAACAT 3540
   ACAGCGCTTC ACAGCGGCAG CCTAACAGTC AGCCTTACCA GTAAAAAAGA AAACCTATTA 3600
   AAAAAACACC ACTCGACACG GCACCAGCTC AATCAGTCAC AGTGTAAAAA AGGGCCAAGT 3660
   GCAGAGCGAG TATATATAGG ACTAAAAAAT GACGTAACGG TTAAAGTCCA CAAAAAACAC 3720
   CCAGAAAACC GCACGCGAAC CTACGCCCAG AAACGAAAGC CAAAAAACCC ACAACTTCCT 3780
   CAAATCGTCA CTTCCGTTTT CCCACGTTAC GTAACTTCCC GGATCCGCGG CATTCACAGT 3840
   TCTCCGCAAG AATTGATTGG CTCCAATTCT TGGAGTGGTG AATCCGTTAG CGAGGTGCCG 3900
   CCGGCTTCCA TTCAGGTCGA GGTGGCCCGG CTCCATGCAC CGCGACGCAA CGCGGGGAGG 3960
   CAGACAAGGT ATAGGGCGGC GCCTACAATC CATGCCAACC CGTTCCATGT GCTCGCCGAG 4020
   GCGGCATAAA TCGCCGTGAC GATCAGCGGT CCAGTGATCG AAGTTAGGCT GGTAAGAGCC 4080
   GCGAGCGATC CTTGAAGCTG TCCCTGATGG TCGTCATCTA CCTGCCTGGA CAGCATGGCC 4140
   TGCAACGCGG GCATCCCGAT GCCGCCGGAA GCGAGAAGAA TCATAATGGG GAAGGCCATC
   4200
   CAGCCTCGCG TCGCGAACGC CAGCAAGACG TAGCCCAGCG CGTCGGCCGC CATGCCCTGC
   4260
   TTCATCCCCG TGGCCCGTTG CTCGCGTTTG CTGGCGGTGT CCCCGGAAGA AATATATTTG
   4320
   CATGTCTTTA GTTCTATGAT GACACAAACC CCGCCCAGCG TCTTGTCATT GGCGAATTCG
   4380
   AACACGCAGA TGCAGTCGGG GCGGCGCGGT CCCAGGTCCA CTTCGCATAT TAAGGTGACG
   4440
   CGTGTGGCCT CGAACACCGA GCGACCCTGC AGCGACCCGC TTAACAGCGT CAACAGCGTG
   4500
   CCGCAGATCC CGGGCAATGA GATATGAAAA AGCCTGAACT CACCGCGACG TCTGTCGAGA
   4560
   AGTTTCTGAT CGAAAAGTTC GACAGCGTCT CCGACCTGAT GCAGCTCTCG GAGGGCGAAG
   4620
   AATCTCGTGC TTTCAGCTTC GATGTAGGAG GGCGTGGATA TGTCCTGCGG GTAAATAGCT
   4680
   GCGCCGATGG TTTCTACAAA GATCGTTATG TTTATCGGCA CTTTGCATCG GCCGCGCTCC
   4740
   CGATTCCGGA AGTGCTTGAC ATTGGGGAAT TCAGCGAGAG CCTGACCTAT TGCATCTCCC
   4800
   GCCGTGCACA GGGTGTCACG TTGCAAGACC TGCCTGAAAC CGAACTGCCC GCTGTTCTGC
   4860
   AGCCGGTCGC GGAGGCCATG GATGCGATCG CTGCGGCCGA TCTTAGCCAG ACGAGCGGGT 4920
   TCGGCCCATT CGGACCGCAA GGAATCGGTC AATACACTAC ATGGCGTGAT TTCATATGCG 4980
   CGATTGCTGA TCCCCATGTG TATCACTGGC AAACTGTGAT GGACGACACC GTCAGTGCGT 5040
   CCGTCGCGCA GGCTCTCGAT GAGCTGATGC TTTGGGCCGA GGACTGCCCC GAAGTCCGGC 5100
   ACCTCGTGCA CGCGGATTTC GGCTCCAACA ATGTCCTGAC GGACAATGGC CGCATAACAG 5160
   CGGTCATTGA CTGGAGCGAG GCGATGTTCG GGGATTCCCA ATACGAGGTC GCCAACATCT 5220
   TCTTCTGGAG GCCGTGGTTG GCTTGTATGG AGCAGCAGAC GCGCTACTTC GAGCGGAGGC 5280
   ATCCGGAGCT TGCAGGATCG CCGCGGCTCC GGGCGTATAT GCTCCGCATT GGTCTTGACC 5340
   AACTCTATCA GAGCTTGGTT GACGGCAATT TCGATGATGC AGCTTGGGCG CAGGGTCGAT 5400
   GCGACGCAAT CGTCCGATCC GGAGCCGGGA CTGTCGGGCG TACACAAATC GCCCGCAGAA 5460
   GCGCGGCCGT CTGGACCGAT GGCTGTGTAG AAGTACTCGC CGATAGTGGA AACCGACGCC 5520
   CCAGCACTCG TCCGAGGGCA AAGGAATAGG GGAGATGGGG GAGGCTAACT GAAACACGGA 5580
   AGGAGACAAT ACCGGAAGGA ACCCGCGCTA TGACGGCAAT AAAAAGACAG AATAAAACGC 5640
   ACGGGTGTTG GGTCGTTTGT TCATAAACGC GGGGTTCGGT CCCAGGGCTG GCACTCTGTC 5700
   GATACCCCAC CGAGACCCCA TTGGGGCCAA TACGCCCGCG TTTCTTCCTT TTCCCCACCC 5760
   CACCCCCCAA GTTCGGGTGA AGGCCCAGGG CTCGCAGCCA ACGTCGGGGC GGCAGGCCCT 5820
   GCCATAGCCA CTGGCCCCGT GGGTTAGGGA CGGGGTCCCC CATGGGGAAT GGTTTATGGT 5880
   TCGTGGGGGT TATTATTTTG GGCGTTGCGT GGGGTCTGGT CCACGACTGG ACTGAGCAGA 5940
   CAGACCCATG GTTTTTGGAT GGCCTGGGCA TGGACCGCAT GTACTGGCGC GACACGAACA 6000
   CCGGGCGTCT GTGGCTGCCA AACACCCCCG ACCCCCAAAA ACCACCGCGC GGATTTCTGG 6060
   CGCCCAGTGC CGTCGACCGG TCATGGCTGC GCCCCGACAC CCGCCAACAC CCGCTGACGC 6120
   GCCCTGACGG GCTTGTCTGC TCCCGGCATC CGCTTACAGA CAAGCTGTGA CCGTCTCCGG 6180
   GAGCTGCATG TGTCAGAGGT TTTCACCGTC ATCACCGAAA CGCGCGAGGC AGCCGGATCA 6240
   TAATCAGCCA TACCACATTT GTAGAGGTTT TACTTGCTTT AAAAAACCTC CCCACCTCCC 6300
   CCTGAACCTG AAACATAAAA TGAATGCAAT TGTTGTTGTT AACTTGTTTA TTGCAGCTTA 6360
   TAATGGTTAC AAATAAAGCA ATAGCATCAC AAATTTCACA AATAAAGCAT TTTTTTCACT 6420
   GCATTCTAGT TGTGGTTTGT CCAAACTCAT CAATGTATCT TATCATGTCT GGATCCACTA 6480
   GTTCTAGAGC GGCCGCCACC GCGGTGGAGC TCCAGCTTTT GTTCCCTTTA GTGAGGGTTA 6540
   ATTTCGAGCT TGGCGTAATC ATGGTCATAG CTGTTTCCTG TGTGAAATTG TTATCCGCTC 6600
   ACAATTCCAC ACAACATACG AGCCGGAAGC ATAAAGTGTA AAGCCTGGGG TGCCTAATGA 6660
   GTGAGCTAAC TCACATTAAT TGCGTTGCGC TCACTGCCCG CTTTCCAGTC GGGAAACCTG 6720
   TCGTGCCAGC TGCATTAATG AATCGGCCAA CGCGCGGGGA GAGGCGGTTT GCGTATTGGG 6780
   CGCTCTTCCG CTTCCTCGCT CACTGACTCG CTGCGCTCGG TCGTTCGGCT GCGGCGAGCG 6840
   GTATCAGCTC ACTCAAAGGC GGTAATACGG TTATCCACAG AATCAGGGGA TAACGCAGGA 6900
   AAGAACATGT GAGCAAAAGG CCAGCAAAAG GCCAGGAACC GTAAAAAGGC CGCGTTGCTG 6960
   GCGTTTTTCC ATAGGCTCCG CCCCCCTGAC GAGCATCACA AAAATCGACG CTCAAGTCAG 7020
   AGGTGGCGAA ACCCGACAGG ACTATAAAGA TACCAGGCGT TTCCCCCTGG AAGCTCCCTC 7080
   GTGCGCTCTC CTGTTCCGAC CCTGCCGCTT ACCGGATACC TGTCCGCCTT TCTCCCTTCG 7140
   GGAAGCGTGG CGCTTTCTCA TAGCTCACGC TGTAGGTATC TCAGTTCGGT GTAGGTCGTT 7200
   CGCTCCAAGC TGGGCTGTGT GCACGAACCC CCCGTTCAGC CCGACCGCTG CGCCTTATCC 7260
   GGTAACTATC GTCTTGAGTC CAACCCGGTA AGACACGACT TATCGCCACT GGCAGCAGCC 7320
   ACTGGTAACA GGATTAGCAG AGCGAGGTAT GTAGGCGGTG CTACAGAGTT CTTGAAGTGG 7380
   TGGCCTAACT ACGGCTACAC TAGAAGGACA GTATTTGGTA TCTGCGCTCT GCTGAAGCCA 7440
   GTTACCTTCG GAAAAAGAGT TGGTAGCTCT TGATCCGGCA AACAAACCAC CGCTGGTAGC 7500
   GGTGGTTTTT TTGTTTGCAA GCAGCAGATT ACGCGCAGAA AAAAAGGATC TCAAGAAGAT 7560
   CCTTTGATCT TTTCTACGGG GTCTGACGCT CAGTGGAACG AAAACTCACG TTAAGGGATT 7620
   TTGGTCATGA GATTATCAAA AAGGATCTTC ACCTAGATCC TTTTAAATTA AAAATGAAGT 7680
   TTTAAATCAA TCTAAAGTAT ATATGAGTAA ACTTGGTCTG ACAGTTACCA ATGCTTAATC 7740
   AGTGAGGCAC CTATCTCAGC GATCTGTCTA TTTCGTTCAT CCATAGTTGC CTGACTCCCC 7800
   GTCGTGTAGA TAACTACGAT ACGGGAGGGC TTACCATCTG GCCCCAGTGC TGCAATGATA 7860
   CCGCGAGACC CACGCTCACC GGCTCCAGAT TTATCAGCAA TAAACCAGCC AGCCGGAAGG 7920
   GCCGAGCGCA GAAGTGGTCC TGCAACTTTA TCCGCCTCCA TCCAGTCTAT TAATTGTTGC 7980
   CGGGAAGCTA GAGTAAGTAG TTCGCCAGTT AATAGTTTGC GCAACGTTGT TGCCATTGCT 8040
   ACAGGCATCG TGGTGTCACG CTCGTCGTTT GGTATGGCTT CATTCAGCTC CGGTTCCCAA 8100
   CGATCAAGGC GAGTTACATG ATCCCCCATG TTGTGCAAAA AAGCGGTTAG CTCCTTCGGT 8160
   CCTCCGATCG TTGTCAGAAG TAAGTTGGCC GCAGTGTTAT CACTCATGGT TATGGCAGCA 8220
   CTGCATAATT CTCTTACTGT CATGCCATCC GTAAGATGCT TTTCTGTGAC TGGTGAGTAC 8280
   TCAACCAAGT CATTCTGAGA ATAGTGTATG CGGCGACCGA GTTGCTCTTG CCCGGCGTCA 8340
   ATACGGGATA ATACCGCGCC ACATAGCAGA ACTTTAAAAG TGCTCATCAT TGGAAAACGT 8400
   TCTTCGGGGC GAAAACTCTC AAGGATCTTA CCGCTGTTGA GATCCAGTTC GATGTAACCC 8460
   ACTCGTGCAC CCAACTGATC TTCAGCATCT TTTACTTTCA CCAGCGTTTC TGGGTGAGCA 8520
   AAAACAGGAA GGCAAAATGC CGCAAAAAAG GGAATAAGGG CGACACGGAA ATGTTGAATA
   8580
   CTCATACTCT TCCTTTTTCA ATATTATTGA AGCATTTATC AGGGTTATTG TCTCATGAGC
   8640
   GGATACATAT TTGAATGTAT TTAGAAAAAT AAACAAATAG GGGTTCCGCG CACATTTCCC
   8700
   CGAAAAGTGC
   8710
(2) INFORMATION FOR SEQ ID NO:5:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:5:
   ATGGGATCCA AGATGAAGCG CGCAAGACCG
   30
(2) INFORMATION FOR SEQ ID NO:6:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:6:
   CATAACGCGG CCGCTTCTTT ATTCTTGGGC
   30
(2) INFORMATION FOR SEQ ID NO:7:
   (i) SEQUENCE CHARACTERISTICS:
      (A) LENGTH: 7148 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:7:
   GACGGATCGG GAGATCTCCC GATCCCCTAT GGTCGACTCT CAGTACAATC TGCTCTGATG
   60
   CCGCATAGTT AAGCCAGTAT CTGCTCCCTG CTTGTGTGTT GGAGGTCGCT GAGTAGTGCG 120
   CGAGCAAAAT TTAAGCTACA ACAAGGCAAG GCTTGACCGA CAATTGCATG AAGAATCTGC 180
   TTAGGGTTAG GCGTTTTGCG CTGCTTCGCG ATGTACGGGC CAGATATACG CGTTGACATT 240
   GATTATTGAC TAGTTATTAA TAGTAATCAA TTACGGGGTC ATTAGTTCAT AGCCCATATA 300
   TGGAGTTCCG CGTTACATAA CTTACGGTAA ATGGCCCGCC TGGCTGACCG CCCAACGACC 360
   CCCGCCCATT GACGTCAATA ATGACGTATG TTCCCATAGT AACGCCAATA GGGACTTTCC 420
   ATTGACGTCA ATGGGTGGAC TATTTACGGT AAACTGCCCA CTTGGCAGTA CATCAAGTGT 480
   ATCATATGCC AAGTACGCCC CCTATTGACG TCAATGACGG TAAATGGCCC GCCTGGCATT 540
   ATGCCCAGTA CATGACCTTA TGGGACTTTC CTACTTGGCA GTACATCTAC GTATTAGTCA 600
   TCGCTATTAC CATGGTGATG CGGTTTTGGC AGTACATCAA TGGGCGTGGA TAGCGGTTTG 660
   ACTCACGGGG ATTTCCAAGT CTCCACCCCA TTGACGTCAA TGGGAGTTTG TTTTGGCACC 720
   AAAATCAACG GGACTTTCCA AAATGTCGTA ACAACTCCGC CCCATTGACG CAAATGGGCG 780
   GTAGGCGTGT ACGGTGGGAG GTCTATATAA GCAGAGCTCT CTGGCTAACT AGAGAACCCA
   840
   CTGCTTACTG GCTTATCGAA ATTAATACGA CTCACTATAG GGAGACCCAA GCTTGGTACC
   900
   GAGCTCGGAT CCAAGATGAA GCGCGCAAGA CCGTCTGAAG ATACCTTCAA CCCCGTGTAT
   960
   CCATATGACA CGGAAACCGG TCCTCCAACT GTGCCTTTTC TTACTCCTCC CTTTGTATCC
   1020
   CCCAATGGGT TTCAAGAGAG TCCCCCTGGG GTACTCTCTT TGCGCCTATC CGAACCTCTA
   1080
   GTTACCTCCA ATGGCATGCT TGCGCTCAAA ATGGGCAACG GCCTCTCTCT GGACGAGGCC
   1140
   GGCAACCTTA CCTCCCAAAA TGTAACCACT GTGAGCCCAC CTCTCAAAAA AACCAAGTCA
   1200
   AACATAAACC TGGAAATATC TGCACCCCTC ACAGTTACCT CAGAAGCCCT AACTGTGGCT
   1260
   GCCGCCGCAC CTCTAATGGT CGCGGGCAAC ACACTCACCA TGCAATCACA GGCCCCGCTA
   1320
   ACCGTGCACG ACTCCAAACT TAGCATTGCC ACCCAAGGAC CCCTCACAGT GTCAGAAGGA
   1380
   AAGCTAGCCC TGCAAACATC AGGCCCCCTC ACCACCACCG ATAGCAGTAC CCTTACTATC
   1440
   ACTGCCTCAC CCCCTCTAAC TACTGCCACT GGTAGCTTGG GCATTGACTT GAAAGAGCCC
   1500
   ATTTATACAC AAAATGGAAA ACTAGGACTA AAGTACGGGG CTCCTTTGCA TGTAACAGAC 1560
   GACCTAAACA CTTTGACCGT AGCAACTGGT CCAGGTGTGA CTATTAATAA TACTTCCTTG 1620
   CAAACTAAAG TTACTGGAGC CTTGGGTTTT GATTCACAAG GCAATATGCA ACTTAATGTA 1680
   GCAGGAGGAC TAAGGATTGA TTCTCAAAAC AGACGCCTTA TACTTGATGT TAGTTATCCG 1740
   TTTGATGCTC AAAACCAACT AAATCTAAGA CTAGGACAGG GCCCTCTTTT TATAAACTCA 1800
   GCCCACAACT TGGATATTAA CTACAACAAA GGCCTTTACT TGTTTACAGC TTCAAACAAT 1860
   TCCAAAAAGC TTGAGGTTAA CCTAAGCACT GCCAAGGGGT TGATGTTTGA CGCTACAGCC 1920
   ATAGCCATTA ATGCAGGAGA TGGGCTTGAA TTTGGTTCAC CTAATGCACC AAACACAAAT 1980
   CCCCTCAAAA CAAAAATTGG CCATGGCCTA GAATTTGATT CAAACAAGGC TATGGTTCCT 2040
   AAACTAGGAA CTGGCCTTAG TTTTGACAGC ACAGGTGCCA TTACAGTAGG AAACAAAAAT 2100
   AATGATAAGC TAACTTTGTG GACCACACCA GCTCCATCTC CTAACTGTAG ACTAAATGCA 2160
   GAGAAAGATG CTAAACTCAC TTTGGTCTTA ACAAAATGTG GCAGTCAAAT ACTTGCTACA 2220
   GTTTCAGTTT TGGCTGTTAA AGGCAGTTTG GCTCCAATAT CTGGAACAGT TCAAAGTGCT 2280
   CATCTTATTA TAAGATTTGA CGAAAATGGA GTGCTACTAA ACAATTCCTT CCTGGACCCA 2340
   GAATATTGGA ACTTTAGAAA TGGAGATCTT ACTGAAGGCA CAGCCTATAC AAACGCTGTT 2400
   GGATTTATGC CTAACCTATC AGCTTATCCA AAATCTCACG GTAAAACTGC CAAAAGTAAC 2460
   ATTGTCAGTC AAGTTTACTT AAACGGAGAC AAAACTAAAC CTGTAACACT AACCATTACA 2520
   CTAAACGGTA CACAGGAAAC AGGAGACACA ACTCCAAGTG CATACTCTAT GTCATTTTCA 2580
   TGGGACTGGT CTGGCCACAA CTACATTAAT GAAATATTTG CCACATCCTC TTACACTTTT 2640
   TCATACATTG CCCAAGAATA AAGAAGCGGC CGCTCGAGCA TGCATCTAGA GGGCCCTATT 2700
   CTATAGTGTC ACCTAAATGC TAGAGCTCGC TGATCAGCCT CGACTGTGCC TTCTAGTTGC 2760
   CAGCCATCTG TTGTTTGCCC CTCCCCCGTG CCTTCCTTGA CCCTGGAAGG TGCCACTCCC 2820
   ACTGTCCTTT CCTAATAAAA TGAGGAAATT GCATCGCATT GTCTGAGTAG GTGTCATTCT 2880
   ATTCTGGGGG GTGGGGTGGG GCAGGACAGC AAGGGGGAGG ATTGGGAAGA CAATAGCAGG 2940
   CATGCTGGGG ATGCGGTGGG CTCTATGGCT TCTGAGGCGG AAAGAACCAG CTGGGGCTCT 3000
   AGGGGGTATC CCCACGCGCC CTGTAGCGGC GCATTAAGCG CGGCGGGTGT GGTGGTTACG 3060
   CGCAGCGTGA CCGCTACACT TGCCAGCGCC CTAGCGCCCG CTCCTTTCGC TTTCTTCCCT 3120
   TCCTTTCTCG CCACGTTCGC CGGCTTTCCC CGTCAAGCTC TAAATCGGGG CATCCCTTTA 3180
   GGGTTCCGAT TTAGTGCTTT ACGGCACCTC GACCCCAAAA AACTTGATTA GGGTGATGGT 3240
   TCACGTAGTG GGCCATCGCC CTGATAGACG GTTTTTCGCC CTTTGACGTT GGAGTCCACG 3300
   TTCTTTAATA GTGGACTCTT GTTCCAAACT GGAACAACAC TCAACCCTAT CTCGGTCTAT 3360
   TCTTTTGATT TATAAGGGAT TTTGGGGATT TCGGCCTATT GGTTAAAAAA TGAGCTGATT 3420
   TAACAAAAAT TTAACGCGAA TTAATTCTGT GGAATGTGTG TCAGTTAGGG TGTGGAAAGT 3480
   CCCCAGGCTC CCCAGGCAGG CAGAAGTATG CAAAGCATGC ATCTCAATTA GTCAGCAACC 3540
   AGGTGTGGAA AGTCCCCAGG CTCCCCAGCA GGCAGAAGTA TGCAAAGCAT GCATCTCAAT 3600
   TAGTCAGCAA CCATAGTCCC GCCCCTAACT CCGCCCATCC CGCCCCTAAC TCCGCCCAGT 3660
   TCCGCCCATT CTCCGCCCCA TGGCTGACTA ATTTTTTTTA TTTATGCAGA GGCCGAGGCC 3720
   GCCTCTGCCT CTGAGCTATT CCAGAAGTAG TGAGGAGGCT TTTTTGGAGG CCTAGGCTTT 3780
   TGCAAAAAGC TCCCGGGAGC TTGTATATCC ATTTTCGGAT CTGATCAAGA GACAGGATGA 3840
   GGATCGTTTC GCATGATTGA ACAAGATGGA TTGCACGCAG GTTCTCCGGC CGCTTGGGTG 3900
   GAGAGGCTAT TCGGCTATGA CTGGGCACAA CAGACAATCG GCTGCTCTGA TGCCGCCGTG 3960
   TTCCGGCTGT CAGCGCAGGG GCGCCCGGTT CTTTTTGTCA AGACCGACCT GTCCGGTGCC 4020
   CTGAATGAAC TGCAGGACGA GGCAGCGCGG CTATCGTGGC TGGCCACGAC GGGCGTTCCT 4080
   TGCGCAGCTG TGCTCGACGT TGTCACTGAA GCGGGAAGGG ACTGGCTGCT ATTGGGCGAA 4140
   GTGCCGGGGC AGGATCTCCT GTCATCTCAC CTTGCTCCTG CCGAGAAAGT ATCCATCATG 4200
   GCTGATGCAA TGCGGCGGCT GCATACGCTT GATCCGGCTA CCTGCCCATT CGACCACCAA 4260
   GCGAAACATC GCATCGAGCG AGCACGTACT CGGATGGAAG CCGGTCTTGT CGATCAGGAT 4320
   GATCTGGACG AAGAGCATCA GGGGCTCGCG CCAGCCGAAC TGTTCGCCAG GCTCAAGGCG 4380
   CGCATGCCCG ACGGCGAGGA TCTCGTCGTG ACCCATGGCG ATGCCTGCTT GCCGAATATC 4440
   ATGGTGGAAA ATGGCCGCTT TTCTGGATTC ATCGACTGTG GCCGGCTGGG TGTGGCGGAC 4500
   CGCTATCAGG ACATAGCGTT GGCTACCCGT GATATTGCTG AAGAGCTTGG CGGCGAATGG 4560
   GCTGACCGCT TCCTCGTGCT TTACGGTATC GCCGCTCCCG ATTCGCAGCG CATCGCCTTC 4620
   TATCGCCTTC TTGACGAGTT CTTCTGAGCG GGACTCTGGG GTTCGAAATG ACCGACCAAG 4680
   CGACGCCCAA CCTGCCATCA CGAGATTTCG ATTCCACCGC CGCCTTCTAT GAAAGGTTGG 4740
   GCTTCGGAAT CGTTTTCCGG GACGCCGGCT GGATGATCCT CCAGCGCGGG GATCTCATGC 4800
   TGGAGTTCTT CGCCCACCCC AACTTGTTTA TTGCAGCTTA TAATGGTTAC AAATAAAGCA 4860
   ATAGCATCAC AAATTTCACA AATAAAGCAT TTTTTTCACT GCATTCTAGT TGTGGTTTGT 4920
   CCAAACTCAT CAATGTATCT TATCATGTCT GTATACCGTC GACCTCTAGC TAGAGCTTGG 4980
   CGTAATCATG GTCATAGCTG TTTCCTGTGT GAAATTGTTA TCCGCTCACA ATTCCACACA 5040
   ACATACGAGC CGGAAGCATA AAGTGTAAAG CCTGGGGTGC CTAATGAGTG AGCTAACTCA 5100
   CATTAATTGC GTTGCGCTCA CTGCCCGCTT TCCAGTCGGG AAACCTGTCG TGCCAGCTGC
   5160
   ATTAATGAAT CGGCCAACGC GCGGGGAGAG GCGGTTTGCG TATTGGGCGC TCTTCCGCTT
   5220
   CCTCGCTCAC TGACTCGCTG CGCTCGGTCG TTCGGCTGCG GCGAGCGGTA TCAGCTCACT
   5280
   CAAAGGCGGT AATACGGTTA TCCACAGAAT CAGGGGATAA CGCAGGAAAG AACATGTGAG
   5340
   CAAAAGGCCA GCAAAAGGCC AGGAACCGTA AAAAGGCCGC GTTGCTGGCG TTTTTCCATA
   5400
   GGCTCCGCCC CCCTGACGAG CATCACAAAA ATCGACGCTC AAGTCAGAGG TGGCGAAACC
   5460
   CGACAGGACT ATAAAGATAC CAGGCGTTTC CCCCTGGAAG CTCCCTCGTG CGCTCTCCTG
   5520
   TTCCGACCCT GCCGCTTACC GGATACCTGT CCGCCTTTCT CCCTTCGGGA AGCGTGGCGC
   5580
   TTTCTCAATG CTCACGCTGT AGGTATCTCA GTTCGGTGTA GGTCGTTCGC TCCAAGCTGG
   5640
   GCTGTGTGCA CGAACCCCCC GTTCAGCCCG ACCGCTGCGC CTTATCCGGT AACTATCGTC
   5700
   TTGAGTCCAA CCCGGTAAGA CACGACTTAT CGCCACTGGC AGCAGCCACT GGTAACAGGA
   5760
   TTAGCAGAGC GAGGTATGTA GGCGGTGCTA CAGAGTTCTT GAAGTGGTGG CCTAACTACG
   5820
   GCTACACTAG AAGGACAGTA TTTGGTATCT GCGCTCTGCT GAAGCCAGTT ACCTTCGGAA
   5880
   AAAGAGTTGG TAGCTCTTGA TCCGGCAAAC AAACCACCGC TGGTAGCGGT GGTTTTTTTG
   5940
   TTTGCAAGCA GCAGATTACG CGCAGAAAAA AAGGATCTCA AGAAGATCCT TTGATCTTTT
   6000
   CTACGGGGTC TGACGCTCAG TGGAACGAAA ACTCACGTTA AGGGATTTTG GTCATGAGAT
   6060
   TATCAAAAAG GATCTTCACC TAGATCCTTT TAAATTAAAA ATGAAGTTTT AAATCAATCT
   6120
   AAAGTATATA TGAGTAAACT TGGTCTGACA GTTACCAATG CTTAATCAGT GAGGCACCTA
   6180
   TCTCAGCGAT CTGTCTATTT CGTTCATCCA TAGTTGCCTG ACTCCCCGTC GTGTAGATAA
   6240
   CTACGATACG GGAGGGCTTA CCATCTGGCC CCAGTGCTGC AATGATACCG CGAGACCCAC
   6300
   GCTCACCGGC TCCAGATTTA TCAGCAATAA ACCAGCCAGC CGGAAGGGCC GAGCGCAGAA
   6360
   GTGGTCCTGC AACTTTATCC GCCTCCATCC AGTCTATTAA TTGTTGCCGG GAAGCTAGAG
   6420
   TAAGTAGTTC GCCAGTTAAT AGTTTGCGCA ACGTTGTTGC CATTGCTACA GGCATCGTGG
   6480
   TGTCACGCTC GTCGTTTGGT ATGGCTTCAT TCAGCTCCGG TTCCCAACGA TCAAGGCGAG
   6540
   TTACATGATC CCCCATGTTG TGCAAAAAAG CGGTTAGCTC CTTCGGTCCT CCGATCGTTG
   6600
   TCAGAAGTAA GTTGGCCGCA GTGTTATCAC TCATGGTTAT GGCAGCACTG CATAATTCTC
   6660
   TTACTGTCAT GCCATCCGTA AGATGCTTTT CTGTGACTGG TGAGTACTCA ACCAAGTCAT
   6720
   TCTGAGAATA GTGTATGCGG CGACCGAGTT GCTCTTGCCC GGCGTCAATA CGGGATAATA
   6780
   CCGCGCCACA TAGCAGAACT TTAAAAGTGC TCATCATTGG AAAACGTTCT TCGGGGCGAA
   6840
   AACTCTCAAG GATCTTACCG CTGTTGAGAT CCAGTTCGAT GTAACCCACT CGTGCACCCA
   6900
   ACTGATCTTC AGCATCTTTT ACTTTCACCA GCGTTTCTGG GTGAGCAAAA ACAGGAAGGC
   6960
   AAAATGCCGC AAAAAAGGGA ATAAGGGCGA CACGGAAATG TTGAATACTC ATACTCTTCC
   7020
   TTTTTCAATA TTATTGAAGC ATTTATCAGG GTTATTGTCT CATGAGCGGA TACATATTTG
   7080
   AATGTATTTA GAAAAATAAA CAAATAGGGG TTCCGCGCAC ATTTCCCCGA AAAGTGCCAC
   7140
   CTGACGTC
   7148
(2) INFORMATION FOR SEQ ID NO:8:
   (i) SEQUENCE CHARACTERISTICS:
      (A) LENGTH: 7469 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:8:
   GACGGATCGG GAGATCTCCC GATCCCCTAT GGTCGACTCT CAGTACAATC TGCTCTGATG 60
   CCGCATAGTT AAGCCAGTAT CTGCTCCCTG CTTGTGTGTT GGAGGTCGCT GAGTAGTGCG 120
   CGAGCAAAAT TTAAGCTACA ACAAGGCAAG GCTTGACCGA CAATTGCATG AAGAATCTGC 180
   TTAGGGTTAG GCGTTTTGCG CTGCTTCGCG ATGTACGGGC CAGATATACG CGTTGACATT 240
   GATTATTGAC TAGTTATTAA TAGTAATCAA TTACGGGGTC ATTAGTTCAT AGCCCATATA 300
   TGGAGTTCCG CGTTACATAA CTTACGGTAA ATGGCCCGCC TGGCTGACCG CCCAACGACC 360
   CCCGCCCATT GACGTCAATA ATGACGTATG TTCCCATAGT AACGCCAATA GGGACTTTCC 420
   ATTGACGTCA ATGGGTGGAC TATTTACGGT AAACTGCCCA CTTGGCAGTA CATCAAGTGT 480
   ATCATATGCC AAGTACGCCC CCTATTGACG TCAATGACGG TAAATGGCCC GCCTGGCATT
   540
   ATGCCCAGTA CATGACCTTA TGGGACTTTC CTACTTGGCA GTACATCTAC GTATTAGTCA
   600
   TCGCTATTAC CATGGTGATG CGGTTTTGGC AGTACATCAA TGGGCGTGGA TAGCGGTTTG
   660
   ACTCACGGGG ATTTCCAAGT CTCCACCCCA TTGACGTCAA TGGGAGTTTG TTTTGGCACC
   720
   AAAATCAACG GGACTTTCCA AAATGTCGTA ACAACTCCGC CCCATTGACG CAAATGGGCG
   780
   GTAGGCGTGT ACGGTGGGAG GTCTATATAA GCAGAGCTCT CTGGCTAACT AGAGAACCCA
   840
   CTGCTTACTG GCTTATCGAA ATTAATACGA CTCACTATAG GGAGACCCAA GCTTGGTACC
   900
   GAGCTCGGAT CTGAATTCGA GCTCGCTGTT GGGCTCGCGG TTGAGGACAA ACTCTTCGCG
   960
   GTCTTTCCAG TACTCTTGGA TCGGAAACCC GTCGGCCTCC GAACGGTACT CCGCCACCGA
   1020
   GGGACCTGAG CGAGTCCGCA TCGACCGGAT CGGAAAACCT CTCGAGAAAG GCGTCTAACC
   1080
   AGTCACAGTC GCAAGGTAGG CTGAGCACCG TGGCGGGCGG CAGCGGGTGG CGGTCGGGGT
   1140
   TGTTTCTGGC GGAGGTGCTG CTGATGATGT AATTAAAGTA GGCGGTCTTG AGACGGCGGA
   1200
   TGGTCGAGGT GAGGTGTGGC AGGCTTGAGA TCCAAGATGA AGCGCGCAAG ACCGTCTGAA 1260
   GATACCTTCA ACCCCGTGTA TCCATATGAC ACGGAAACCG GTCCTCCAAC TGTGCCTTTT 1320
   CTTACTCCTC CCTTTGTATC CCCCAATGGG TTTCAAGAGA GTCCCCCTGG GGTACTCTCT 1380
   TTGCGCCTAT CCGAACCTCT AGTTACCTCC AATGGCATGC TTGCGCTCAA AATGGGCAAC 1440
   GGCCTCTCTC TGGACGAGGC CGGCAACCTT ACCTCCCAAA ATGTAACCAC TGTGAGCCCA 1500
   CCTCTCAAAA AAACCAAGTC AAACATAAAC CTGGAAATAT CTGCACCCCT CACAGTTACC 1560
   TCAGAAGCCC TAACTGTGGC TGCCGCCGCA CCTCTAATGG TCGCGGGCAA CACACTCACC 1620
   ATGCAATCAC AGGCCCCGCT AACCGTGCAC GACTCCAAAC TTAGCATTGC CACCCAAGGA 1680
   CCCCTCACAG TGTCAGAAGG AAAGCTAGCC CTGCAAACAT CAGGCCCCCT CACCACCACC 1740
   GATAGCAGTA CCCTTACTAT CACTGCCTCA CCCCCTCTAA CTACTGCCAC TGGTAGCTTG 1800
   GGCATTGACT TGAAAGAGCC CATTTATACA CAAAATGGAA AACTAGGACT AAAGTACGGG 1860
   GCTCCTTTGC ATGTAACAGA CGACCTAAAC ACTTTGACCG TAGCAACTGG TCCAGGTGTG 1920
   ACTATTAATA ATACTTCCTT GCAAACTAAA GTTACTGGAG CCTTGGGTTT TGATTCACAA 1980
   GGCAATATGC AACTTAATGT AGCAGGAGGA CTAAGGATTG ATTCTCAAAA CAGACGCCTT 2040
   ATACTTGATG TTAGTTATCC GTTTGATGCT CAAAACCAAC TAAATCTAAG ACTAGGACAG 2100
   GGCCCTCTTT TTATAAACTC AGCCCACAAC TTGGATATTA ACTACAACAA AGGCCTTTAC 2160
   TTGTTTACAG CTTCAAACAA TTCCAAAAAG CTTGAGGTTA ACCTAAGCAC TGCCAAGGGG 2220
   TTGATGTTTG ACGCTACAGC CATAGCCATT AATGCAGGAG ATGGGCTTGA ATTTGGTTCA 2280
   CCTAATGCAC CAAACACAAA TCCCCTCAAA ACAAAAATTG GCCATGGCCT AGAATTTGAT 2340
   TCAAACAAGG CTATGGTTCC TAAACTAGGA ACTGGCCTTA GTTTTGACAG CACAGGTGCC 2400
   ATTACAGTAG GAAACAAAAA TAATGATAAG CTAACTTTGT GGACCACACC AGCTCCATCT 2460
   CCTAACTGTA GACTAAATGC AGAGAAAGAT GCTAAACTCA CTTTGGTCTT AACAAAATGT 2520
   GGCAGTCAAA TACTTGCTAC AGTTTCAGTT TTGGCTGTTA AAGGCAGTTT GGCTCCAATA 2580
   TCTGGAACAG TTCAAAGTGC TCATCTTATT ATAAGATTTG ACGAAAATGG AGTGCTACTA 2640
   AACAATTCCT TCCTGGACCC AGAATATTGG AACTTTAGAA ATGGAGATCT TACTGAAGGC
   2700
   ACAGCCTATA CAAACGCTGT TGGATTTATG CCTAACCTAT CAGCTTATCC AAAATCTCAC
   2760
   GGTAAAACTG CCAAAAGTAA CATTGTCAGT CAAGTTTACT TAAACGGAGA CAAAACTAAA
   2820
   CCTGTAACAC TAACCATTAC ACTAAACGGT ACACAGGAAA CAGGAGACAC AACTCCAAGT
   2880
   GCATACTCTA TGTCATTTTC ATGGGACTGG TCTGGCCACA ACTACATTAA TGAAATATTT
   2940
   GCCACATCCT CTTACACTTT TTCATACATT GCCCAAGAAT AAAGAAGCGG CCGCTCGAGC
   3000
   ATGCATCTAG AGGGCCCTAT TCTATAGTGT CACCTAAATG CTAGAGCTCG CTGATCAGCC
   3060
   TCGACTGTGC CTTCTAGTTG CCAGCCATCT GTTGTTTGCC CCTCCCCCGT GCCTTCCTTG
   3120
   ACCCTGGAAG GTGCCACTCC CACTGTCCTT TCCTAATAAA ATGAGGAAAT TGCATCGCAT
   3180
   TGTCTGAGTA GGTGTCATTC TATTCTGGGG GGTGGGGTGG GGCAGGACAG CAAGGGGGAG
   3240
   GATTGGGAAG ACAATAGCAG GCATGCTGGG GATGCGGTGG GCTCTATGGC TTCTGAGGCG
   3300
   GAAAGAACCA GCTGGGGCTC TAGGGGGTAT CCCCACGCGC CCTGTAGCGG CGCATTAAGC
   3360
   GCGGCGGGTG TGGTGGTTAC GCGCAGCGTG ACCGCTACAC TTGCCAGCGC CCTAGCGCCC 3420
   GCTCCTTTCG CTTTCTTCCC TTCCTTTCTC GCCACGTTCG CCGGCTTTCC CCGTCAAGCT 3480
   CTAAATCGGG GCATCCCTTT AGGGTTCCGA TTTAGTGCTT TACGGCACCT CGACCCCAAA 3540
   AAACTTGATT AGGGTGATGG TTCACGTAGT GGGCCATCGC CCTGATAGAC GGTTTTTCGC 3600
   CCTTTGACGT TGGAGTCCAC GTTCTTTAAT AGTGGACTCT TGTTCCAAAC TGGAACAACA 3660
   CTCAACCCTA TCTCGGTCTA TTCTTTTGAT TTATAAGGGA TTTTGGGGAT TTCGGCCTAT 3720
   TGGTTAAAAA ATGAGCTGAT TTAACAAAAA TTTAACGCGA ATTAATTCTG TGGAATGTGT 3780
   GTCAGTTAGG GTGTGGAAAG TCCCCAGGCT CCCCAGGCAG GCAGAAGTAT GCAAAGCATG 3840
   CATCTCAATT AGTCAGCAAC CAGGTGTGGA AAGTCCCCAG GCTCCCCAGC AGGCAGAAGT 3900
   ATGCAAAGCA TGCATCTCAA TTAGTCAGCA ACCATAGTCC CGCCCCTAAC TCCGCCCATC 3960
   CCGCCCCTAA CTCCGCCCAG TTCCGCCCAT TCTCCGCCCC ATGGCTGACT AATTTTTTTT 4020
   ATTTATGCAG AGGCCGAGGC CGCCTCTGCC TCTGAGCTAT TCCAGAAGTA GTGAGGAGGC 4080
   TTTTTTGGAG GCCTAGGCTT TTGCAAAAAG CTCCCGGGAG CTTGTATATC CATTTTCGGA 4140
   TCTGATCAAG AGACAGGATG AGGATCGTTT CGCATGATTG AACAAGATGG ATTGCACGCA 4200
   GGTTCTCCGG CCGCTTGGGT GGAGAGGCTA TTCGGCTATG ACTGGGCACA ACAGACAATC 4260
   GGCTGCTCTG ATGCCGCCGT GTTCCGGCTG TCAGCGCAGG GGCGCCCGGT TCTTTTTGTC 4320
   AAGACCGACC TGTCCGGTGC CCTGAATGAA CTGCAGGACG AGGCAGCGCG GCTATCGTGG 4380
   CTGGCCACGA CGGGCGTTCC TTGCGCAGCT GTGCTCGACG TTGTCACTGA AGCGGGAAGG 4440
   GACTGGCTGC TATTGGGCGA AGTGCCGGGG CAGGATCTCC TGTCATCTCA CCTTGCTCCT 4500
   GCCGAGAAAG TATCCATCAT GGCTGATGCA ATGCGGCGGC TGCATACGCT TGATCCGGCT 4560
   ACCTGCCCAT TCGACCACCA AGCGAAACAT CGCATCGAGC GAGCACGTAC TCGGATGGAA 4620
   GCCGGTCTTG TCGATCAGGA TGATCTGGAC GAAGAGCATC AGGGGCTCGC GCCAGCCGAA 4680
   CTGTTCGCCA GGCTCAAGGC GCGCATGCCC GACGGCGAGG ATCTCGTCGT GACCCATGGC 4740
   GATGCCTGCT TGCCGAATAT CATGGTGGAA AATGGCCGCT TTTCTGGATT CATCGACTGT 4800
   GGCCGGCTGG GTGTGGCGGA CCGCTATCAG GACATAGCGT TGGCTACCCG TGATATTGCT
   4860
   GAAGAGCTTG GCGGCGAATG GGCTGACCGC TTCCTCGTGC TTTACGGTAT CGCCGCTCCC
   4920
   GATTCGCAGC GCATCGCCTT CTATCGCCTT CTTGACGAGT TCTTCTGAGC GGGACTCTGG
   4980
   GGTTCGAAAT GACCGACCAA GCGACGCCCA ACCTGCCATC ACGAGATTTC GATTCCACCG
   5040
   CCGCCTTCTA TGAAAGGTTG GGCTTCGGAA TCGTTTTCCG GGACGCCGGC TGGATGATCC
   5100
   TCCAGCGCGG GGATCTCATG CTGGAGTTCT TCGCCCACCC CAACTTGTTT ATTGCAGCTT
   5160
   ATAATGGTTA CAAATAAAGC AATAGCATCA CAAATTTCAC AAATAAAGCA TTTTTTTCAC
   5220
   TGCATTCTAG TTGTGGTTTG TCCAAACTCA TCAATGTATC TTATCATGTC TGTATACCGT
   5280
   CGACCTCTAG CTAGAGCTTG GCGTAATCAT GGTCATAGCT GTTTCCTGTG TGAAATTGTT
   5340
   ATCCGCTCAC AATTCCACAC AACATACGAG CCGGAAGCAT AAAGTGTAAA GCCTGGGGTG
   5400
   CCTAATGAGT GAGCTAACTC ACATTAATTG CGTTGCGCTC ACTGCCCGCT TTCCAGTCGG
   5460
   GAAACCTGTC GTGCCAGCTG CATTAATGAA TCGGCCAACG CGCGGGGAGA GGCGGTTTGC
   5520
   GTATTGGGCG CTCTTCCGCT TCCTCGCTCA CTGACTCGCT GCGCTCGGTC GTTCGGCTGC 5580
   GGCGAGCGGT ATCAGCTCAC TCAAAGGCGG TAATACGGTT ATCCACAGAA TCAGGGGATA 5640
   ACGCAGGAAA GAACATGTGA GCAAAAGGCC AGCAAAAGGC CAGGAACCGT AAAAAGGCCG 5700
   CGTTGCTGGC GTTTTTCCAT AGGCTCCGCC CCCCTGACGA GCATCACAAA AATCGACGCT 5760
   CAAGTCAGAG GTGGCGAAAC CCGACAGGAC TATAAAGATA CCAGGCGTTT CCCCCTGGAA 5820
   GCTCCCTCGT GCGCTCTCCT GTTCCGACCC TGCCGCTTAC CGGATACCTG TCCGCCTTTC 5880
   TCCCTTCGGG AAGCGTGGCG CTTTCTCAAT GCTCACGCTG TAGGTATCTC AGTTCGGTGT 5940
   AGGTCGTTCG CTCCAAGCTG GGCTGTGTGC ACGAACCCCC CGTTCAGCCC GACCGCTGCG 6000
   CCTTATCCGG TAACTATCGT CTTGAGTCCA ACCCGGTAAG ACACGACTTA TCGCCACTGG 6060
   CAGCAGCCAC TGGTAACAGG ATTAGCAGAG CGAGGTATGT AGGCGGTGCT ACAGAGTTCT 6120
   TGAAGTGGTG GCCTAACTAC GGCTACACTA GAAGGACAGT ATTTGGTATC TGCGCTCTGC 6180
   TGAAGCCAGT TACCTTCGGA AAAAGAGTTG GTAGCTCTTG ATCCGGCAAA CAAACCACCG 6240
   CTGGTAGCGG TGGTTTTTTT GTTTGCAAGC AGCAGATTAC GCGCAGAAAA AAAGGATCTC 6300
   AAGAAGATCC TTTGATCTTT TCTACGGGGT CTGACGCTCA GTGGAACGAA AACTCACGTT 6360
   AAGGGATTTT GGTCATGAGA TTATCAAAAA GGATCTTCAC CTAGATCCTT TTAAATTAAA 6420
   AATGAAGTTT TAAATCAATC TAAAGTATAT ATGAGTAAAC TTGGTCTGAC AGTTACCAAT 6480
   GCTTAATCAG TGAGGCACCT ATCTCAGCGA TCTGTCTATT TCGTTCATCC ATAGTTGCCT 6540
   GACTCCCCGT CGTGTAGATA ACTACGATAC GGGAGGGCTT ACCATCTGGC CCCAGTGCTG 6600
   CAATGATACC GCGAGACCCA CGCTCACCGG CTCCAGATTT ATCAGCAATA AACCAGCCAG 6660
   CCGGAAGGGC CGAGCGCAGA AGTGGTCCTG CAACTTTATC CGCCTCCATC CAGTCTATTA 6720
   ATTGTTGCCG GGAAGCTAGA GTAAGTAGTT CGCCAGTTAA TAGTTTGCGC AACGTTGTTG 6780
   CCATTGCTAC AGGCATCGTG GTGTCACGCT CGTCGTTTGG TATGGCTTCA TTCAGCTCCG 6840
   GTTCCCAACG ATCAAGGCGA GTTACATGAT CCCCCATGTT GTGCAAAAAA GCGGTTAGCT 6900
   CCTTCGGTCC TCCGATCGTT GTCAGAAGTA AGTTGGCCGC AGTGTTATCA CTCATGGTTA 6960
   TGGCAGCACT GCATAATTCT CTTACTGTCA TGCCATCCGT AAGATGCTTT TCTGTGACTG 7020
   GTGAGTACTC AACCAAGTCA TTCTGAGAAT AGTGTATGCG GCGACCGAGT TGCTCTTGCC 7080
   CGGCGTCAAT ACGGGATAAT ACCGCGCCAC ATAGCAGAAC TTTAAAAGTG CTCATCATTG 7140
   GAAAACGTTC TTCGGGGCGA AAACTCTCAA GGATCTTACC GCTGTTGAGA TCCAGTTCGA 7200
   TGTAACCCAC TCGTGCACCC AACTGATCTT CAGCATCTTT TACTTTCACC AGCGTTTCTG 7260
   GGTGAGCAAA AACAGGAAGG CAAAATGCCG CAAAAAAGGG AATAAGGGCG ACACGGAAAT 7320
   GTTGAATACT CATACTCTTC CTTTTTCAAT ATTATTGAAG CATTTATCAG GGTTATTGTC 7380
   TCATGAGCGG ATACATATTT GAATGTATTT AGAAAAATAA ACAAATAGGG GTTCCGCGCA 7440
   CATTTCCCCG AAAAGTGCCA CCTGACGTC 7469
(2) INFORMATION FOR SEQ ID NO:9:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:9:
   TGCTTAAGCG GCCGCGAAGG AGAAGTCC 28
(2) INFORMATION FOR SEQ ID NO:10:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:10:
   CCGAGCTAGC GACTGAAAAT GAG 23
(2) INFORMATION FOR SEQ ID NO:11:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:11:
   CCTCTCGAGA GACAGCAAGA CAC 23
(2) INFORMATION FOR SEQ ID NO:12:
   (i) SEQUENCE CHARACTERISTICS:
      (A) LENGTH: 11152 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:12:
   AAGCTTGGGC AGAAATGGTT GAACTCCCGA GAGTGTCCTA CACCTAGGGG AGAAGCAGCC 60
   AAGGGGTTGT TTCCCACCAA GGACGACCCG TCTGCGCACA AACGGATGAG CCCATCAGAC 120
   AAAGACATAT TCATTCTCTG CTGCAAACTT GGCATAGCTC TGCTTTGCCT GGGGCTATTG 180
   GGGGAAGTTG CGGTTCGTGC TCGCAGGGCT CTCACCCTTG ACTCTTTTAA TAGCTCTTCT 240
   GTGCAAGATT ACAATCTAAA CAATTCGGAG AACTCGACCT TCCTCCTGAG GCAAGGACCA 300
   CAGCCAACTT CCTCTTACAA GCCGCATCGA TTTTGTCCTT CAGAAATAGA AATAAGAATG 360
   CTTGCTAAAA ATTATATTTT TACCAATAAG ACCAATCCAA TAGGTAGATT ATTAGTTACT 420
   ATGTTAAGAA ATGAATCATT ATCTTTTAGT ACTATTTTTA CTCAAATTCA GAAGTTAGAA 480
   ATGGGAATAG AAAATAGAAA GAGACGCTCA ACCTCAATTG AAGAACAGGT GCAAGGACTA 540
   TTGACCACAG GCCTAGAAGT AAAAAAGGGA AAAAAGAGTG TTTTTGTCAA AATAGGAGAC 600
   AGGTGGTGGC AACCAGGGAC TTATAGGGGA CCTTACATCT ACAGACCAAC AGATGCCCCC 660
   TTACCATATA CAGGAAGATA TGACTTAAAT TGGGATAGGT GGGTTACAGT CAATGGCTAT 720
   AAAGTGTTAT ATAGATCCCT CCCTTTTCGT GAAAGACTCG CCAGAGCTAG ACCTCCTTGG 780
   TGTATGTTGT CTCAAGAAGA AAAAGACGAC ATGAAACAAC AGGTACATGA TTATATTTAT 840
   CTAGGAACAG GAATGCACTT TTGGGGAAAG ATTTTCCATA CCAAGGAGGG GACAGTGGCT
   900
   GGACTAATAG AACATTATTC TGCAAAAACT CATGGCATGA GTTATTATGA ATAGCCTTTA
   960
   TTGGCCCAAC CTTGCGGTTC CCAGGGCTTA AGTAAGTTTT TGGTTACAAA CTGTTCTTAA
   1020
   AACGAGGATG TGAGACAAGT GGTTTCCTGA CTTGGTTTGG TATCAAAGGT TCTGATCTGA
   1080
   GCTCTGAGTG TTCTATTTTC CTATGTTCTT TTGGAATTTA TCCAAATCTT ATGTAAATGC
   1140
   TTATGTAAAC CAAGATATAA AAGAGTGCTG ATTTTTTGAG TAAACTTGCA ACAGTCCTAA
   1200
   CATTCACCTC TTGTGTGTTT GTGTCTGTTC GCCATCCCGT CTCCGCTCGT CACTTATCCT
   1260
   TCACTTTCCA GAGGGTCCCC CCGCAGACCC CGGCGACCCT CAGGTCGGCC GACTGCGGCA
   1320
   GCTGGCGCCC GAACAGGGAC CCTCGGATAA GTGACCCTTG TCTCTATTTC TACTATTTGG
   1380
   TGTTTGTCTT GTATTGTCTC TTTCTTGTCT GGCTATCATC ACAAGAGCGG AACGGACTCA
   1440
   CCATAGGGAC CAAGCTAGCG ACTGAAAATG AGACATATTA TCTGCCACGG AGGTGTTATT
   1500
   ACCGAAGAAA TGGCCGCCAG TCTTTTGGAC CAGCTGATCG AAGAGGTACT GGCTGATAAT
   1560
   CTTCCACCTC CTAGCCATTT TGAACCACCT ACCCTTCACG AACTGTATGA TTTAGACGTG 1620
   ACGGCCCCCG AAGATCCCAA CGAGGAGGCG GTTTCGCAGA TTTTTCCCGA CTCTGTAATG 1680
   TTGGCGGTGC AGGAAGGGAT TGACTTACTC ACTTTTCCGC CGGCGCCCGG TTCTCCGGAG 1740
   CCGCCTCACC TTTCCCGGCA GCCCGAGCAG CCGGAGCAGA GAGCCTTGGG TCCGGTTTCT 1800
   ATGCCAAACC TTGTACCGGA GGTGATCGAT CTTACCTGCC ACGAGGCTGG CTTTCCACCC 1860
   AGTGACGACG AGGATGAAGA GGGTGAGGAG TTTGTGTTAG ATTATGTGGA GCACCCCGGG 1920
   CACGGTTGCA GGTCTTGTCA TTATCACCGG AGGAATACGG GGGACCCAGA TATTATGTGT 1980
   TCGCTTTGCT ATATGAGGAC CTGTGGCATG TTTGTCTACA GTAAGTGAAA ATTATGGGCA 2040
   GTGGGTGATA GAGTGGTGGG TTTGGTGTGG TAATTTTTTT TTTAATTTTT ACAGTTTTGT 2100
   GGTTTAAAGA ATTTTGTATT GTGATTTTTT TAAAAGGTCC TGTGTCTGAA CCTGAGCCTG 2160
   AGCCCGAGCC AGAACCGGAG CCTGCAAGAC CTACCCGCCG TCCTAAAATG GCGCCTGCTA 2220
   TCCTGAGACG CCCGACATCA CCTGTGTCTA GAGAATGCAA TAGTAGTACG GATAGCTGTG 2280
   ACTCCGGTCC TTCTAACACA CCTCCTGAGA TACACCCGGT GGTCCCGCTG TGCCCCATTA 2340
   AACCAGTTGC CGTGAGAGTT GGTGGGCGTC GCCAGGCTGT GGAATGTATC GAGGACTTGC 2400
   TTAACGAGCC TGGGCAACCT TTGGACTTGA GCTGTAAACG CCCCAGGCCA TAAGGTGTAA 2460
   ACCTGTGATT GCGTGTGTGG TTAACGCCTT TGTTTGCTGA ATGAGTTGAT GTAAGTTTAA 2520
   TAAAGGGTGA GATAATGTTT AACTTGCATG GCGTGTTAAA TGGGGCGGGG CTTAAAGGGT 2580
   ATATAATGCG CCGTGGGCTA ATCTTGGTTA CATCTGACCT CATGGAGGCT TGGGAGTGTT 2640
   TGGAAGATTT TTCTGCTGTG CGTAACTTGC TGGAACAGAG CTCTAACAGT ACCTCTTGGT 2700
   TTTGGAGGTT TCTGTGGGGC TCATCCCAGG CAAAGTTAGT CTGCAGAATT AAGGAGGATT 2760
   ACAAGTGGGA ATTTGAAGAG CTTTTGAAAT CCTGTGGTGA GCTGTTTGAT TCTTTGAATC 2820
   TGGGTCACCA GGCGCTTTTC CAAGAGAAGG TCATCAAGAC TTTGGATTTT TCCACACCGG 2880
   GGCGCGCTGC GGCTGCTGTT GCTTTTTTGA GTTTTATAAA GGATAAATGG AGCGAAGAAA 2940
   CCCATCTGAG CGGGGGGTAC CTGCTGGATT TTCTGGCCAT GCATCTGTGG AGAGCGGTTG 3000
   TGAGACACAA GAATCGCCTG CTACTGTTGT CTTCCGTCCG CCCGGCGATA ATACCGACGG 3060
   AGGAGCAGCA GCAGCAGCAG GAGGAAGCCA GGCGGCGGCG GCAGGAGCAG AGCCCATGGA 3120
   ACCCGAGAGC CGGCCTGGAC CCTCGGGAAT GAATGTTGTA CAGGTGGCTG AACTGTATCC 3180
   AGAACTGAGA CGCATTTTGA CAATTACAGA GGATGGGCAG GGGCTAAAGG GGGTAAAGAG 3240
   GGAGCGGGGG GCTTGTGAGG CTACAGAGGA GGCTAGGAAT CTAGCTTTTA GCTTAATGAC 3300
   CAGACACCGT CCTGAGTGTA TTACTTTTCA ACAGATCAAG GATAATTGCG CTAATGAGCT 3360
   TGATCTGCTG GCGCAGAAGT ATTCCATAGA GCAGCTGACC ACTTACTGGC TGCAGCCAGG 3420
   GGATGATTTT GAGGAGGCTA TTAGGGTATA TGCAAAGGTG GCACTTAGGC CAGATTGCAA 3480
   GTACAAGATC AGCAAACTTG TAAATATCAG GAATTGTTGC TACATTTCTG GGAACGGGGC 3540
   CGAGGTGGAG ATAGATACGG AGGATAGGGT GGCCTTTAGA TGTAGCATGA TAAATATGTG 3600
   GCCGGGGGTG CTTGGCATGG ACGGGGTGGT TATTATGAAT GTAAGGTTTA CTGGCCCCAA 3660
   TTTTAGCGGT ACGGTTTTCC TGGCCAATAC CAACCTTATC CTACACGGTG TAAGCTTCTA 3720
   TGGGTTTAAC AATACCTGTG TGGAAGCCTG GACCGATGTA AGGGTTCGGG GCTGTGCCTT
   3780
   TTACTGCTGC TGGAAGGGGG TGGTGTGTCG CCCCAAAAGC AGGGCTTCAA TTAAGAAATG
   3840
   CCTCTTTGAA AGGTGTACCT TGGGTATCCT GTCTGAGGGT AACTCCAGGG TGCGCCACAA
   3900
   TGTGGCCTCC GACTGTGGTT GCTTCATGCT AGTGAAAAGC GTGGCTGTGA TTAAGCATAA
   3960
   CATGGTATGT GGCAACTGCG AGGACAGGGC CTCTCAGATG CTGACCTGCT CGGACGGCAA
   4020
   CTGTCACCTG CTGAAGACCA TTCACGTAGC CAGCCACTCT CGCAAGGCCT GGCCAGTGTT
   4080
   TGAGCATAAC ATACTGACCC GCTGTTCCTT GCATTTGGGT AACAGGAGGG GGGTGTTCCT
   4140
   ACCTTACCAA TGCAATTTGA GTCACACTAA GATATTGCTT GAGCCCGAGA GCATGTCCAA
   4200
   GGTGAACCTG AACGGGGTGT TTGACATGAC CATGAAGATC TGGAAGGTGC TGAGGTACGA
   4260
   TGAGACCCGC ACCAGGTGCA GACCCTGCGA GTGTGGCGGT AAACATATTA GGAACCAGCC
   4320
   TGTGATGCTG GATGTGACCG AGGAGCTGAG GCCCGATCAC TTGGTGCTGG CCTGCACCCG
   4380
   CGCTGAGTTT GGCTCTAGCG ATGAAGATAC AGATTGAGGT ACTGAAATGT GTGGGCGTGG
   4440
   CTTAAGGGTG GGAAAGAATA TATAAGGTGG GGGTCTTATG TAGTTTTGTA TCTGTTTTGC
   4500
   AGCAGCCGCC GCCGCCATGA GCACCAACTC GTTTGATGGA AGCATTGTGA GCTCATATTT
   4560
   GACAACGCGC ATGCCCCCAT GGGCCGGGGT GCGTCAGAAT GTGATGGGCT CCAGCATTGA
   4620
   TGGTCGCCCC GTCCTGCCCG CAAACTCTAC TACCTTGACC TACGAGACCG TGTCTGGAAC
   4680
   GCCGTTGGAG ACTGCAGCCT CCGCCGCCGC TTCAGCCGCT GCAGCCACCG CCCGCGGGAT
   4740
   TGTGACTGAC TTTGCTTTCC TGAGCCCGCT TGCAAGCAGT GCAGCTTCCC GTTCATCCGC
   4800
   CCGCGATGAC AAGTTGACGG CTCTTTTGGC ACAATTGGAT TCTTTGACCC GGGAACTTAA
   4860
   TGTCGTTTCT CAGCAGCTGT TGGATCTGCG CCAGCAGGTT TCTGCCCTGA AGGCTTCCTC
   4920
   CCCTCCCAAT GCGGTTTAAA ACATAAATAA AAAACCAGAC TCTGTTTGGA TTTGGATCAA
   4980
   GCAAGTGTCT TGCTGTCTCT CGAGGGATCT TTGTGAAGGA ACCTTACTTC TGTGGTGTGA
   5040
   CATAATTGGA CAAACTACCT ACAGAGATTT AAAGCTCTAA GGTAAATATA AAATTTTTAA
   5100
   GTGTATAATG TGTTAAACTA CTGATTCTAA TTGTTTGTGT ATTTTAGATT CCAACCTATG
   5160
   GAACTGATGA ATGGGAGCAG TGGTGGAATG CCTTTAATGA GGAAAACCTG TTTTGCTCAG
   5220
   AAGAAATGCC ATCTAGTGAT GATGAGGCTA CTGCTGACTC TCAACATTCT ACTCCTCCAA
   5280
   AAAAGAAGAG AAAGGTAGAA GACCCCAAGG ACTTTCCTTC AGAATTGCTA AGTTTTTTGA
   5340
   GTCATGCTGT GTTTAGTAAT AGAACTCTTG CTTGCTTTGC TATTTACACC ACAAAGGAAA
   5400
   AAGCTGCACT GCTATACAAG AAAATTATGG AAAAATATTC TGTAACCTTT ATAAGTAGGC
   5460
   ATAACAGTTA TAATCATAAC ATACTGTTTT TTCTTACTCC ACACAGGCAT AGAGTGTCTG
   5520
   CTATTAATAA CTATGCTCAA AAATTGTGTA CCTTTAGCTT TTTAATTTGT AAAGGGGTTA
   5580
   ATAAGGAATA TTTGATGTAT AGTGCCTTGA CTAGAGATCA TAATCAGCCA TACCACATTT
   5640
   GTAGAGGTTT TACTTGCTTT AAAAAACCTC CCACACCTCC CCCTGAACCT GAAACATAAA
   5700
   ATGAATGCAA TTGTTGTTGT TAACTTGTTT ATTGCAGCTT ATAATGGTTA CAAATAAAGC
   5760
   AATAGCATCA CAAATTTCAC AAATAAAGCA TTTTTTTCAC TGCATTCTAG TTGTGGTTTG
   5820
   TCCAAACTCA TCAATGTATC TTATCATGTC TGGATCCGGC TGTGGAATGT GTGTCAGTTA
   5880
   GGGTGTGGAA AGTCCCCAGG CTCCCCAGCA GGCAGAAGTA TGCAAAGCAT GCATCTCAAT 5940
   TAGTCAGCAA CCAGGTGTGG AAAGTCCCCA GGCTCCCCAG CAGGCAGAAG TATGCAAAGC 6000
   ATGCATCTCA ATTAGTCAGC AACCATAGTC CCGCCCCTAA CTCCGCCCAT CCCGCCCCTA 6060
   ACTCCGCCCA GTTCCGCCCA TTCTCCGCCC CATGGCTGAC TAATTTTTTT TATTTATGCA 6120
   GAGGCCGAGG CCGCCTCGGC CTCTGAGCTA TTCCAGAAGT AGTGAGGAGG CTTTTTTGGA 6180
   GGCCTAGGCT TTTGCAAAAA GCTTGGACAC AAGACAGGCT TGCGAGATAT GTTTGAGAAT 6240
   ACCACTTTAT CCCGCGTCAG GGAGAGGCAG TGCGTAAAAA GACGCGGACT CATGTGAAAT 6300
   ACTGGTTTTT AGTGCGCCAG ATCTCTATAA TCTCGCGCAA CCTATTTTCC CCTCGAACAC 6360
   TTTTTAAGCC GTAGATAAAC AGGCTGGGAC ACTTCACATG AGCGAAAAAT ACATCGTCAC 6420
   CTGGGACATG TTGCAGATCC ATGCACGTAA ACTCGCAAGC CGACTGATGC CTTCTGAACA 6480
   ATGGAAAGGC ATTATTGCCG TAAGCCGTGG CGGTCTGGTA CCGGGTGCGT TACTGGCGCG 6540
   TGAACTGGGT ATTCGTCATG TCGATACCGT TTGTATTTCC AGCTACGATC ACGACAACCA 6600
   GCGCGAGCTT AAAGTGCTGA AACGCGCAGA AGGCGATGGC GAAGGCTTCA TCGTTATTGA 6660
   TGACCTGGTG GATACCGGTG GTACTGCGGT TGCGATTCGT GAAATGTATC CAAAAGCGCA 6720
   CTTTGTCACC ATCTTCGCAA AACCGGCTGG TCGTCCGCTG GTTGATGACT ATGTTGTTGA 6780
   TATCCCGCAA GATACCTGGA TTGAACAGCC GTGGGATATG GGCGTCGTAT TCGTCCCGCC 6840
   AATCTCCGGT CGCTAATCTT TTCAACGCCT GGCACTGCCG GGCGTTGTTC TTTTTAACTT 6900
   CAGGCGGGTT ACAATAGTTT CCAGTAAGTA TTCTGGAGGC TGCATCCATG ACACAGGCAA 6960
   ACCTGAGCGA AACCCTGTTC AAACCCCGCT TTAAACATCC TGAAACCTCG ACGCTAGTCC 7020
   GCCGCTTTAA TCACGGCGCA CAACCGCCTG TGCAGTCGGC CCTTGATGGT AAAACCATCC 7080
   CTCACTGGTA TCGCATGATT AACCGTCTGA TGTGGATCTG GCGCGGCATT GACCCACGCG 7140
   AAATCCTCGA CGTCCAGGCA CGTATTGTGA TGAGCGATGC CGAACGTACC GACGATGATT 7200
   TATACGATAC GGTGATTGGC TACCGTGGCG GCAACTGGAT TTATGAGTGG GCCCCGGATC 7260
   TTTGTGAAGG AACCTTACTT CTGTGGTGTG ACATAATTGG ACAAACTACC TACAGAGATT 7320
   TAAAGCTCTA AGGTAAATAT AAAATTTTTA AGTGTATAAT GTGTTAAACT ACTGATTCTA 7380
   ATTGTTTGTG TATTTTAGAT TCCAACCTAT GGAACTGATG AATGGGAGCA GTGGTGGAAT 7440
   GCCTTTAATG AGGAAAACCT GTTTTGCTCA GAAGAAATGC CATCTAGTGA TGATGAGGCT 7500
   ACTGCTGACT CTCAACATTC TACTCCTCCA AAAAAGAAGA GAAAGGTAGA AGACCCCAAG 7560
   GACTTTCCTT CAGAATTGCT AAGTTTTTTG AGTCATGCTG TGTTTAGTAA TAGAACTCTT 7620
   GCTTGCTTTG CTATTTACAC CACAAAGGAA AAAGCTGCAC TGCTATACAA GAAAATTATG 7680
   GAAAAATATT CTGTAACCTT TATAAGTAGG CATAACAGTT ATAATCATAA CATACTGTTT 7740
   TTTCTTACTC CACACAGGCA TAGAGTGTCT GCTATTAATA ACTATGCTCA AAAATTGTGT 7800
   ACCTTTAGCT TTTTAATTTG TAAAGGGGTT AATAAGGAAT ATTTGATGTA TAGTGCCTTG 7860
   ACTAGAGATC ATAATCAGCC ATACCACATT TGTAGAGGTT TTACTTGCTT TAAAAAACCT 7920
   CCCACACCTC CCCCTGAACC TGAAACATAA AATGAATGCA ATTGTTGTTG TTAACTTGTT 7980
   TATTGCAGCT TATAATGGTT ACAAATAAAG CAATAGCATC ACAAATTTCA CAAATAAAGC 8040
   ATTTTTTTCA CTGCATTCTA GTTGTGGTTT GTCCAAACTC ATCAATGTAT CTTATCATGT 8100
   CTGGATCCCC AGGAAGCTCC TCTGTGTCCT CATAAACCCT AACCTCCTCT ACTTGAGAGG 8160
   ACATTCCAAT CATAGGCTGC CCATCCACCC TCTGTGTCCT CCTGTTAATT AGGTCACTTA 8220
   ACAAAAAGGA AATTGGGTAG GGGTTTTTCA CAGACCGCTT TCTAAGGGTA ATTTTAAAAT 8280
   ATCTGGGAAG TCCCTTCCAC TGCTGTGTTC CAGAAGTGTT GGTAAACAGC CCACAAATGT 8340
   CAACAGCAGA AACATACAAG CTGTCAGCTT TGCACAAGGG CCCAACACCC TGCTCATCAA 8400
   GAAGCACTGT GGTTGCTGTG TTAGTAATGT GCAAAACAGG AGGCACATTT TCCCCACCTG 8460
   TGTAGGTTCC AAAATATCTA GTGTTTTCAT TTTTACTTGG ATCAGGAACC CAGCACTCCA 8520
   CTGGATAAGC ATTATCCTTA TCCAAAACAG CCTTGTGGTC AGTGTTCATC TGCTGACTGT 8580
   CAACTGTAGC ATTTTTTGGG GTTACAGTTT GAGCAGGATA TTTGGTCCTG TAGTTTGCTA 8640
   ACACACCCTG CAGCTCCAAA GGTTCCCCAC CAACAGCAAA AAAATGAAAA TTTGACCCTT 8700
   GAATGGGTTT TCCAGCACCA TTTTCATGAG TTTTTTGTGT CCCTGAATGC AAGTTTAACA 8760
   TAGCAGTTAC CCCAATAACC TCAGTTTTAA CAGTAACAGC TTCCCACATC AAAATATTTC
   8820
   CACAGGTTAA GTCCTCATTT AAATTAGGCA AAGGAATTCT TGAAGACGAA AGGGCCTCGT
   8880
   GATACGCCTA TTTTTATAGG TTAATGTCAT GATAATAATG GTTTCTTAGA CGTCAGGTGG
   8940
   CACTTTTCGG GGAAATGTGC GCGGAACCCC TATTTGTTTA TTTTTCTAAA TACATTCAAA
   9000
   TATGTATCCG CTCATGAGAC AATAACCCTG ATAAATGCTT CAATAATATT GAAAAAGGAA
   9060
   GAGTATGAGT ATTCAACATT TCCGTGTCGC CCTTATTCCC TTTTTTGCGG CATTTTGCCT
   9120
   TCCTGTTTTT GCTCACCCAG AAACGCTGGT GAAAGTAAAA GATGCTGAAG ATCAGTTGGG
   9180
   TGCACGAGTG GGTTACATCG AACTGGATCT CAACAGCGGT AAGATCCTTG AGAGTTTTCG
   9240
   CCCCGAAGAA CGTTTTCCAA TGATGAGCAC TTTTAAAGTT CTGCTATGTG GCGCGGTATT
   9300
   ATCCCGTGTT GACGCCGGGC AAGAGCAACT CGGTCGCCGC ATACACTATT CTCAGAATGA
   9360
   CTTGGTTGAG TACTCACCAG TCACAGAAAA GCATCTTACG GATGGCATGA CAGTAAGAGA
   9420
   ATTATGCAGT GCTGCCATAA CCATGAGTGA TAACACTGCG GCCAACTTAC TTCTGACAAC
   9480
   GATCGGAGGA CCGAAGGAGC TAACCGCTTT TTTGCACAAC ATGGGGGATC ATGTAACTCG
   9540
   CCTTGATCGT TGGGAACCGG AGCTGAATGA AGCCATACCA AACGACGAGC GTGACACCAC
   9600
   GATGCCTGCA GCAATGGCAA CAACGTTGCG CAAACTATTA ACTGGCGAAC TACTTACTCT
   9660
   AGCTTCCCGG CAACAATTAA TAGACTGGAT GGAGGCGGAT AAAGTTGCAG GACCACTTCT
   9720
   GCGCTCGGCC CTTCCGGCTG GCTGGTTTAT TGCTGATAAA TCTGGAGCCG GTGAGCGTGG
   9780
   GTCTCGCGGT ATCATTGCAG CACTGGGGCC AGATGGTAAG CCCTCCCGTA TCGTAGTTAT
   9840
   CTACACGACG GGGAGTCAGG CAACTATGGA TGAACGAAAT AGACAGATCG CTGAGATAGG
   9900
   TGCCTCACTG ATTAAGCATT GGTAACTGTC AGACCAAGTT TACTCATATA TACTTTAGAT
   9960
   TGATTTAAAA CTTCATTTTT AATTTAAAAG GATCTAGGTG AAGATCCTTT TTGATAATCT
   10020
   CATGACCAAA ATCCCTTAAC GTGAGTTTTC GTTCCACTGA GCGTCAGACC CCGTAGAAAA
   10080
   GATCAAAGGA TCTTCTTGAG ATCCTTTTTT TCTGCGCGTA ATCTGCTGCT TGCAAACAAA
   10140
   AAAACCACCG CTACCAGCGG TGGTTTGTTT GCCGGATCAA GAGCTACCAA CTCTTTTTCC
   10200
   GAAGGTAACT GGCTTCAGCA GAGCGCAGAT ACCAAATACT GTCCTTCTAG TGTAGCCGTA 10260
   GTTAGGCCAC CACTTCAAGA ACTCTGTAGC ACCGCCTACA TACCTCGCTC TGCTAATCCT 10320
   GTTACCAGTG GCTGCTGCCA GTGGCGATAA GTCGTGTCTT ACCGGGTTGG ACTCAAGACG 10380
   ATAGTTACCG GATAAGGCGC AGCGGTCGGG CTGAACGGGG GGTTCGTGCA CACAGCCCAG 10440
   CTTGGAGCGA ACGACCTACA CCGAACTGAG ATACCTACAG CGTGAGCTAT GAGAAAGCGC 10500
   CACGCTTCCC GAAGGGAGAA AGGCGGACAG GTATCCGGTA AGCGGCAGGG TCGGAACAGG 10560
   AGAGCGCACG AGGGAGCTTC CAGGGGGAAA CGCCTGGTAT CTTTATAGTC CTGTCGGGTT 10620
   TCGCCACCTC TGACTTGAGC GTCGATTTTT GTGATGCTCG TCAGGGGGGC GGAGCCTATG 10680
   GAAAAACGCC AGCAACGCGG CCTTTTTACG GTTCCTGGCC TTTTGCTGGC CTTTTGCTCA 10740
   CATGTTCTTT CCTGCGTTAT CCCCTGATTC TGTGGATAAC CGTATTACCG CCTTTGAGTG 10800
   AGCTGATACC GCTCGCCGCA GCCGAACGAC CGAGCGCAGC GAGTCAGTGA GCGAGGAAGC 10860
   GGAAGAGCGC CTGATGCGGT ATTTTCTCCT TACGCATCTG TGCGGTATTT CACACCGCAT 10920
   ATGGTGCACT CTCAGTACAA TCTGCTCTGA TGCCGCATAG TTAAGCCAGT ATACACTCCG
   10980
   CTATCGCTAC GTGACTGGGT CATGGCTGCG CCCCGACACC CGCCAACACC CGCTGACGCG
   11040
   CCCTGACGGG CTTGTCTGCT CCCGGCATCC GCTTACAGAC AAGCTGTGAC CGTCTCCGGG
   11100
   AGCTGCATGT GTCAGAGGTT TTCACCGTCA TCACCGAAAC GCGCGAGGCA GC
   11152
(2) INFORMATION FOR SEQ ID NO:13:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:13:
   GACGGATCGG GAGATCTCC
   19
(2) INFORMATION FOR SEQ ID NO:14:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:14:
   CCGCCTCAGA AGCCATAGAG CC
   22
(2) INFORMATION FOR SEQ ID NO:15:
   (i) SEQUENCE CHARACTERISTICS:
      (A) LENGTH: 14455 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:15:
   AAGCTTGGGC AGAAATGGTT GAACTCCCGA GAGTGTCCTA CACCTAGGGG AGAAGCAGCC
   60
   AAGGGGTTGT TTCCCACCAA GGACGACCCG TCTGCGCACA AACGGATGAG CCCATCAGAC 120
   AAAGACATAT TCATTCTCTG CTGCAAACTT GGCATAGCTC TGCTTTGCCT GGGGCTATTG 180
   GGGGAAGTTG CGGTTCGTGC TCGCAGGGCT CTCACCCTTG ACTCTTTTAA TAGCTCTTCT 240
   GTGCAAGATT ACAATCTAAA CAATTCGGAG AACTCGACCT TCCTCCTGAG GCAAGGACCA 300
   CAGCCAACTT CCTCTTACAA GCCGCATCGA TTTTGTCCTT CAGAAATAGA AATAAGAATG 360
   CTTGCTAAAA ATTATATTTT TACCAATAAG ACCAATCCAA TAGGTAGATT ATTAGTTACT 420
   ATGTTAAGAA ATGAATCATT ATCTTTTAGT ACTATTTTTA CTCAAATTCA GAAGTTAGAA 480
   ATGGGAATAG AAAATAGAAA GAGACGCTCA ACCTCAATTG AAGAACAGGT GCAAGGACTA 540
   TTGACCACAG GCCTAGAAGT AAAAAAGGGA AAAAAGAGTG TTTTTGTCAA AATAGGAGAC 600
   AGGTGGTGGC AACCAGGGAC TTATAGGGGA CCTTACATCT ACAGACCAAC AGATGCCCCC 660
   TTACCATATA CAGGAAGATA TGACTTAAAT TGGGATAGGT GGGTTACAGT CAATGGCTAT 720
   AAAGTGTTAT ATAGATCCCT CCCTTTTCGT GAAAGACTCG CCAGAGCTAG ACCTCCTTGG 780
   TGTATGTTGT CTCAAGAAGA AAAAGACGAC ATGAAACAAC AGGTACATGA TTATATTTAT 840
   CTAGGAACAG GAATGCACTT TTGGGGAAAG ATTTTCCATA CCAAGGAGGG GACAGTGGCT
   900
   GGACTAATAG AACATTATTC TGCAAAAACT CATGGCATGA GTTATTATGA ATAGCCTTTA
   960
   TTGGCCCAAC CTTGCGGTTC CCAGGGCTTA AGTAAGTTTT TGGTTACAAA CTGTTCTTAA
   1020
   AACGAGGATG TGAGACAAGT GGTTTCCTGA CTTGGTTTGG TATCAAAGGT TCTGATCTGA
   1080
   GCTCTGAGTG TTCTATTTTC CTATGTTCTT TTGGAATTTA TCCAAATCTT ATGTAAATGC
   1140
   TTATGTAAAC CAAGATATAA AAGAGTGCTG ATTTTTTGAG TAAACTTGCA ACAGTCCTAA
   1200
   CATTCACCTC TTGTGTGTTT GTGTCTGTTC GCCATCCCGT CTCCGCTCGT CACTTATCCT
   1260
   TCACTTTCCA GAGGGTCCCC CCGCAGACCC CGGCGACCCT CAGGTCGGCC GACTGCGGCA
   1320
   GCTGGCGCCC GAACAGGGAC CCTCGGATAA GTGACCCTTG TCTCTATTTC TACTATTTGG
   1380
   TGTTTGTCTT GTATTGTCTC TTTCTTGTCT GGCTATCATC ACAAGAGCGG AACGGACTCA
   1440
   CCATAGGGAC CAAGCTAGCG ACTGAAAATG AGACATATTA TCTGCCACGG AGGTGTTATT
   1500
   ACCGAAGAAA TGGCCGCCAG TCTTTTGGAC CAGCTGATCG AAGAGGTACT GGCTGATAAT
   1560
   CTTCCACCTC CTAGCCATTT TGAACCACCT ACCCTTCACG AACTGTATGA TTTAGACGTG 1620
   ACGGCCCCCG AAGATCCCAA CGAGGAGGCG GTTTCGCAGA TTTTTCCCGA CTCTGTAATG 1680
   TTGGCGGTGC AGGAAGGGAT TGACTTACTC ACTTTTCCGC CGGCGCCCGG TTCTCCGGAG 1740
   CCGCCTCACC TTTCCCGGCA GCCCGAGCAG CCGGAGCAGA GAGCCTTGGG TCCGGTTTCT 1800
   ATGCCAAACC TTGTACCGGA GGTGATCGAT CTTACCTGCC ACGAGGCTGG CTTTCCACCC 1860
   AGTGACGACG AGGATGAAGA GGGTGAGGAG TTTGTGTTAG ATTATGTGGA GCACCCCGGG 1920
   CACGGTTGCA GGTCTTGTCA TTATCACCGG AGGAATACGG GGGACCCAGA TATTATGTGT 1980
   TCGCTTTGCT ATATGAGGAC CTGTGGCATG TTTGTCTACA GTAAGTGAAA ATTATGGGCA 2040
   GTGGGTGATA GAGTGGTGGG TTTGGTGTGG TAATTTTTTT TTTAATTTTT ACAGTTTTGT 2100
   GGTTTAAAGA ATTTTGTATT GTGATTTTTT TAAAAGGTCC TGTGTCTGAA CCTGAGCCTG 2160
   AGCCCGAGCC AGAACCGGAG CCTGCAAGAC CTACCCGCCG TCCTAAAATG GCGCCTGCTA 2220
   TCCTGAGACG CCCGACATCA CCTGTGTCTA GAGAATGCAA TAGTAGTACG GATAGCTGTG 2280
   ACTCCGGTCC TTCTAACACA CCTCCTGAGA TACACCCGGT GGTCCCGCTG TGCCCCATTA 2340
   AACCAGTTGC CGTGAGAGTT GGTGGGCGTC GCCAGGCTGT GGAATGTATC GAGGACTTGC 2400
   TTAACGAGCC TGGGCAACCT TTGGACTTGA GCTGTAAACG CCCCAGGCCA TAAGGTGTAA 2460
   ACCTGTGATT GCGTGTGTGG TTAACGCCTT TGTTTGCTGA ATGAGTTGAT GTAAGTTTAA 2520
   TAAAGGGTGA GATAATGTTT AACTTGCATG GCGTGTTAAA TGGGGCGGGG CTTAAAGGGT 2580
   ATATAATGCG CCGTGGGCTA ATCTTGGTTA CATCTGACCT CATGGAGGCT TGGGAGTGTT 2640
   TGGAAGATTT TTCTGCTGTG CGTAACTTGC TGGAACAGAG CTCTAACAGT ACCTCTTGGT 2700
   TTTGGAGGTT TCTGTGGGGC TCATCCCAGG CAAAGTTAGT CTGCAGAATT AAGGAGGATT 2760
   ACAAGTGGGA ATTTGAAGAG CTTTTGAAAT CCTGTGGTGA GCTGTTTGAT TCTTTGAATC 2820
   TGGGTCACCA GGCGCTTTTC CAAGAGAAGG TCATCAAGAC TTTGGATTTT TCCACACCGG 2880
   GGCGCGCTGC GGCTGCTGTT GCTTTTTTGA GTTTTATAAA GGATAAATGG AGCGAAGAAA 2940
   CCCATCTGAG CGGGGGGTAC CTGCTGGATT TTCTGGCCAT GCATCTGTGG AGAGCGGTTG 3000
   TGAGACACAA GAATCGCCTG CTACTGTTGT CTTCCGTCCG CCCGGCGATA ATACCGACGG 3060
   AGGAGCAGCA GCAGCAGCAG GAGGAAGCCA GGCGGCGGCG GCAGGAGCAG AGCCCATGGA 3120
   ACCCGAGAGC CGGCCTGGAC CCTCGGGAAT GAATGTTGTA CAGGTGGCTG AACTGTATCC 3180
   AGAACTGAGA CGCATTTTGA CAATTACAGA GGATGGGCAG GGGCTAAAGG GGGTAAAGAG 3240
   GGAGCGGGGG GCTTGTGAGG CTACAGAGGA GGCTAGGAAT CTAGCTTTTA GCTTAATGAC 3300
   CAGACACCGT CCTGAGTGTA TTACTTTTCA ACAGATCAAG GATAATTGCG CTAATGAGCT 3360
   TGATCTGCTG GCGCAGAAGT ATTCCATAGA GCAGCTGACC ACTTACTGGC TGCAGCCAGG 3420
   GGATGATTTT GAGGAGGCTA TTAGGGTATA TGCAAAGGTG GCACTTAGGC CAGATTGCAA 3480
   GTACAAGATC AGCAAACTTG TAAATATCAG GAATTGTTGC TACATTTCTG GGAACGGGGC 3540
   CGAGGTGGAG ATAGATACGG AGGATAGGGT GGCCTTTAGA TGTAGCATGA TAAATATGTG 3600
   GCCGGGGGTG CTTGGCATGG ACGGGGTGGT TATTATGAAT GTAAGGTTTA CTGGCCCCAA 3660
   TTTTAGCGGT ACGGTTTTCC TGGCCAATAC CAACCTTATC CTACACGGTG TAAGCTTCTA 3720
   TGGGTTTAAC AATACCTGTG TGGAAGCCTG GACCGATGTA AGGGTTCGGG GCTGTGCCTT
   3780
   TTACTGCTGC TGGAAGGGGG TGGTGTGTCG CCCCAAAAGC AGGGCTTCAA TTAAGAAATG
   3840
   CCTCTTTGAA AGGTGTACCT TGGGTATCCT GTCTGAGGGT AACTCCAGGG TGCGCCACAA
   3900
   TGTGGCCTCC GACTGTGGTT GCTTCATGCT AGTGAAAAGC GTGGCTGTGA TTAAGCATAA
   3960
   CATGGTATGT GGCAACTGCG AGGACAGGGC CTCTCAGATG CTGACCTGCT CGGACGGCAA
   4020
   CTGTCACCTG CTGAAGACCA TTCACGTAGC CAGCCACTCT CGCAAGGCCT GGCCAGTGTT
   4080
   TGAGCATAAC ATACTGACCC GCTGTTCCTT GCATTTGGGT AACAGGAGGG GGGTGTTCCT
   4140
   ACCTTACCAA TGCAATTTGA GTCACACTAA GATATTGCTT GAGCCCGAGA GCATGTCCAA
   4200
   GGTGAACCTG AACGGGGTGT TTGACATGAC CATGAAGATC TGGAAGGTGC TGAGGTACGA
   4260
   TGAGACCCGC ACCAGGTGCA GACCCTGCGA GTGTGGCGGT AAACATATTA GGAACCAGCC
   4320
   TGTGATGCTG GATGTGACCG AGGAGCTGAG GCCCGATCAC TTGGTGCTGG CCTGCACCCG
   4380
   CGCTGAGTTT GGCTCTAGCG ATGAAGATAC AGATTGAGGT ACTGAAATGT GTGGGCGTGG
   4440
   CTTAAGGGTG GGAAAGAATA TATAAGGTGG GGGTCTTATG TAGTTTTGTA TCTGTTTTGC
   4500
   AGCAGCCGCC GCCGCCATGA GCACCAACTC GTTTGATGGA AGCATTGTGA GCTCATATTT
   4560
   GACAACGCGC ATGCCCCCAT GGGCCGGGGT GCGTCAGAAT GTGATGGGCT CCAGCATTGA
   4620
   TGGTCGCCCC GTCCTGCCCG CAAACTCTAC TACCTTGACC TACGAGACCG TGTCTGGAAC
   4680
   GCCGTTGGAG ACTGCAGCCT CCGCCGCCGC TTCAGCCGCT GCAGCCACCG CCCGCGGGAT
   4740
   TGTGACTGAC TTTGCTTTCC TGAGCCCGCT TGCAAGCAGT GCAGCTTCCC GTTCATCCGC
   4800
   CCGCGATGAC AAGTTGACGG CTCTTTTGGC ACAATTGGAT TCTTTGACCC GGGAACTTAA
   4860
   TGTCGTTTCT CAGCAGCTGT TGGATCTGCG CCAGCAGGTT TCTGCCCTGA AGGCTTCCTC
   4920
   CCCTCCCAAT GCGGTTTAAA ACATAAATAA AAAACCAGAC TCTGTTTGGA TTTGGATCAA
   4980
   GCAAGTGTCT TGCTGTCTCT CGAGGGATCT TTGTGAAGGA ACCTTACTTC TGTGGTGTGA
   5040
   CATAATTGGA CAAACTACCT ACAGAGATTT AAAGCTCTAA GGTAAATATA AAATTTTTAA
   5100
   GTGTATAATG TGTTAAACTA CTGATTCTAA TTGTTTGTGT ATTTTAGATT CCAACCTATG
   5160
   GAACTGATGA ATGGGAGCAG TGGTGGAATG CCTTTAATGA GGAAAACCTG TTTTGCTCAG
   5220
   AAGAAATGCC ATCTAGTGAT GATGAGGCTA CTGCTGACTC TCAACATTCT ACTCCTCCAA
   5280
   AAAAGAAGAG AAAGGTAGAA GACCCCAAGG ACTTTCCTTC AGAATTGCTA AGTTTTTTGA
   5340
   GTCATGCTGT GTTTAGTAAT AGAACTCTTG CTTGCTTTGC TATTTACACC ACAAAGGAAA
   5400
   AAGCTGCACT GCTATACAAG AAAATTATGG AAAAATATTC TGTAACCTTT ATAAGTAGGC
   5460
   ATAACAGTTA TAATCATAAC ATACTGTTTT TTCTTACTCC ACACAGGCAT AGAGTGTCTG
   5520
   CTATTAATAA CTATGCTCAA AAATTGTGTA CCTTTAGCTT TTTAATTTGT AAAGGGGTTA
   5580
   ATAAGGAATA TTTGATGTAT AGTGCCTTGA CTAGAGATCA TAATCAGCCA TACCACATTT
   5640
   GTAGAGGTTT TACTTGCTTT AAAAAACCTC CCACACCTCC CCCTGAACCT GAAACATAAA
   5700
   ATGAATGCAA TTGTTGTTGT TAACTTGTTT ATTGCAGCTT ATAATGGTTA CAAATAAAGC
   5760
   AATAGCATCA CAAATTTCAC AAATAAAGCA TTTTTTTCAC TGCATTCTAG TTGTGGTTTG
   5820
   TCCAAACTCA TCAATGTATC TTATCATGTC TGGATCCGGC TGTGGAATGT GTGTCAGTTA
   5880
   GGGTGTGGAA AGTCCCCAGG CTCCCCAGCA GGCAGAAGTA TGCAAAGCAT GCATCTCAAT
   5940
   TAGTCAGCAA CCAGGTGTGG AAAGTCCCCA GGCTCCCCAG CAGGCAGAAG TATGCAAAGC
   6000
   ATGCATCTCA ATTAGTCAGC AACCATAGTC CCGCCCCTAA CTCCGCCCAT CCCGCCCCTA
   6060
   ACTCCGCCCA GTTCCGCCCA TTCTCCGCCC CATGGCTGAC TAATTTTTTT TATTTATGCA
   6120
   GAGGCCGAGG CCGCCTCGGC CTCTGAGCTA TTCCAGAAGT AGTGAGGAGG CTTTTTTGGA
   6180
   GGCCTAGGCT TTTGCAAAAA GCTTGGACAC AAGACAGGCT TGCGAGATAT GTTTGAGAAT
   6240
   ACCACTTTAT CCCGCGTCAG GGAGAGGCAG TGCGTAAAAA GACGCGGACT CATGTGAAAT
   6300
   ACTGGTTTTT AGTGCGCCAG ATCTCTATAA TCTCGCGCAA CCTATTTTCC CCTCGAACAC
   6360
   TTTTTAAGCC GTAGATAAAC AGGCTGGGAC ACTTCACATG AGCGAAAAAT ACATCGTCAC
   6420
   CTGGGACATG TTGCAGATCC ATGCACGTAA ACTCGCAAGC CGACTGATGC CTTCTGAACA
   6480
   ATGGAAAGGC ATTATTGCCG TAAGCCGTGG CGGTCTGGTA CCGGGTGCGT TACTGGCGCG
   6540
   TGAACTGGGT ATTCGTCATG TCGATACCGT TTGTATTTCC AGCTACGATC ACGACAACCA
   6600
   GCGCGAGCTT AAAGTGCTGA AACGCGCAGA AGGCGATGGC GAAGGCTTCA TCGTTATTGA 6660
   TGACCTGGTG GATACCGGTG GTACTGCGGT TGCGATTCGT GAAATGTATC CAAAAGCGCA 6720
   CTTTGTCACC ATCTTCGCAA AACCGGCTGG TCGTCCGCTG GTTGATGACT ATGTTGTTGA 6780
   TATCCCGCAA GATACCTGGA TTGAACAGCC GTGGGATATG GGCGTCGTAT TCGTCCCGCC 6840
   AATCTCCGGT CGCTAATCTT TTCAACGCCT GGCACTGCCG GGCGTTGTTC TTTTTAACTT 6900
   CAGGCGGGTT ACAATAGTTT CCAGTAAGTA TTCTGGAGGC TGCATCCATG ACACAGGCAA 6960
   ACCTGAGCGA AACCCTGTTC AAACCCCGCT TTAAACATCC TGAAACCTCG ACGCTAGTCC 7020
   GCCGCTTTAA TCACGGCGCA CAACCGCCTG TGCAGTCGGC CCTTGATGGT AAAACCATCC 7080
   CTCACTGGTA TCGCATGATT AACCGTCTGA TGTGGATCTG GCGCGGCATT GACCCACGCG 7140
   AAATCCTCGA CGTCCAGGCA CGTATTGTGA TGAGCGATGC CGAACGTACC GACGATGATT 7200
   TATACGATAC GGTGATTGGC TACCGTGGCG GCAACTGGAT TTATGAGTGG GCCCCGGATC 7260
   TTTGTGAAGG AACCTTACTT CTGTGGTGTG ACATAATTGG ACAAACTACC TACAGAGATT 7320
   TAAAGCTCTA AGGTAAATAT AAAATTTTTA AGTGTATAAT GTGTTAAACT ACTGATTCTA 7380
   ATTGTTTGTG TATTTTAGAT TCCAACCTAT GGAACTGATG AATGGGAGCA GTGGTGGAAT 7440
   GCCTTTAATG AGGAAAACCT GTTTTGCTCA GAAGAAATGC CATCTAGTGA TGATGAGGCT 7500
   ACTGCTGACT CTCAACATTC TACTCCTCCA AAAAAGAAGA GAAAGGTAGA AGACCCCAAG 7560
   GACTTTCCTT CAGAATTGCT AAGTTTTTTG AGTCATGCTG TGTTTAGTAA TAGAACTCTT 7620
   GCTTGCTTTG CTATTTACAC CACAAAGGAA AAAGCTGCAC TGCTATACAA GAAAATTATG 7680
   GAAAAATATT CTGTAACCTT TATAAGTAGG CATAACAGTT ATAATCATAA CATACTGTTT 7740
   TTTCTTACTC CACACAGGCA TAGAGTGTCT GCTATTAATA ACTATGCTCA AAAATTGTGT 7800
   ACCTTTAGCT TTTTAATTTG TAAAGGGGTT AATAAGGAAT ATTTGATGTA TAGTGCCTTG 7860
   ACTAGAGATC ATAATCAGCC ATACCACATT TGTAGAGGTT TTACTTGCTT TAAAAAACCT 7920
   CCCACACCTC CCCCTGAACC TGAAACATAA AATGAATGCA ATTGTTGTTG TTAACTTGTT 7980
   TATTGCAGCT TATAATGGTT ACAAATAAAG CAATAGCATC ACAAATTTCA CAAATAAAGC 8040
   ATTTTTTTCA CTGCATTCTA GTTGTGGTTT GTCCAAACTC ATCAATGTAT CTTATCATGT 8100
   CTGGATCCCC AGGAAGCTCC TCTGTGTCCT CATAAACCCT AACCTCCTCT ACTTGAGAGG 8160
   ACATTCCAAT CATAGGCTGC CCATCCACCC TCTGTGTCCT CCTGTTAATT AGGTCACTTA 8220
   ACAAAAAGGA AATTGGGTAG GGGTTTTTCA CAGACCGCTT TCTAAGGGTA ATTTTAAAAT 8280
   ATCTGGGAAG TCCCTTCCAC TGCTGTGTTC CAGAAGTGTT GGTAAACAGC CCACAAATGT 8340
   CAACAGCAGA AACATACAAG CTGTCAGCTT TGCACAAGGG CCCAACACCC TGCTCATCAA 8400
   GAAGCACTGT GGTTGCTGTG TTAGTAATGT GCAAAACAGG AGGCACATTT TCCCCACCTG 8460
   TGTAGGTTCC AAAATATCTA GTGTTTTCAT TTTTACTTGG ATCAGGAACC CAGCACTCCA 8520
   CTGGATAAGC ATTATCCTTA TCCAAAACAG CCTTGTGGTC AGTGTTCATC TGCTGACTGT 8580
   CAACTGTAGC ATTTTTTGGG GTTACAGTTT GAGCAGGATA TTTGGTCCTG TAGTTTGCTA 8640
   ACACACCCTG CAGCTCCAAA GGTTCCCCAC CAACAGCAAA AAAATGAAAA TTTGACCCTT 8700
   GAATGGGTTT TCCAGCACCA TTTTCATGAG TTTTTTGTGT CCCTGAATGC AAGTTTAACA 8760
   TAGCAGTTAC CCCAATAACC TCAGTTTTAA CAGTAACAGC TTCCCACATC AAAATATTTC
   8820
   CACAGGTTAA GTCCTCATTT AAATTAGGCA AAGGAATTCT TGAAGACGAA AGGGCCTCGT
   8880
   GATACGCCTA TTTTTATAGG TTAATGTCAT GATAATAATG GTTTCTTAGA CGTCAGGTGG
   8940
   CACTTTTCGG GGAAATGTGC GCGGAACCCC TATTTGTTTA TTTTTCTAAA TACATTCAAA
   9000
   TATGTATCCG CTCATGAGAC AATAACCCTG ATAAATGCTT CAATAATATT GAAAAAGGAA
   9060
   GAGTATGAGT ATTCAACATT TCCGTGTCGC CCTTATTCCC TTTTTTGCGG CATTTTGCCT
   9120
   TCCTGTTTTT GCTCACCCAG AAACGCTGGT GAAAGTAAAA GATGCTGAAG ATCAGTTGGG
   9180
   TGCACGAGTG GGTTACATCG AACTGGATCT CAACAGCGGT AAGATCCTTG AGAGTTTTCG
   9240
   CCCCGAAGAA CGTTTTCCAA TGATGAGCAC TTTTAAAGTT CTGCTATGTG GCGCGGTATT
   9300
   ATCCCGTGTT GACGCCGGGC AAGAGCAACT CGGTCGCCGC ATACACTATT CTCAGAATGA
   9360
   CTTGGTTGAG TACTCACCAG TCACAGAAAA GCATCTTACG GATGGCATGA CAGTAAGAGA
   9420
   ATTATGCAGT GCTGCCATAA CCATGAGTGA TAACACTGCG GCCAACTTAC TTCTGACAAC
   9480
   GATCGGAGGA CCGAAGGAGC TAACCGCTTT TTTGCACAAC ATGGGGGATC ATGTAACTCG 9540
   CCTTGATCGT TGGGAACCGG AGCTGAATGA AGCCATACCA AACGACGAGC GTGACACCAC 9600
   GATGCCTGCA GCAATGGCAA CAACGTTGCG CAAACTATTA ACTGGCGAAC TACTTACTCT 9660
   AGCTTCCCGG CAACAATTAA TAGACTGGAT GGAGGCGGAT AAAGTTGCAG GACCACTTCT 9720
   GCGCTCGGCC CTTCCGGCTG GCTGGTTTAT TGCTGATAAA TCTGGAGCCG GTGAGCGTGG 9780
   GTCTCGCGGT ATCATTGCAG CACTGGGGCC AGATGGTAAG CCCTCCCGTA TCGTAGTTAT 9840
   CTACACGACG GGGAGTCAGG CAACTATGGA TGAACGAAAT AGACAGATCG CTGAGATAGG 9900
   TGCCTCACTG ATTAAGCATT GGTAACTGTC AGACCAAGTT TACTCATATA TACTTTAGAT 9960
   TGATTTAAAA CTTCATTTTT AATTTAAAAG GATCTAGGTG AAGATCCTTT TTGATAATCT 10020
   CATGACCAAA ATCCCTTAAC GTGAGTTTTC GTTCCACTGA GCGTCAGACC CCGTAGAAAA 10080
   GATCAAAGGA TCTTCTTGAG ATCCTTTTTT TCTGCGCGTA ATCTGCTGCT TGCAAACAAA 10140
   AAAACCACCG CTACCAGCGG TGGTTTGTTT GCCGGATCAA GAGCTACCAA CTCTTTTTCC 10200
   GAAGGTAACT GGCTTCAGCA GAGCGCAGAT ACCAAATACT GTCCTTCTAG TGTAGCCGTA 10260
   GTTAGGCCAC CACTTCAAGA ACTCTGTAGC ACCGCCTACA TACCTCGCTC TGCTAATCCT 10320
   GTTACCAGTG GCTGCTGCCA GTGGCGATAA GTCGTGTCTT ACCGGGTTGG ACTCAAGACG 10380
   ATAGTTACCG GATAAGGCGC AGCGGTCGGG CTGAACGGGG GGTTCGTGCA CACAGCCCAG 10440
   CTTGGAGCGA ACGACCTACA CCGAACTGAG ATACCTACAG CGTGAGCTAT GAGAAAGCGC 10500
   CACGCTTCCC GAAGGGAGAA AGGCGGACAG GTATCCGGTA AGCGGCAGGG TCGGAACAGG 10560
   AGAGCGCACG AGGGAGCTTC CAGGGGGAAA CGCCTGGTAT CTTTATAGTC CTGTCGGGTT 10620
   TCGCCACCTC TGACTTGAGC GTCGATTTTT GTGATGCTCG TCAGGGGGGC GGAGCCTATG 10680
   GAAAAACGCC AGCAACGCGG CCTTTTTACG GTTCCTGGCC TTTTGCTGGC CTTTTGCTCA 10740
   CATGTTCTTT CCTGCGTTAT CCCCTGATTC TGTGGATAAC CGTATTACCG CCTTTGAGTG 10800
   AGCTGATACC GCTCGCCGCA GCCGAACGAC CGAGCGCAGC GAGTCAGTGA GCGAGGAAGC 10860
   GGAAGAGCGC CTGATGCGGT ATTTTCTCCT TACGCATCTG TGCGGTATTT CACACCGCAT 10920
   ACCGCCTCAG AAGCCATAGA GCCCACCGCA TCCCCAGCAT GCCTGCTATT GTCTTCCCAA 10980
   TCCTCCCCCT TGCTGTCCTG CCCCACCCCA CCCCCCAGAA TAGAATGACA CCTACTCAGA 11040
   CAATGCGATG CAATTTCCTC ATTTTATTAG GAAAGGACAG TGGGAGTGGC ACCTTCCAGG 11100
   GTCAAGGAAG GCACGGGGGA GGGGCAAACA ACAGATGGCT GGCAACTAGA AGGCACAGTC 11160
   GAGGCTGATC AGCGAGCTCT AGCATTTAGG TGACACTATA GAATAGGGCC CTCTAGATGC 11220
   ATGCTCGAGC GGCCGCTTCT TTATTCTTGG GCAATGTATG AAAAAGTGTA AGAGGATGTG 11280
   GCAAATATTT CATTAATGTA GTTGTGGCCA GACCAGTCCC ATGAAAATGA CATAGAGTAT 11340
   GCACTTGGAG TTGTGTCTCC TGTTTCCTGT GTACCGTTTA GTGTAATGGT TAGTGTTACA 11400
   GGTTTAGTTT TGTCTCCGTT TAAGTAAACT TGACTGACAA TGTTACTTTT GGCAGTTTTA 11460
   CCGTGAGATT TTGGATAAGC TGATAGGTTA GGCATAAATC CAACAGCGTT TGTATAGGCT 11520
   GTGCCTTCAG TAAGATCTCC ATTTCTAAAG TTCCAATATT CTGGGTCCAG GAAGGAATTG 11580
   TTTAGTAGCA CTCCATTTTC GTCAAATCTT ATAATAAGAT GAGCACTTTG AACTGTTCCA 11640
   GATATTGGAG CCAAACTGCC TTTAACAGCC AAAACTGAAA CTGTAGCAAG TATTTGACTG 11700
   CCACATTTTG TTAAGACCAA AGTGAGTTTA GCATCTTTCT CTGCATTTAG TCTACAGTTA 11760
   GGAGATGGAG CTGGTGTGGT CCACAAAGTT AGCTTATCAT TATTTTTGTT TCCTACTGTA 11820
   ATGGCACCTG TGCTGTCAAA ACTAAGGCCA GTTCCTAGTT TAGGAACCAT AGCCTTGTTT 11880
   GAATCAAATT CTAGGCCATG GCCAATTTTT GTTTTGAGGG GATTTGTGTT TGGTGCATTA 11940
   GGTGAACCAA ATTCAAGCCC ATCTCCTGCA TTAATGGCTA TGGCTGTAGC GTCAAACATC 12000
   AACCCCTTGG CAGTGCTTAG GTTAACCTCA AGCTTTTTGG AATTGTTTGA AGCTGTAAAC 12060
   AAGTAAAGGC CTTTGTTGTA GTTAATATCC AAGTTGTGGG CTGAGTTTAT AAAAAGAGGG 12120
   CCCTGTCCTA GTCTTAGATT TAGTTGGTTT TGAGCATCAA ACGGATAACT AACATCAAGT 12180
   ATAAGGCGTC TGTTTTGAGA ATCAATCCTT AGTCCTCCTG CTACATTAAG TTGCATATTG 12240
   CCTTGTGAAT CAAAACCCAA GGCTCCAGTA ACTTTAGTTT GCAAGGAAGT ATTATTAATA 12300
   GTCACACCTG GACCAGTTGC TACGGTCAAA GTGTTTAGGT CGTCTGTTAC ATGCAAAGGA 12360
   GCCCCGTACT TTAGTCCTAG TTTTCCATTT TGTGTATAAA TGGGCTCTTT CAAGTCAATG 12420
   CCCAAGCTAC CAGTGGCAGT AGTTAGAGGG GGTGAGGCAG TGATAGTAAG GGTACTGCTA 12480
   TCGGTGGTGG TGAGGGGGCC TGATGTTTGC AGGGCTAGCT TTCCTTCTGA CACTGTGAGG 12540
   GGTCCTTGGG TGGCAATGCT AAGTTTGGAG TCGTGCACGG TTAGCGGGGC CTGTGATTGC 12600
   ATGGTGAGTG TGTTGCCCGC GACCATTAGA GGTGCGGCGG CAGCCACAGT TAGGGCTTCT 12660
   GAGGTAACTG TGAGGGGTGC AGATATTTCC AGGTTTATGT TTGACTTGGT TTTTTTGAGA 12720
   GGTGGGCTCA CAGTGGTTAC ATTTTGGGAG GTAAGGTTGC CGGCCTCGTC CAGAGAGAGG 12780
   CCGTTGCCCA TTTTGAGCGC AAGCATGCCA TTGGAGGTAA CTAGAGGTTC GGATAGGCGC 12840
   AAAGAGAGTA CCCCAGGGGG ACTCTCTTGA AACCCATTGG GGGATACAAA GGGAGGAGTA 12900
   AGAAAAGGCA CAGTTGGAGG ACCGGTTTCC GTGTCATATG GATACACGGG GTTGAAGGTA 12960
   TCTTCAGACG GTCTTGCGCG CTTCATCTTG GATCTCAAGC CTGCCACACC TCACCTCGAC 13020
   CATCCGCCGT CTCAAGACCG CCTACTTTAA TTACATCATC AGCAGCACCT CCGCCAGAAA 13080
   CAACCCCGAC CGCCACCCGC TGCCGCCCGC CACGGTGCTC AGCCTACCTT GCGACTGTGA
   13140
   CTGGTTAGAC GCCTTTCTCG AGAGGTTTTC CGATCCGGTC GATGCGGACT CGCTCAGGTC
   13200
   CCTCGGTGGC GGAGTACCGT TCGGAGGCCG ACGGGTTTCC GATCCAAGAG TACTGGAAAG
   13260
   ACCGCGAAGA GTTTGTCCTC AACCGCGAGC CCAACAGCGA GCTCGAATTC AGATCCGAGC
   13320
   TCGGTACCAA GCTTGGGTCT CCCTATAGTG AGTCGTATTA ATTTCGATAA GCCAGTAAGC
   13380
   AGTGGGTTCT CTAGTTAGCC AGAGAGCTCT GCTTATATAG ACCTCCCACC GTACACGCCT
   13440
   ACCGCCCATT TGCGTCAATG GGGCGGAGTT GTTACGACAT TTTGGAAAGT CCCGTTGATT
   13500
   TTGGTGCCAA AACAAACTCC CATTGACGTC AATGGGGTGG AGACTTGGAA ATCCCCGTGA
   13560
   GTCAAACCGC TATCCACGCC CATTGATGTA CTGCCAAAAC CGCATCACCA TGGTAATAGC
   13620
   GATGACTAAT ACGTAGATGT ACTGCCAAGT AGGAAAGTCC CATAAGGTCA TGTACTGGGC
   13680
   ATAATGCCAG GCGGGCCATT TACCGTCATT GACGTCAATA GGGGGCGTAC TTGGCATATG
   13740
   ATACACTTGA TGTACTGCCA AGTGGGCAGT TTACCGTAAA TAGTCCACCC ATTGACGTCA
   13800
   ATGGAAAGTC CCTATTGGCG TTACTATGGG AACATACGTC ATTATTGACG TCAATGGGCG 13860
   GGGGTCGTTG GGCGGTCAGC CAGGCGGGCC ATTTACCGTA AGTTATGTAA CGCGGAACTC 13920
   CATATATGGG CTATGAACTA ATGACCCCGT AATTGATTAC TATTAATAAC TAGTCAATAA 13980
   TCAATGTCAA CGCGTATATC TGGCCCGTAC ATCGCGAAGC AGCGCAAAAC GCCTAACCCT 14040
   AAGCAGATTC TTCATGCAAT TGTCGGTCAA GCCTTGCCTT GTTGTAGCTT AAATTTTGCT 14100
   CGCGCACTAC TCAGCGACCT CCAACACACA AGCAGGGAGC AGATACTGGC TTAACTATGC 14160
   GGCATCAGAG CAGATTGTAC TGAGAGTCGA CCATAGGGGA TCGGGAGATC TCCCGATCCG 14220
   TCTATGGTGC ACTCTCAGTA CAATCTGCTC TGATGCCGCA TAGTTAAGCC AGTATACACT 14280
   CCGCTATCGC TACGTGACTG GGTCATGGCT GCGCCCCGAC ACCCGCCAAC ACCCGCTGAC 14340
   GCGCCCTGAC GGGCTTGTCT GCTCCCGGCA TCCGCTTACA GACAAGCTGT GACCGTCTCC 14400
   GGGAGCTGCA TGTGTCAGAG GTTTTCACCG TCATCACCGA AACGCGCGAG GCAGC 14455
(2) INFORMATION FOR SEQ ID NO:16:
   (i) SEQUENCE CHARACTERISTICS:
      (A) LENGTH: 10610 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:16:
   GACGGATCGG GAGATCCGCG CGGTACACAG AATTCAGGAG ACACAACTCC AAGTGCATAC
   60
   TCTATGTCAT TTTCATGGGA CTGGTCTGGC CACAACTACA TTAATGAAAT ATTTGCCACA
   120
   TCCTCTTACA CTTTTTCATA CATTGCCCAA GAATAAAGAA TCGTTTGTGT TATGTTTCAA
   180
   CGTGTTTATT TTTCAATTGC AGAAAATTTC AAGTCATTTT TCATTCAGTA GTATAGCCCC
   240
   ACCACCACAT AGCTTATACA GATCACCGTA CCTTAATCAA ACTCACAGAA CCCTAGTATT
   300
   CAACCTGCCA CCTCCCTCCC AACACACAGA GTACACAGTC CTTTCTCCCC GGCTGGCCTT
   360
   AAAAAGCATC ATATCATGGG TAACAGACAT ATTCTTAGGT GTTATATTCC ACACGGTTTC
   420
   CTGTCGAGCC AAACGCTCAT CAGTGATATT AATAAACTCC CCGGGCAGCT CACTTAAGTT 480
   CATGTCGCTG TCCAGCTGCT GAGCCACAGG CTGCTGTCCA ACTTGCGGTT GCTTAACGGG 540
   CGGCGAAGGA GAAGTCCACG CCTACATGGG GGTAGAGTCA TAATCGTGCA TCAGGATAGG 600
   GCGGTGGTGC TGCAGCAGCG CGCGAATAAA CTGCTGCCGC CGCCGCTCCG TCCTGCAGGA 660
   ATACAACATG GCAGTGGTCT CCTCAGCGAT GATTCGCACC GCCCGCAGCA TAAGGCGCCT 720
   TGTCCTCCGG GCACAGCAGC GCACCCTGAT CTCACTTAAA TCAGCACAGT AACTGCAGCA 780
   CAGCACCACA ATATTGTTCA AAATCCCACA GTGCAAGGCG CTGTATCCAA AGCTCATGGC 840
   GGGGACCACA GAACCCACGT GGCCATCATA CCACAAGCGC AGGTAGATTA AGTGGCGACC 900
   CCTCATAAAC ACGCTGGACA TAAACATTAC CTCTTTTGGC ATGTTGTAAT TCACCACCTC 960
   CCGGTACCAT ATAAACCTCT GATTAAACAT GGCGCCATCC ACCACCATCC TAAACCAGCT 1020
   GGCCAAAACC TGCCCGCCGG CTATACACTG CAGGGAACCG GGACTGGAAC AATGACAGTG 1080
   GAGAGCCCAG GACTCGTAAC CATGGATCAT CATGCTCGTC ATGATATCAA TGTTGGCACA 1140
   ACACAGGCAC ACGTGCATAC ACTTCCTCAG GATTACAAGC TCCTCCCGCG TTAGAACCAT 1200
   ATCCCAGGGA ACAACCCATT CCTGAATCAG CGTAAATCCC ACACTGCAGG GAAGACCTCG 1260
   CACGTAACTC ACGTTGTGCA TTGTCAAAGT GTTACATTCG GGCAGCAGCG GATGATCCTC 1320
   CAGTATGGTA GCGCGGGTTT CTGTCTCAAA AGGAGGTAGA CGATCCCTAC TGTACGGAGT 1380
   GCGCCGAGAC AACCGAGATC GTGTTGGTCG TAGTGTCATG CCAAATGGAA CGCCGGACGT 1440
   AGTCATATTT CCTGAAGCAA AACCAGGTGC GGGCGTGACA AACAGATCTG CGTCTCCGGT 1500
   CTCGCCGCTT AGATCGCTCT GTGTAGTAGT TGTAGTATAT CCACTCTCTC AAAGCATCCA 1560
   GGCGCCCCCT GGCTTCGGGT TCTATGTAAA CTCCTTCATG CGCCGCTGCC CTGATAACAT 1620
   CCACCACCGC AGAATAAGCC ACACCCAGCC AACCTACACA TTCGTTCTGC GAGTCACACA 1680
   CGGGAGGAGC GGGAAGAGCT GGAAGAACCA TGTTTTTTTT TTTATTCCAA AAGATTATCC 1740
   AAAACCTCAA AATGAAGATC TATTAAGTGA ACGCGCTCCC CTCCGGTGGC GTGGTCAAAC 1800
   TCTACAGCCA AAGAACAGAT AATGGCATTT GTAAGATGTT GCACAATGGC TTCCAAAAGG 1860
   CAAACGGCCC TCACGTCCAA GTGGACGTAA AGGCTAAACC CTTCAGGGTG AATCTCCTCT 1920
   ATAAACATTC CAGCACCTTC AACCATGCCC AAATAATTCT CATCTCGCCA CCTTCTCAAT 1980
   ATATCTCTAA GCAAATCCCG AATATTAAGT CCGGCCATTG TAAAAATCTG CTCCAGAGCG 2040
   CCCTCCACCT TCAGCCTCAA GCAGCGAATC ATGATTGCAA AAATTCAGGT TCCTCACAGA 2100
   CCTGTATAAG ATTCAAAAGC GGAACATTAA CAAAAATACC GCGATCCCGT AGGTCCCTTC 2160
   GCAGGGCCAG CTGAACATAA TCGTGCAGGT CTGCACGGAC CAGCGCGGCC ACTTCCCCGC 2220
   CAGGAACCTT GACAAAAGAA CCCACACTGA TTATGACACG CATACTCGGA GCTATGCTAA 2280
   CCAGCGTAGC CCCGATGTAA GCTTTGTTGC ATGGGCGGCG ATATAAAATG CAAGGTGCTG 2340
   CTCAAAAAAT CAGGCAAAGC CTCGCGCAAA AAAGAAAGCA CATCGTAGTC ATGCTCATGC 2400
   AGATAAAGGC AGGTAAGCTC CGGAACCACC ACAGAAAAAG ACACCATTTT TCTCTCAAAC 2460
   ATGTCTGCGG GTTTCTGCAT AAACACAAAA TAAAATAACA AAAAAACATT TAAACATTAG 2520
   AAGCCTGTCT TACAACAGGA AAAACAACCC TTATAAGCAT AAGACGGACT ACGGCCATGC 2580
   CGGCGTGACC GTAAAAAAAC TGGTCACCGT GATTAAAAAG CACCACCGAC AGCTCCTCGG 2640
   TCATGTCCGG AGTCATAATG TAAGACTCGG TAAACACATC AGGTTGATTC ATCGGTCAGT 2700
   GCTAAAAAGC GACCGAAATA GCCCGGGGGA ATACATACCC GCAGGCGTAG AGACAACATT 2760
   ACAGCCCCCA TAGGAGGTAT AACAAAATTA ATAGGAGAGA AAAACACATA AACACCTGAA 2820
   AAACCCTCCT GCCTAGGCAA AATAGCACCC TCCCGCTCCA GAACAACATA CAGCGCTTCA 2880
   CAGCGGCAGC CTAACAGTCA GCCTTACCAG TAAAAAAGAA AACCTATTAA AAAAACACCA 2940
   CTCGACACGG CACCAGCTCA ATCAGTCACA GTGTAAAAAA GGGCCAAGTG CAGAGCGAGT 3000
   ATATATAGGA CTAAAAAATG ACGTAACGGT TAAAGTCCAC AAAAAACACC CAGAAAACCG 3060
   CACGCGAACC TACGCCCAGA AACGAAAGCC AAAAAACCCA CAACTTCCTC AAATCGTCAC 3120
   TTCCGTTTTC CCACGTTACG TAACTTCCCG GATCCTCTCC CGATCCCCTA TGGTCGACTC 3180
   TCAGTACAAT CTGCTCTGAT GCCGCATAGT TAAGCCAGTA TCTGCTCCCT GCTTGTGTGT 3240
   TGGAGGTCGC TGAGTAGTGC GCGAGCAAAA TTTAAGCTAC AACAAGGCAA GGCTTGACCG 3300
   ACAATTGCAT GAAGAATCTG CTTAGGGTTA GGCGTTTTGC GCTGCTTCGC GATGTACGGG
   3360
   CCAGATATAC GCGTTGACAT TGATTATTGA CTAGTTATTA ATAGTAATCA ATTACGGGGT
   3420
   CATTAGTTCA TAGCCCATAT ATGGAGTTCC GCGTTACATA ACTTACGGTA AATGGCCCGC
   3480
   CTGGCTGACC GCCCAACGAC CCCCGCCCAT TGACGTCAAT AATGACGTAT GTTCCCATAG
   3540
   TAACGCCAAT AGGGACTTTC CATTGACGTC AATGGGTGGA CTATTTACGG TAAACTGCCC
   3600
   ACTTGGCAGT ACATCAAGTG TATCATATGC CAAGTACGCC CCCTATTGAC GTCAATGACG
   3660
   GTAAATGGCC CGCCTGGCAT TATGCCCAGT ACATGACCTT ATGGGACTTT CCTACTTGGC
   3720
   AGTACATCTA CGTATTAGTC ATCGCTATTA CCATGGTGAT GCGGTTTTGG CAGTACATCA
   3780
   ATGGGCGTGG ATAGCGGTTT GACTCACGGG GATTTCCAAG TCTCCACCCC ATTGACGTCA
   3840
   ATGGGAGTTT GTTTTGGCAC CAAAATCAAC GGGACTTTCC AAAATGTCGT AACAACTCCG
   3900
   CCCCATTGAC GCAAATGGGC GGTAGGCGTG TACGGTGGGA GGTCTATATA AGCAGAGCTC
   3960
   TCTGGCTAAC TAGAGAACCC ACTGCTTACT GGCTTATCGA AATTAATACG ACTCACTATA
   4020
   GGGAGACCCA AGCTTGGTAC CGAGCTCGGA TCTGAATTCG AGCTCGCTGT TGGGCTCGCG
   4080
   GTTGAGGACA AACTCTTCGC GGTCTTTCCA GTACTCTTGG ATCGGAAACC CGTCGGCCTC
   4140
   CGAACGGTAC TCCGCCACCG AGGGACCTGA GCGAGTCCGC ATCGACCGGA TCGGAAAACC
   4200
   TCTCGAGAAA GGCGTCTAAC CAGTCACAGT CGCAAGGTAG GCTGAGCACC GTGGCGGGCG
   4260
   GCAGCGGGTG GCGGTCGGGG TTGTTTCTGG CGGAGGTGCT GCTGATGATG TAATTAAAGT
   4320
   AGGCGGTCTT GAGACGGCGG ATGGTCGAGG TGAGGTGTGG CAGGCTTGAG ATCCAAGATG
   4380
   AAGCGCGCAA GACCGTCTGA AGATACCTTC AACCCCGTGT ATCCATATGA CACGGAAACC
   4440
   GGTCCTCCAA CTGTGCCTTT TCTTACTCCT CCCTTTGTAT CCCCCAATGG GTTTCAAGAG
   4500
   AGTCCCCCTG GGGTACTCTC TTTGCGCCTA TCCGAACCTC TAGTTACCTC CAATGGCATG
   4560
   CTTGCGCTCA AAATGGGCAA CGGCCTCTCT CTGGACGAGG CCGGCAACCT TACCTCCCAA
   4620
   AATGTAACCA CTGTGAGCCC ACCTCTCAAA AAAACCAAGT CAAACATAAA CCTGGAAATA
   4680
   TCTGCACCCC TCACAGTTAC CTCAGAAGCC CTAACTGTGG CTGCCGCCGC ACCTCTAATG
   4740
   GTCGCGGGCA ACACACTCAC CATGCAATCA CAGGCCCCGC TAACCGTGCA CGACTCCAAA 4800
   CTTAGCATTG CCACCCAAGG ACCCCTCACA GTGTCAGAAG GAAAGCTAGC CCTGCAAACA 4860
   TCAGGCCCCC TCACCACCAC CGATAGCAGT ACCCTTACTA TCACTGCCTC ACCCCCTCTA 4920
   ACTACTGCCA CTGGTAGCTT GGGCATTGAC TTGAAAGAGC CCATTTATAC ACAAAATGGA 4980
   AAACTAGGAC TAAAGTACGG GGCTCCTTTG CATGTAACAG ACGACCTAAA CACTTTGACC 5040
   GTAGCAACTG GTCCAGGTGT GACTATTAAT AATACTTCCT TGCAAACTAA AGTTACTGGA 5100
   GCCTTGGGTT TTGATTCACA AGGCAATATG CAACTTAATG TAGCAGGAGG ACTAAGGATT 5160
   GATTCTCAAA ACAGACGCCT TATACTTGAT GTTAGTTATC CGTTTGATGC TCAAAACCAA 5220
   CTAAATCTAA GACTAGGACA GGGCCCTCTT TTTATAAACT CAGCCCACAA CTTGGATATT 5280
   AACTACAACA AAGGCCTTTA CTTGTTTACA GCTTCAAACA ATTCCAAAAA GCTTGAGGTT 5340
   AACCTAAGCA CTGCCAAGGG GTTGATGTTT GACGCTACAG CCATAGCCAT TAATGCAGGA 5400
   GATGGGCTTG AATTTGGTTC ACCTAATGCA CCAAACACAA ATCCCCTCAA AACAAAAATT 5460
   GGCCATGGCC TAGAATTTGA TTCAAACAAG GCTATGGTTC CTAAACTAGG AACTGGCCTT 5520
   AGTTTTGACA GCACAGGTGC CATTACAGTA GGAAACAAAA ATAATGATAA GCTAACTTTG 5580
   TGGACCACAC CAGCTCCATC TCCTAACTGT AGACTAAATG CAGAGAAAGA TGCTAAACTC 5640
   ACTTTGGTCT TAACAAAATG TGGCAGTCAA ATACTTGCTA CAGTTTCAGT TTTGGCTGTT 5700
   AAAGGCAGTT TGGCTCCAAT ATCTGGAACA GTTCAAAGTG CTCATCTTAT TATAAGATTT 5760
   GACGAAAATG GAGTGCTACT AAACAATTCC TTCCTGGACC CAGAATATTG GAACTTTAGA 5820
   AATGGAGATC TTACTGAAGG CACAGCCTAT ACAAACGCTG TTGGATTTAT GCCTAACCTA 5880
   TCAGCTTATC CAAAATCTCA CGGTAAAACT GCCAAAAGTA ACATTGTCAG TCAAGTTTAC 5940
   TTAAACGGAG ACAAAACTAA ACCTGTAACA CTAACCATTA CACTAAACGG TACACAGGAA 6000
   ACAGGAGACA CAACTCCAAG TGCATACTCT ATGTCATTTT CATGGGACTG GTCTGGCCAC 6060
   AACTACATTA ATGAAATATT TGCCACATCC TCTTACACTT TTTCATACAT TGCCCAAGAA 6120
   TAAAGAAGCG GCCGCTCGAG CATGCATCTA GAGGGCCCTA TTCTATAGTG TCACCTAAAT 6180
   GCTAGAGCTC GCTGATCAGC CTCGACTGTG CCTTCTAGTT GCCAGCCATC TGTTGTTTGC
   6240
   CCCTCCCCCG TGCCTTCCTT GACCCTGGAA GGTGCCACTC CCACTGTCCT TTCCTAATAA
   6300
   AATGAGGAAA TTGCATCGCA TTGTCTGAGT AGGTGTCATT CTATTCTGGG GGGTGGGGTG
   6360
   GGGCAGGACA GCAAGGGGGA GGATTGGGAA GACAATAGCA GGCATGCTGG GGATGCGGTG
   6420
   GGCTCTATGG CTTCTGAGGC GGAAAGAACC AGCTGGGGCT CTAGGGGGTA TCCCCACGCG
   6480
   CCCTGTAGCG GCGCATTAAG CGCGGCGGGT GTGGTGGTTA CGCGCAGCGT GACCGCTACA
   6540
   CTTGCCAGCG CCCTAGCGCC CGCTCCTTTC GCTTTCTTCC CTTCCTTTCT CGCCACGTTC
   6600
   GCCGGCTTTC CCCGTCAAGC TCTAAATCGG GGCATCCCTT TAGGGTTCCG ATTTAGTGCT
   6660
   TTACGGCACC TCGACCCCAA AAAACTTGAT TAGGGTGATG GTTCACGTAG TGGGCCATCG
   6720
   CCCTGATAGA CGGTTTTTCG CCCTTTGACG TTGGAGTCCA CGTTCTTTAA TAGTGGACTC
   6780
   TTGTTCCAAA CTGGAACAAC ACTCAACCCT ATCTCGGTCT ATTCTTTTGA TTTATAAGGG
   6840
   ATTTTGGGGA TTTCGGCCTA TTGGTTAAAA AATGAGCTGA TTTAACAAAA ATTTAACGCG
   6900
   AATTAATTCT GTGGAATGTG TGTCAGTTAG GGTGTGGAAA GTCCCCAGGC TCCCCAGGCA 6960
   GGCAGAAGTA TGCAAAGCAT GCATCTCAAT TAGTCAGCAA CCAGGTGTGG AAAGTCCCCA 7020
   GGCTCCCCAG CAGGCAGAAG TATGCAAAGC ATGCATCTCA ATTAGTCAGC AACCATAGTC 7080
   CCGCCCCTAA CTCCGCCCAT CCCGCCCCTA ACTCCGCCCA GTTCCGCCCA TTCTCCGCCC 7140
   CATGGCTGAC TAATTTTTTT TATTTATGCA GAGGCCGAGG CCGCCTCTGC CTCTGAGCTA 7200
   TTCCAGAAGT AGTGAGGAGG CTTTTTTGGA GGCCTAGGCT TTTGCAAAAA GCTCCCGGGA 7260
   GCTTGTATAT CCATTTTCGG ATCTGATCAA GAGACAGGAT GAGGATCGTT TCGCATGATT 7320
   GAACAAGATG GATTGCACGC AGGTTCTCCG GCCGCTTGGG TGGAGAGGCT ATTCGGCTAT 7380
   GACTGGGCAC AACAGACAAT CGGCTGCTCT GATGCCGCCG TGTTCCGGCT GTCAGCGCAG 7440
   GGGCGCCCGG TTCTTTTTGT CAAGACCGAC CTGTCCGGTG CCCTGAATGA ACTGCAGGAC 7500
   GAGGCAGCGC GGCTATCGTG GCTGGCCACG ACGGGCGTTC CTTGCGCAGC TGTGCTCGAC 7560
   GTTGTCACTG AAGCGGGAAG GGACTGGCTG CTATTGGGCG AAGTGCCGGG GCAGGATCTC 7620
   CTGTCATCTC ACCTTGCTCC TGCCGAGAAA GTATCCATCA TGGCTGATGC AATGCGGCGG 7680
   CTGCATACGC TTGATCCGGC TACCTGCCCA TTCGACCACC AAGCGAAACA TCGCATCGAG 7740
   CGAGCACGTA CTCGGATGGA AGCCGGTCTT GTCGATCAGG ATGATCTGGA CGAAGAGCAT 7800
   CAGGGGCTCG CGCCAGCCGA ACTGTTCGCC AGGCTCAAGG CGCGCATGCC CGACGGCGAG 7860
   GATCTCGTCG TGACCCATGG CGATGCCTGC TTGCCGAATA TCATGGTGGA AAATGGCCGC 7920
   TTTTCTGGAT TCATCGACTG TGGCCGGCTG GGTGTGGCGG ACCGCTATCA GGACATAGCG 7980
   TTGGCTACCC GTGATATTGC TGAAGAGCTT GGCGGCGAAT GGGCTGACCG CTTCCTCGTG 8040
   CTTTACGGTA TCGCCGCTCC CGATTCGCAG CGCATCGCCT TCTATCGCCT TCTTGACGAG 8100
   TTCTTCTGAG CGGGACTCTG GGGTTCGAAA TGACCGACCA AGCGACGCCC AACCTGCCAT 8160
   CACGAGATTT CGATTCCACC GCCGCCTTCT ATGAAAGGTT GGGCTTCGGA ATCGTTTTCC 8220
   GGGACGCCGG CTGGATGATC CTCCAGCGCG GGGATCTCAT GCTGGAGTTC TTCGCCCACC 8280
   CCAACTTGTT TATTGCAGCT TATAATGGTT ACAAATAAAG CAATAGCATC ACAAATTTCA 8340
   CAAATAAAGC ATTTTTTTCA CTGCATTCTA GTTGTGGTTT GTCCAAACTC ATCAATGTAT
   8400
   CTTATCATGT CTGTATACCG TCGACCTCTA GCTAGAGCTT GGCGTAATCA TGGTCATAGC
   8460
   TGTTTCCTGT GTGAAATTGT TATCCGCTCA CAATTCCACA CAACATACGA GCCGGAAGCA
   8520
   TAAAGTGTAA AGCCTGGGGT GCCTAATGAG TGAGCTAACT CACATTAATT GCGTTGCGCT
   8580
   CACTGCCCGC TTTCCAGTCG GGAAACCTGT CGTGCCAGCT GCATTAATGA ATCGGCCAAC
   8640
   GCGCGGGGAG AGGCGGTTTG CGTATTGGGC GCTCTTCCGC TTCCTCGCTC ACTGACTCGC
   8700
   TGCGCTCGGT CGTTCGGCTG CGGCGAGCGG TATCAGCTCA CTCAAAGGCG GTAATACGGT
   8760
   TATCCACAGA ATCAGGGGAT AACGCAGGAA AGAACATGTG AGCAAAAGGC CAGCAAAAGG
   8820
   CCAGGAACCG TAAAAAGGCC GCGTTGCTGG CGTTTTTCCA TAGGCTCCGC CCCCCTGACG
   8880
   AGCATCACAA AAATCGACGC TCAAGTCAGA GGTGGCGAAA CCCGACAGGA CTATAAAGAT
   8940
   ACCAGGCGTT TCCCCCTGGA AGCTCCCTCG TGCGCTCTCC TGTTCCGACC CTGCCGCTTA
   9000
   CCGGATACCT GTCCGCCTTT CTCCCTTCGG GAAGCGTGGC GCTTTCTCAA TGCTCACGCT
   9060
   GTAGGTATCT CAGTTCGGTG TAGGTCGTTC GCTCCAAGCT GGGCTGTGTG CACGAACCCC
   9120
   CCGTTCAGCC CGACCGCTGC GCCTTATCCG GTAACTATCG TCTTGAGTCC AACCCGGTAA
   9180
   GACACGACTT ATCGCCACTG GCAGCAGCCA CTGGTAACAG GATTAGCAGA GCGAGGTATG
   9240
   TAGGCGGTGC TACAGAGTTC TTGAAGTGGT GGCCTAACTA CGGCTACACT AGAAGGACAG
   9300
   TATTTGGTAT CTGCGCTCTG CTGAAGCCAG TTACCTTCGG AAAAAGAGTT GGTAGCTCTT
   9360
   GATCCGGCAA ACAAACCACC GCTGGTAGCG GTGGTTTTTT TGTTTGCAAG CAGCAGATTA
   9420
   CGCGCAGAAA AAAAGGATCT CAAGAAGATC CTTTGATCTT TTCTACGGGG TCTGACGCTC
   9480
   AGTGGAACGA AAACTCACGT TAAGGGATTT TGGTCATGAG ATTATCAAAA AGGATCTTCA
   9540
   CCTAGATCCT TTTAAATTAA AAATGAAGTT TTAAATCAAT CTAAAGTATA TATGAGTAAA
   9600
   CTTGGTCTGA CAGTTACCAA TGCTTAATCA GTGAGGCACC TATCTCAGCG ATCTGTCTAT
   9660
   TTCGTTCATC CATAGTTGCC TGACTCCCCG TCGTGTAGAT AACTACGATA CGGGAGGGCT
   9720
   TACCATCTGG CCCCAGTGCT GCAATGATAC CGCGAGACCC ACGCTCACCG GCTCCAGATT
   9780
   TATCAGCAAT AAACCAGCCA GCCGGAAGGG CCGAGCGCAG AAGTGGTCCT GCAACTTTAT 9840
   CCGCCTCCAT CCAGTCTATT AATTGTTGCC GGGAAGCTAG AGTAAGTAGT TCGCCAGTTA 9900
   ATAGTTTGCG CAACGTTGTT GCCATTGCTA CAGGCATCGT GGTGTCACGC TCGTCGTTTG 9960
   GTATGGCTTC ATTCAGCTCC GGTTCCCAAC GATCAAGGCG AGTTACATGA TCCCCCATGT 10020
   TGTGCAAAAA AGCGGTTAGC TCCTTCGGTC CTCCGATCGT TGTCAGAAGT AAGTTGGCCG 10080
   CAGTGTTATC ACTCATGGTT ATGGCAGCAC TGCATAATTC TCTTACTGTC ATGCCATCCG 10140
   TAAGATGCTT TTCTGTGACT GGTGAGTACT CAACCAAGTC ATTCTGAGAA TAGTGTATGC 10200
   GGCGACCGAG TTGCTCTTGC CCGGCGTCAA TACGGGATAA TACCGCGCCA CATAGCAGAA 10260
   CTTTAAAAGT GCTCATCATT GGAAAACGTT CTTCGGGGCG AAAACTCTCA AGGATCTTAC 10320
   CGCTGTTGAG ATCCAGTTCG ATGTAACCCA CTCGTGCACC CAACTGATCT TCAGCATCTT 10380
   TTACTTTCAC CAGCGTTTCT GGGTGAGCAA AAACAGGAAG GCAAAATGCC GCAAAAAAGG 10440
   GAATAAGGGC GACACGGAAA TGTTGAATAC TCATACTCTT CCTTTTTCAA TATTATTGAA 10500
   GCATTTATCA GGGTTATTGT CTCATGAGCG GATACATATT TGAATGTATT TAGAAAAATA
   10560
   AACAAATAGG GGTTCCGCGC ACATTTCCCC GAAAAGTGCC ACCTGACGTC
   10610
(2) INFORMATION FOR SEQ ID NO:17:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:17:
      TGTACACCGG ATCCGGCGCA CACC24
(2) INFORMATION FOR SEQ ID NO:18:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:18:
(2) INFORMATION FOR SEQ ID NO:19:
   (i) SEQUENCE CHARACTERISTICS:
      (A) LENGTH: 4 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:19:
   Thr Leu Trp Thr
   1
(2) INFORMATION FOR SEQ ID NO:20:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:20:
   Pro Ser Ala Ser Ala Ser Ala Ser Ala Pro Gly Ser
   1 5 10